# EUROPÄISCHE PATENTANMELDUNG

(11) **EP 4 289 479 A2**
(43) Veröffentlichungstag der Anmeldung: **13.12.2023**
(21) Anmeldenummer: 23205262.1
(22) Anmeldetag: 01.10.2021
(51) Int. Cl.: A61P 29/00

(54) **1,8-CINEOL ENTHALTENDE ZUSAMMENSETZUNG FÜR DIE THERAPEUTISCHE VERWENDUNG**

(30) Priorität: 22.12.2020 DE 102020134587
(62) Teilanmeldung aus: 21789625.7
(71) Anmelder: Maria Clementine Martin Klosterfrau Vertriebsgesellschaft mbH, 50670 Köln (DE)
(72) Erfinder: PLOCH, Michael, 50670 Köln (DE); PRIES, Ralph, 23538 Lübeck (DE); BRUCHHAGE, Karl-Ludwig, 23538 Lübeck (DE)
(74) Vertreter: Strehlke, Ingo Kurt

(57) **Zusammenfassung**

Die vorliegende Erfindung betrifft eine 1,8-Cineol enthaltende Zusammensetzung in Form eines Nahrungsergänzungsmittels zur Verwendung bei der Verringerung pathogener Keime im menschlichen Darm bzw. zur Verwendung bei der Reduktion der Besiedlung des menschlichen Darms mit pathogenen Keimen.

## Beschreibung

Die vorliegende Erfindung betrifft das technische (d. h. medizinischpharmazeutische) Gebiet des Mikrobioms des menschlichen Darms und insbesondere der menschlichen Darmflora und weiterführend von entzündlichen Erkrankungen des menschlichen Körpers, welche insbesondere im Zusammenhang mit pathogenen Keimen in der Darmflora bzw. einer Fehlbesiedlung des Darms im Zusammenhang stehen bzw. hierdurch hervorgerufen oder verstärkt werden.

Die vorliegende Erfindung betrifft im Speziellen einen Wirkstoff und ein Arzneimittel bzw. eine Zusammensetzung jeweils zur Verwendung bei der Verringerung pathogener Keime im menschlichen Darm bzw. zur Verwendung bei der Reduktion der Besiedlung des menschlichen Darms mit pathogenen Keimen, und zwar vorzugsweise zu Zwecken der Vermeidung, Verringerung oder Heilung entzündlicher Erkrankungen bzw. vorzugsweise zu Zwecken der prophylaktischen bzw. therapeutischen Behandlung entzündlicher Erkrankungen des menschlichen Körpers. Gleichermaßen betrifft die vorliegende Erfindung auch einen Wirkstoff und ein Arzneimittel bzw. eine Zusammensetzung jeweils zur Verwendung bei der prophylaktischen bzw. therapeutischen Behandlung von Dysbiose bzw. Fehlbesiedlungen des menschlichen Darms. In entsprechender Weise betrifft die vorliegende Erfindung auch eine diesbezügliche Verwendung eines Wirkstoffs und eines Arzneimittels oder einer Zusammensetzung.

Erfindungsgemäß kommt dabei als Wirkstoff Cineol, vorzugsweise 1,8-Cineol, bzw. ein diesen Wirkstoff enthaltendes Arzneimittel bzw. eine entsprechende Zusammensetzung zum Einsatz.

Darüber hinaus betrifft die vorliegende Erfindung auch eine pharmazeutische Kombination auf Basis des erfindungsgemäß eingesetzten Wirkstoffs in Form von Cineol, vorzugsweise 1,8-Cineol.

Der menschliche Verdauungstrakt bzw. Gastrointestinaltrakt wird im Allgemeinen von Organen gebildet, welche der Aufnahme, der Zerkleinerung und dem Weitertransport von Nahrung dienen, wobei im Verdauungstrakt stoffwechselspezifische Vorgänge ablaufen, welche zu einer Freisetzung bzw. Aufspaltung von in der Nahrung enthaltenen Nährstoffen führen, damit diese für den Körper in verwertbarer Form vorliegen und um eine Aufnahme der Nährstoffe in den Körper zu ermöglichen. So finden im Verdauungstrakt insbesondere ein mechanischer und ein enzymatischer Aufschluss der Nahrung, gefolgt von einer Resorption von Nährstoffen, Elektrolyten und Wasser, sowie zudem auch eine Ausscheidung unverdaulicher bzw. nicht verwertbarer Nahrungsbestandteile aus dem Körper statt.

Eine bedeutende Rolle bei der Aufspaltung und Aufnahme von Nährstoffen sowie Elektrolyten und Wasser spielt dabei auch der menschliche Darm mit dem hierzu zählenden Dünn- bzw. Dickdarm. Dabei sind neben Enzymen auch zahlreiche Mikroorganismen, welche den Darm besiedeln, an der Verdauung beteiligt. Die Gesamtheit der Mikroorganismen, die den Darm des Menschen besiedeln, wird dabei als Darmflora bezeichnet, welche ein separates bzw. eigenständiges Mikrobiom des Menschen darstellt. In diesem Zusammenhang stellt der Darm mit der zugrundeliegenden Darmflora ein komplexes und dynamisches bakterielles Ökosystem dar, welches sich innerhalb der ersten Lebensjahre etabliert und welches sich mit zunehmendem Lebensalter entwickelt und dabei auch Veränderungen unterworfen ist, die auch pathologischer Natur sein können. Die Darmflora zeichnet sich durch das Vorhandensein einer großen Vielzahl verschiedener Bakterienfamilien- bzw. -gattungen und diesbezüglich zugrundeliegender Spezies aus.

Unerwünschte Veränderungen der Darmflora können beispielsweise durch eine falsche Ernährung, die Aufnahme kontaminierter Nahrung sowie durch den übermäßigen Einsatz von Medikamenten, wie Antibiotika, Cortison oder dergleichen, hervorgerufen werden. Zudem kann auch ein übermäßiger Alkoholkonsum zu einer unerwünschten Veränderung der Darmflora führen. Dabei können Veränderungen der Darmflora mit einer Unter- bzw. Überbesiedlung sowie mit einer Veränderung der bakteriellen Zusammensetzung einhergehen, welche sowohl im Bereich des Dickdarms als auch im Bereich des Dünndarm auftreten können. Eine Fehlbesiedlung des Darmes, welche insbesondere auch mit einer übermäßigen Präsenz bzw. einem übermäßigen Wachstum von pathogenen Keimen in der Darmflora einhergeht, kann dabei über längere Zeiträume fortbestehen und somit chronisch ausgebildet sein. Das im Rahmen einer Fehlbesiedlung des Darm resultierende Ungleichgewicht der Darmflora wird im Allgemeinen auch als Dysbiose bzw. Dysbakterie bezeichnet, welche auf eine übermäßige Präsenz von schädlicher bzw. pathogener Fremdflora im Darm abstellt.

Im Stand der Technik sind dabei zahlreiche Ansätze zur Einflussnahme auf die Darmflora bekannt, wobei ein diesbezüglicher Fokus insbesondere in der Behandlung akuter Störungen der Darmflora zu sehen ist, und zwar maßgeblich im Hinblick auf eine Linderung oder Unterbindung von mit akuten Beeinträchtigungen der Darmflora einhergehenden Durchfallsymptomen bzw. -erkrankungen, wie es beispielsweise für die häufig auftretende Reisediarrhoe der Fall ist. Hierbei handelt es sich um eine Infektionskrankheit des Darms, die durch pathogene Bakterien bzw. deren Toxine verursacht wird, wobei in mehr als 50 % der auftretenden Fälle pathogene, enterotoxinbildende Bakterien vom Typ *Escherichia coli* (*ETEC*) beteiligt sind. Der mit einem Durchfall verbundene Flüssigkeitsverlust kann zu Austrocknung, einhergehend mit einem signifikanten Elektrolytverlust und daraus folgendem Elektrolytmangel führen.

Im Stand der Technik kommen zur Behandlung von Durchfallerkrankungen oftmals pharmazeutische Präparate zum Einsatz, welche die Darmtätigkeit vermindern bzw. unterdrücken, wie es für den Wirkstoff Loperamid, welches zu den Opioiden zählt, der Fall ist. Der zugrundeliegende Wirkeffekt liegt in einer Verringerung der Darmperestaltik, wodurch die Durchfallsymptomatik vermindert wird. Jedoch wird hierdurch die Ausscheidung von Krankheitserregern bzw. von Toxinen behindert bzw. verzögert. Speziell im Hinblick auf mit einer Fehlbesiedlung des Darms einhergehende akute Durchfallerkrankung werden im Stand der Technik auch Elektrolyte bzw. Mineralstoffe, welche zudem mit Glukose kombiniert sein können, insbesondere in Form von wässrigen Lösungen verabreicht. Hierdurch kann zwar der Elektrolyt- und Flüssigkeitsverlust kompensiert werden, jedoch werden hierdurch die die Durchfallerkrankung auslösenden Erreger nicht bekämpft. Zudem kommt im Stand der Technik insbesondere zur Behandlung von Durchfallerkrankung auch die Verabreichung von lebenden bzw. lebensfähigen Mikroorganismen in Betracht. Die Wirkeffizienz derartiger Anwendungen ist jedoch nicht immer ausreichend.

Darüber hinaus werden im Stand der Technik im Hinblick auf die mit einer Fehlbesiedlung des Darms einhergehenden Durchfallerkrankungen auch Präparate eingesetzt, welche auf präbiotischen Komponenten und die Darmflora beeinflussenden Mikroorganismen basieren. So betrifft die DE 10 2008 059 070 A1 eine Zusammensetzung, welche insbesondere zur therapeutischen bzw. prophylaktischen Behandlung von Durchfallerkrankungen eingesetzt werden soll, wobei die diesbezügliche Zusammensetzung Mikroorganismen und mindestens ein Präbiotikum enthält. Hierdurch soll die körpereigene, natürliche Darmflora unterstützt werden. Auf dieser Basis können zwar relativ gute Effekte im Hinblick auf die Behandlung von akuten Durchfallsymptomen bzw. -erkrankungen erreicht werden. Jedoch ist auf dieser Basis eine gezielte Beeinflussung bzw. Einstellung der Darmflora mitunter nicht möglich, und zwar insbesondere nicht im Hinblick auf den Aspekt der Verbesserung der durch das Mikrobiom des Darms bzw. die Darmflora beeinflussten Immunsystems bzw. des diesbezüglich zugrundeliegenden Immunstatus und damit einhergehender oder hiermit in Zusammenhang stehender und auch auf körperlicher bzw. systemischer Ebene vorliegende entzündliche Erkrankungen.

Denn neben der wichtigen Verdauungsfunktion mit beispielsweise der weiterführenden Aufspaltung von Nährstoffen kommt der Darmflora bzw. den diesbezüglich zugrundeliegenden Keimen bzw. Bakterien auch eine große Bedeutung im Hinblick auf das Immunsystem bzw. entzündliche Prozesse im menschlichen Körper zu, beispielsweise was die Abwehr und Bekämpfung von pathogenen Erregern sowie eine weiterführende Immunmodulation und Einflussnahme auf entzündliche Prozesse anbelangt. Die Darmflora bzw. das Mikrobiom des Darms hat dabei auch einen hohen regulatorischen Einfluss auf Entzündungsprozesse und die dazugehörige Immunregulation. Neben einem negativen Einfluss auf die Verdauungsfunktion geht eine übermäßige Fehlbesiedlung des Darms mit pathogenen Keimen bzw. eine Dysbiose folglich oftmals auch mit nachteiligen Einflüssen auf das Immunsystem bzw. auf zugrundeliegende Immun(abwehr)reaktionen bzw. -regulationen einher.

Dabei ist auch beachtlich, dass im menschlichen Körper etwa 70 % bis 80 % sämtlicher (Immun-)Zellen, die Antikörper zu produzieren imstande sind, in der Darmschleimhaut bzw. in dem Darmepithel lokalisiert sind. Derartige Antikörper produzierende Zellen benötigen eine intakte Darmflora, um in optimaler Weise zu funktionieren. Dementsprechend kann eine nachhaltige Störung der Darmflora bzw. Dysbiose auch Auswirkungen auf derartige immunrelevante Zellen haben, einhergehend mit einer Unterfunktion bzw. Fehlsteuerung des Immunsystems auch im Hinblick auf entzündliche Prozesse im menschlichen Körper insgesamt.

Hinzu kommt, dass pathogene Keime bzw. pathogene Bakterien über Eigenschaften verfügen können, welche entzündliche Reaktionen initiieren bzw. beeinflussen und somit zu entsprechenden entzündlichen Erkrankungen führen bzw. entzündliche Erkrankungen verstärken bzw. modulieren können, wie beispielsweise die Produktion bzw. Freisetzung entsprechender entzündungsrelevanter Substanzen, welche nicht zuletzt auch auf (gesamt)körperlicher Ebene von Relevanz sind. Eine Fehlbesiedlung des Darms kann somit zu einer Beeinflussung des Immunsystems führen, einhergehend mit einer erhöhten Anfälligkeit für Infekte oder einer unerwünschten Verstärkung oder Initiierung entzündlicher Prozesse.

Eine Fehlbesiedlung des menschlichen Darms ist im Hinblick auf deren Einfluss auf immun- bzw. entzündungsrelevante Prozesse umso problematischer, als sich das Vorhandensein pathogener Keime im Darm und das Vorliegen eines beispielsweise hierdurch geschwächten Darmepithels hinsichtlich ihrer negativen Wirkung auf das Immunsystem bzw. entzündliche Prozesse insgesamt in unerwünschter Weise verstärken können.

Die Folgen einer Fehlbesiedlung des Darms bzw. einer Dysbiose liegen somit nicht nur in einer negativen Auswirkungen auf die Verdauungsfunktion als solche, sondern darüber hinaus auch in einer negativen Einflussnahme auf den Immunstatus eines hiervon betroffenen Patienten, und zwar auch im Hinblick auf die Beeinflussung bzw. das Hervorrufen entzündlicher Prozesse und einer damit einhergehenden Immunregulation. Eine durch eine Fehlbesiedlung des menschlichen Darms mit pathogenen Keimen bzw. eine durch Dysbiose verursachte Veränderung des Immunstatus bzw. der Abwehrstärke kann in entsprechender Weise auch Auswirkungen auf gesamtkörperliche Prozesse eines hiervon betroffenen Patienten haben. So führen derartige Fehlbesiedlungen des Darms bzw. Dysbiosen mitunter auch zu einer höheren Anfälligkeit gegenüber Infekten bzw. entzündlichen Erkrankungen insgesamt bzw. zu entzündlichen Reaktionen auf gesamtkörperlicher Ebene, und zwar auch im Hinblick auf eine Beeinflussung bzw. Modulation von im Körper insgesamt stattfindenden bzw. bereits vorhandenen entzündlichen Erkrankungen bzw. Prozessen. Folglich weisen Fehlbesiedlungen bzw. Dysbiosen eine über den menschlichen Darm als Ort ihres Vorliegens hinausgehende Wirkung auf den menschlichen Körper insgesamt auf, und zwar insbesondere im Hinblick auf einen regulatorischen Einfluss auf Entzündungsprozesse und entsprechender immunregulatorischer Vorgänge, so dass auf dieser Basis Erkrankungen bzw. entzündliche Prozesse im Körper insgesamt beeinflusst bzw. verstärkt werden können.

Im Lichte der obigen Ausführungen besteht somit - insbesondere auch aufgrund der mitunter großen Auswirkung einer Fehlbesiedlung des Darms mit pathogenen Keimen bzw. einer Dysbiose auf den Gesamtgesundheitszustand bzw. den Immunstatus - ein großer Bedarf, geeignete Therapieformen auf Basis effizienter Wirksubstanzen bzw. diesbezüglicher Zusammensetzungen und Arzneimittel bereitzustellen, welche sich in effizienter Weise zur Verringerung pathogener Keime im menschlichen Darm bzw. in der menschlichen Darmflora eignen, und zwar auch vor dem Hintergrund der Verbesserung des Immunstatus bzw. der Vermeidung bzw. Verringerung entzündlicher Erkrankungen des menschlichen Körpers insgesamt, welche insbesondere im Zusammenhang mit zugrundeliegenden Fehlbesiedlungen des menschlichen Darms mit pathogenen Keimen vorliegen bzw. hervorgerufen bzw. hierdurch negativ beeinflusst oder verstärkt werden.

Somit liegt der vorliegenden Erfindung die Aufgabe zugrunde, ein effizientes Konzept bzw. diesbezügliche Wirkstoffe und Zusammensetzungen sowie Arzneimittel bereitzustellen, welche bei ihrer Anwendung bzw. Applikation zu einer Verringerung pathogener Keime im menschlichen Darm bzw. zu einer Reduktion der Besiedlung des menschlichen Darms mit pathogenen Keimen und somit zu einer Normalisierung oder Verbesserung der menschlichen Darmflora führen, wobei diesbezüglich auch eine Verringerung bzw. Vermeidung bzw. Heilung entzündlicher Erkrankungen des menschlichen Körpers bzw. eine entsprechende Behandlung entzündlicher Erkrankungen des menschlichen Körpers ermöglicht werden soll. Neben einer guten Wirksamkeit soll gleichzeitig auch eine hervorragender Verträglichkeit und einfache Anwendung gewährleistet sein.

Gleichermaßen ist eine wiederum weitere Aufgabe der vorliegenden Erfindung auch darin zu sehen, entsprechende Wirkstoffe sowie hierauf basierende Zusammensetzungen und Arzneimittel bereitzustellen, welche sich zur insbesondere prophylaktischen und/oder therapeutischen Behandlung von Dysbiose bzw. Fehlbesiedlung des menschlichen Darms als solchen mit pathogenen Keimen eignen, und zwar insbesondere auch im Hinblick auf eine weiterführende positive Beeinflussung entzündlicher Erkrankungen des menschlichen Körpers insgesamt, wobei auch diesbezüglich bei hoher Wirkeffizienz eine gute Verträglichkeit bei einfacher Anwendbarkeit gegeben sein soll.

Eine weitere Aufgabe der vorliegenden Erfindung ist auch darin zu sehen, entsprechende Wirksubstanzen bzw. hierauf basierende Zusammensetzungen und Arzneimittel im Hinblick auf die Verringerung pathogener Keime im menschlichen Darm bzw. im Hinblick auf die Reduktion der Besiedlung des menschlichen Darms mit derartigen pathogenen Keimen bereitzustellen, welche die zuvor geschilderten Nachteile des Standes der Technik zumindest weitgehend vermeiden oder aber wenigstens abschwächen.

In vollkommen überraschender Weise hat die Anmelderin nunmehr herausgefunden, dass sich Cineol, insbesondere 1,8-Cineol, unerwartet und in effizienter Weise als Wirkstoff für eine nachhaltige Verringerung pathogener Keime im menschlichen Darm bzw. für eine diesbezügliche Reduktion der Besiedlung des menschlichen Darms mit pathogenen Keimen eignet, und zwar weiterführend auch im Zusammenhang mit einer Vermeidung, Verringerung bzw. Heilung entzündlicher Erkrankungen des menschlichen Körpers, welche insbesondere mit einer derartigen Fehlbesiedlung einhergehen bzw. hiermit im Zusammenhang stehen bzw. von pathogenen Keimen der Darmflora hervorgerufen bzw. hierdurch induziert bzw. gesteigert werden.

Zur Lösung der zuvor geschilderten Problemstellung schlägt daher die vorliegende Erfindung - gemäß einem **ersten** Aspekt der vorliegenden Erfindung - Cineol, insbesondere 1,8-Cineol, zur Verwendung bei der Verringerung pathogener Keime im menschlichen Darm bzw. zur Verwendung bei der Reduktion der Besieglung des menschlichen Darms mit pathogenen Keimen, vorzugsweise zu Zwecken der Vermeidung, Verringerung bzw. Heilung entzündlicher Erkrankungen des menschlichen Körpers bzw. vorzugsweise zu Zwecken der prophylaktischen bzw. therapeutischen Behandlung entzündlicher Erkrankungen des menschlichen Körpers, gemäß Patentanspruch 1 vor. Jeweils vorteilhafte Weiterbildungen und Ausgestaltungen dieses Erfindungsaspekts sind Gegenstand der diesbezüglichen Unteransprüche. Gemäß dem vorliegenden Aspekt betrifft die vorliegende Erfindung zudem auch Cineol, vorzugsweise 1,8-Cineol, zur Verwendung bei der prophylaktischen bzw. therapeutischen Behandlung von Dysbiose bzw. von Fehlbesiedlungen des menschlichen Darms mit pathogenen Keimen gemäß dem diesbezüglichen Nebenanspruch. Jeweils vorteilhafte Weiterbildungen und Ausgestaltungen dieses Erfindungsaspekts sind Gegenstand der betreffenden Unteransprüche.

Weiterer Gegenstand der vorliegenden Erfindung - gemäß einem **zweiten** Aspekt der vorliegenden Erfindung - ist zudem die erfindungsgemäße Verwendung von Cineol, vorzugsweise 1,8-Cineol, (zur Herstellung eines Arzneimittels oder Medikaments) zur Verringerung pathogener Keime im menschlichen Darm bzw. zur Reduktion der Besiedlung des menschlichen Darms mit pathogenen Keimen, und zwar vorzugsweise zu Zwecken der Vermeidung, Verringerung oder Heilung entzündlicher Erkrankungen des menschlichen Körpers bzw. vorzugsweise zu Zwecken der prophylaktischen bzw. therapeutischen Behandlung entzündlicher Erkrankungen des menschlichen Körpers gemäß den diesbezüglichen unabhängigen Patentansprüchen. Gemäß dem vorliegenden Aspekt betrifft die vorliegende Erfindung auch die Verwendung von Cineol, vorzugsweise 1,8-Cineol, zur prophylaktischen bzw. therapeutischen Behandlung von Dysbiose bzw. von Fehlbesiedlungen des menschlichen Darms mit pathogenen Keimen gemäß dem diesbezüglichen Nebenanspruch. Jeweils vorteilhafte Weiterbildungen und Ausgestaltungen dieses Erfindungsaspekts sind Gegenstand der betreffenden Unteransprüche.

Gleichermaßen Gegenstand der vorliegenden Erfindung - gemäß einem **dritten** Aspekt der vorliegenden Erfindung - ist die erfindungsgemäße Verwendung von Cineol, vorzugsweise 1,8-Cineol, als Darmbehandlungsmittel gemäß dem diesbezüglich unabhängigen Patentanspruch. Jeweils vorteilhafte Weiterbildungen und Ausgestaltungen dieses Erfindungsaspekts sind Gegenstand der betreffenden Unteransprüche.

Wiederum weiterer Gegenstand der vorliegenden Erfindung - gemäß einem **vierten** Aspekt der vorliegenden Erfindung - ist ein Verfahren zur Verringerung pathogener Keime im menschlichen Darm bzw. zur Reduktion der Besiedlung des menschlichen Darms mit pathogenen Keimen, und zwar vorzugsweise zu Zwecken der Vermeidung, Verringerung bzw. Heilung entzündlicher Erkrankungen des menschlichen Körpers bzw. zu Zwecken der prophylaktischen bzw. therapeutischen Behandlung entzündlicher Erkrankungen des menschlichen Körpers, gemäß dem diesbezüglich unabhängigen Patentanspruch. Gemäß diesem Aspekt betrifft die vorliegende Erfindung auch ein diesbezügliches Verfahren zur prophylaktischen bzw. therapeutischen Behandlung von Dysbiose bzw. von Fehlbesiedlungen des menschlichen Darms mit pathogenen Keimen gemäß dem diesbezüglichen Nebenanspruch. Jeweils vorteilhafte Weiterbildungen und Ausgestaltungen dieses Erfindungsaspekts sind Gegenstand der betreffenden Unteransprüche.

Ebenfalls Gegenstand der vorliegenden Erfindung - gemäß einem **fünften** Aspekt der vorliegenden Erfindung - ist zudem ein Arzneimittel oder Medikament zur (Verwendung bei der) Verringerung pathogener Keime im menschlichen Darm, bzw. zur (Verwendung bei der) Reduktion der Besiedelung des menschlichen Darms mit pathogenen Keimen gemäß dem diesbezüglichen unabhängigen Patentanspruch. Gemäß diesem Aspekt betrifft die vorliegende Erfindung auch ein Arzneimittel bzw. Medikament zur prophylaktischen bzw. therapeutischen Behandlung von Dysbiose bzw. von Fehlbesiedlungen des menschlichen Darms mit pathogenen Keimen gemäß dem diesbezüglichen Nebenanspruch. Jeweils vorteilhafte Weiterbildungen und Ausgestaltungen dieses Erfindungsaspekts sind Gegenstand der betreffenden Unteransprüche.

Gleichermaßen ist Gegenstand der vorliegenden Erfindung - gemäß einem **sechsten** Aspekt der vorliegenden Erfindung - eine Zusammensetzung zur (Verwendung bei der) Verringerung pathogener Keime im menschlichen Darm, bzw. zur (Verwendung bei der) Reduktion der Besiedelung des menschlichen Darms mit pathogenen Keimen gemäß dem diesbezüglichen unabhängigen Patentanspruch. Gemäß diesem Aspekt betrifft die vorliegende Erfindung auch eine diesbezügliche Zusammensetzung zur (Verwendung bei der) prophylaktischen bzw. therapeutischen Behandlung von Dysbiose bzw. von Fehlbesiedlungen des menschlichen Darms mit pathogenen Keimen gemäß dem diesbezüglichen Nebenanspruch. Jeweils vorteilhafte Weiterbildungen und Ausgestaltungen dieses Erfindungsaspekts sind Gegenstand der betreffenden Unteransprüche.

Schließlich ist Gegenstand der vorliegenden Erfindung - gemäß einem **siebten** Aspekt der vorliegenden Erfindung - gleichermaßen eine pharmazeutische Kombination zur (Verwendung bei der) Verringerung pathogener Keime im menschlichen Darm, bzw. zur (Verwendung bei der) Reduktion der Besiedelung des menschlichen Darms mit pathogenen Keimen gemäß dem diesbezüglichen unabhängigen Patentanspruch. In diesem Zusammenhang betrifft die vorliegende Erfindung auch eine pharmazeutische Kombination zur (Verwendung bei der) prophylaktischen bzw. therapeutischen Behandlung von Dysbiose bzw. von Fehlbesiedlungen des menschlichen Darms gemäß dem diesbezüglichen Nebenanspruch. Jeweils vorteilhafte Weiterbildungen und Ausgestaltungen dieses Erfindungsaspekts sind Gegenstand der betreffenden Unteransprüche.

Es versteht sich von selbst, dass Ausgestaltungen, Ausführungsformen, Vorteile und dergleichen, welche nachfolgend zu Zwecken der Vermeidung von Wiederholungen zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Darüber hinaus verhält es sich im Hinblick auf die nachfolgende Beschreibung der vorliegenden Erfindung auch derart, dass die jeweils im Zusammenhang mit den speziellen Ausgestaltungen, Ausführungsformen, Vorteilen, Beispielen oder dergleichen angeführten Merkmale der vorliegenden Erfindung auch in deren Kombination als offenbart gelten. Somit gelten vorliegend auch übergeordnete Kombinationen einzelner oder mehrerer Merkmale, welche für jeweilige Ausgestaltungen, Ausführungsformen, Anwendungsbeispiele oder dergleichen angeführt sind, als offenbart.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von dem angegebenen Bereich bzw. Angaben abgewichen werden kann, ohne dass der Rahmen der vorliegenden Erfindung verlassen ist.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungsmethoden ermittelt bzw. bestimmt werden können.

Ferner ist bei allen nachstehend genannten relativen bzw. prozentualen, insbesondere gewichtsbezogenen Mengenangaben zu beachten, dass diese Angaben im Rahmen der erfindungsgemäßen Zusammensetzung vom Fachmann derart auszuwählen bzw. zu kombinieren sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % bzw. 100 Gew.-% resultieren. Dies versteht sich für den Fachmann aber von selbst.

Zudem gilt, dass der Begriff des Arzneimittels bzw. Medikaments (synonym auch "Pharmazeutikum"), wie er im Rahmen der vorliegenden Erfindung verwendet wird, sehr umfänglich zu verstehen ist und nicht nur Arzneimittel bzw. Pharmazeutika als solche (d. h. in arzneimittelrechtlicher Hinsicht), sondern vor allem auch sogenannte Medizinprodukte und darüber hinaus aber auch Homöopathika und Nahrungsergänzungsmittel sowie Kosmetika und Gebrauchsgegenstände umfasst. Mit anderen Worten kann also die erfindungsgemäße Zusammensetzung in Form eines Arzneimittels (Pharmazeutikums), Medizinprodukts, Homöopathikums, Nahrungsergänzungsmittels, Kosmetikums oder in Form eines Gebrauchsgegenstands vorliegen.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit Cineol, vorzugsweise 1,8-Cineol, zur Verwendung bei der Verringerung pathogener Keime im menschlichen Darm, insbesondere in der menschlichen Darmflora, und/oder zur Verwendung bei der Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers.

Der Begriff "menschlicher Darm" ist im Rahmen der vorliegenden Erfindung sehr breit zu verstehen und bezieht sich insbesondere auf den sich vom Magenpförtner bis zum After erstreckenden Abschnitt des Verdauungstrakts. Insbesondere bezieht sich der in Rede stehende Begriff auf den Dünndarm- und/oder Dickdarmabschnitt des menschlichen Darms. Somit betrifft die vorliegende Erfindung insbesondere auch Cineol, vorzugsweise 1,8-Cineol, zur Verwendung bei der Verringerung pathogener Keime im menschlichen Dünndarm und/oder Dickdarm, insbesondere in der menschlichen Darmflora des Dünndarms und/oder Dickdarms, bzw. zur Verwendung bei der Reduktion der Besiedlung des menschlichen Dünndarms und/oder Dickdarms, insbesondere der menschlichen Darmflora des Dünndarms und/oder Dickdarms, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers.

Im Rahmen der vorliegenden Erfindung wird das Cineol insbesondere zur Reduktion der Keimlast bzw. der Keimanzahl der pathogenen Keime im menschlichen Darm bzw. insbesondere zur Reduktion des Keimanteils der pathogenen Keime, bezogen auf die Gesamtkeimanzahl im menschlichen Darm, insbesondere in der menschlichen Darmflora, verwendet oder eingesetzt.

Denn - wie zuvor ausgeführt - die Anmelderin hat in vollkommen überraschender Weise gefunden, dass Cineol, insbesondere 1,8-Cineol, sich unerwartet und in effizienter Weise als Wirkstoff zur Verringerung pathogener Keime im menschlichen Darm bzw. zur Reduktion der Besiedlung des menschlichen Darms mit pathogenen Keimen, und zwar insbesondere zu Zwecken der Vermeidung, Verringerung oder Heilung entzündlicher Erkrankungen des menschlichen Körpers bzw. insbesondere zu Zwecken der prophylaktischen und/oder therapeutischen Behandlung entzündlicher Erkrankungen des menschlichen Körpers eignet. Auch in diesem Zusammenhang eignet sich die vorliegende Wirksubstanz gleichermaßen zur prophylaktischen bzw. therapeutischen Behandlung von Dysbiose bzw. von diesbezüglichen Fehlbesiedlungen des menschlichen Darms.

Auf Basis der vorliegenden Erfindung kann durch den ziel- und zweckgerichteten Einsatz von Cineol, vorzugsweise 1,8-Cineol, somit in selektiver Weise eine Verringerung in Bezug auf pathogene Keime im Darm und bzw. eine diesbezügliche Erhöhung von nichtpathogenen bzw. gesundheitsfördernden Keimen im Darm erreicht werden, so dass auf Basis der erfindungsgemäßen Konzeption insgesamt eine positive Veränderung bzw. Beeinflussung der menschlichen Darmflora hinsichtlich ihrer Keimzusammensetzung bzw. -komposition erreicht wird. Erfindungsgemäß resultiert somit unter Anwendung von Cineol, insbesondere 1,8-Cineol, eine deutliche Verschiebung der mikrobiellen und insbesondere der bakteriellen Zusammensetzung des Darmmikrobioms, und zwar dahingehend, dass pathogene und somit schädliche bzw. mit Entzündungen im Zusammenhang stehende Keime in der Darmflora nachhaltig reduziert werden. Hierdurch wird auch das Vorhandensein von entzündungsrelevanten Substanzen bzw. Entzündungsmediatoren, welche von den pathogenen Keimen stammen, verringert.

Auf Basis der überraschend gefundenen gezielten Veränderung bzw. Einflussnahme auf das Mikrobiom des menschlichen Darms bzw. der menschlichen Darmflora durch Cineol, vorzugsweise 1,8-Cineol, mit der Verringerung pathogener Keime, wird - ohne sich auf diese Theorie berufen oder beschränken zu wollen - in diesem Zusammenhang ein sozusagen ganzheitlicher bzw. systemischer Effekt erreicht, wie im Rahmen der vorliegenden Erfindung gleichermaßen in völlig überraschender Weise gefunden, nämlich dahingehend, dass entzündliche Erkrankungen bzw. entzündliche Vorgänge im menschlichen Körper insgesamt im Sinne einer Verringerung bzw. Vermeidung bzw. Heilung positiv beeinflusst bzw. gesteuert werden können, und zwar über den menschlichen Darm selbst als primärer Wirkort für Cineol, insbesondere 1,8-Cineol, hinaus auch in anderen und vom menschlichen Darm verschiedenen Organen bzw. Körperregionen, wie beispielsweise dem Mund/Rachen-Raum oder dem Hals/Nasen/Ohren-Raum (wie nachfolgend noch ausgeführt) bzw. auf körperlicher bzw. systemischer Ebene insgesamt. Durch den erfindungsgemäß vorliegenden positiven Effekt auf die Darmflora mit der Verringerung entsprechend pathogener Keime kann dabei eine Verbesserung des Immunstatus bzw. des Immunsystems insgesamt insbesondere auch dahingehend erreicht werden, dass auch auf dieser Basis entzündliche Prozesse bzw. entzündliche Erkrankungen vermieden bzw. verringert bzw. geheilt werden können.

In diesem Zusammenhang hat die Anmelderin gleichermaßen in völlig überraschender Weise gefunden, dass entzündliche Erkrankungen im menschlichen Körper, wie beispielsweise entzündliche Erkrankungen des Mund/Rachen-Raums bzw. des Hals/Nasen/Ohren-Raums, wie Rhinosinusitis oder dergleichen, mit einer Fehlbesiedlung des menschlichen Darms mit pathogenen Keimen bzw. mit einer übermäßigen Präsenz pathogener Keime in der menschlichen Darmflora einhergehen können. Folglich liegt auch von daher im Rahmen der vorliegenden Erfindung ein effektiver und bislang nicht beschriebener oder angedachter Ansatzpunkt von Cineol, vorzugsweise 1,8-Cineol, mit der gezielten Beeinflussung der Darmflora dahingehend vor, dass hierdurch erstmals auch damit im Zusammenhang stehende entzündliche Erkrankungen des menschlichen Körpers prophylaktisch bzw. therapeutisch behandelt werden können.

Diesbezüglich verhält es sich im Rahmen der vorliegenden Erfindung somit insbesondere derart, dass bei gleichzeitiger Behandlung bzw. Therapie von abnormen bzw. krankhaften Zuständen des Mikrobioms des Darms bzw. der menschlichen Darmflora, eine entsprechende Reduktion bzw. Supprimierung entzündlicher Prozesse im Körper erfolgt, was weiterführend mit einem positiven Effekt auf entzündliche Erkrankungen bzw. Zuständen des menschlichen Körpers insgesamt einhergeht, so dass erfindungsgemäß erstmals ein ganzheitliches Therapiekonzept im Hinblick auf Fehlbesiedlungen des Darmes und damit im Zusammenhang stehenden entzündlichen Prozessen bzw. Erkrankungen des Körpers bereitgestellt wird.

Auf Basis der erfindungsgemäßen Konzeption mit der Verwendung von Cineol, vorzugsweise 1,8-Cineol, zur Verringerung pathogener Keime im menschlichen Darm zu Zwecken der Vermeidung, Verringerung bzw. Heilung entzündlicher Erkrankungen des menschlichen Körpers werden - gleichermaßen ohne sich auf diese Theorie berufen oder beschränken zu wollen - im Speziellen auch systemische Entzündungsparameter positiv beeinflusst, so dass auch auf dieser Basis ein insgesamt positiver und insbesondere entzündungshemmender bzw. -regulierender Effekt hinsichtlich der Behandlung weiterführender entzündlicher Erkrankungen des menschlichen Körpers vorliegt, welche nicht nur im Bereich des Darms selbst, sondern darüber hinaus in weiteren bzw. hiervon verschiedenen Körperabschnitten bzw. -regionen vorliegen können, wie nachfolgend noch im Detail ausgeführt.

Erfindungsgemäß wird somit durch den gezielten Einsatz von Cineol, vorzugsweise 1,8-Cineol, zu Zwecken der Reduktion der Besiedlung des menschlichen Darms bzw. der menschlichen Darmflora mit pathogenen Keimen - ohne sich auf diese Theorie beschränken oder berufen zu wollen - erstmals ein ganzheitlicher bzw. systemischer Ansatz in Bezug auf die damit einhergehende positive Wirkung auf entzündliche Erkrankungen des menschlichen Körpers bereitgestellt, wobei zudem erfindungsgemäß sozusagen ein universeller und im Hinblick auf die entzündliche Erkrankung des menschlichen Körpers unspezifischer bzw. allgemeingültiger Ansatz vorliegt, was zu der Möglichkeit der Behandlung einer Vielzahl verschiedener entzündlicher Erkrankungen führt, da erfindungsgemäß sozusagen übergeordnet relevante immun- bzw. entzündungsregulatorische Prozesse positiv beeinflusst bzw. reguliert werden können.

Erfindungsgemäß verhält es sich auch in diesem Zusammenhang insbesondere derart, dass das Cineol, vorzugsweise 1,8-Cineol, eine dysbiotische, insbesondere eine Fehlbesiedlung des menschlichen Darms mit pathogenen Keimen entgegenwirkende, Wirkung aufweist. Diesbezüglich kann das Cineol, vorzugsweise 1,8-Cineol, insbesondere eine den systemischen Immunstatus des menschlichen Körpers verbessernde bzw. regulierende, insbesondere eine eine Entzündungshemmung induzierende, Wirkung aufweisen.

Aufgrund der im Rahmen der vorliegenden Erfindung völlig überraschend gefundenen antidysbiotischen Wirkung des Cineols, vorzugsweise 1,8-Cineols, wird insgesamt ein entzündungshemmender Effekt bereitgestellt, und zwar auch auf systemischer Ebene, wobei dieser Effekt - ohne sich auf diese Theorie beschränken oder berufen zu wollen, mehrfach begründet ist:
So liegt (i) zum einen am primären Wirkort, nämlich dem menschlichen Darm, eine positive Wirkung auf das Darmepithel mit den dort vorliegenden Immunzellen vor, und zwar insbesondere auf Basis der Verringerung pathogener Keime im menschlichen Mikrobiom des Darms, so dass deren negativer Einfluss auf das Darmepithel verringert bzw. unterbunden wird, was zu einer Verbesserung bzw. Regulation des diesbezüglichen Immunstatus führt (Induzierung einer aktiven bzw. primären Entzündungshemmungsvermittlung). Zudem wird (ii) zum anderen durch die infolge der Verabreichung von Cineol, vorzugsweise 1,8-Cineol, hervorgerufene Verringerung bzw. Ausräumung von pathologischen Keimen im menschlichen Darm das Vorhandensein bzw. die Freisetzung von entsprechenden entzündungsrelevanten Substanzen, wie Entzündungsmediatoren oder dergleichen, welche von den pathogenen Keimen stammen, verringert bzw. vermieden (Induzierung einer passiven bzw. sekundären Entzündungshemmungsvermittlung). Zudem kann es sich im Rahmen der vorliegenden Erfindung zudem und insbesondere ergänzend zu (i) und (ii) auch derart verhalten, dass (iii) überschüssiges Cineol bzw. 1,8-Cineol (d. h. welches nicht für die antidysbiotische Wirkung im Darm benötigt bzw. sozusagen hierfür verbraucht wird) systemisch resorbiert bzw. systemisch aufgenommen und beispielsweise im Blutsystem ein kortikosteroidartiges Wirkprofil entfalten kann, so dass auch auf dieser Basis eine ergänzende antientzündliche Wirkung ausgeübt werden kann.

Durch das Ineinandergreifen und sich Verstärken der zugrundeliegenden Wirkungen bzw. Effekte resultiert erfindungsgemäß somit insgesamt eine nachhaltige antientzündliche Wirkung, welche nicht nur auf den Darm selbst beschränkt ist, sondern zudem darmübergreifend ist und sich somit auf den ganzen Körper positiv auswirkt. Dabei liegt auch eine regio- bzw. organunspezifische antientzündliche Wirkung bzw. eine allgemein-systemische antientzündliche Wirkung mit entsprechender Herabregulation entzündlicher Prozesse insgesamt vor, so dass auch auf dieser Basis eine hohe Wirkeffizienz und Wirkbreite resultiert, welche für eine große Anzahl verschiedener entzündlicher Erkrankungen bzw. entzündlicher Reaktionen im menschlichen Körper von Relevanz ist.

Die von der Anmelderin im Rahmen der vorliegenden Erfindung überraschend aufgefundene, spezielle und neue wie erfinderische Anwendung bzw. medizinische Indikation für Cineol, insbesondere 1,8-Cineol, ist im Stand der Technik bislang nicht beschrieben und auch nicht erkannt worden, obwohl es sich bei Cineol, insbesondere 1,8-Cineol, an sich um einen hinlänglichen bekannten Wirkstoff handelt.

Bei dem erfindungsgemäß eingesetzten Wirkstoff Cineol, insbesondere 1,8-Cineol, handelt es sich um ein sogenanntes Terpen, insbesondere Monoterpen. Bei Terpenen handelt es sich im Allgemeinen um Naturstoffe, welche als Bestandteil der sogenannten ätherischen Öle in Form von Flüssigkeiten aus Pflanzen oder deren Bestandteilen isoliert werden können. Sie sind oftmals Duft- und Geschmacksstoffe, welche im Bereich der Lebensmittelindustrie oder der Kosmetikindustrie Anwendung finden. Daneben gewinnt jedoch auch der Einsatz von Terpenen für medizinische Zwecke an Bedeutung, da sich für eine Vielzahl von Terpenen pharmakologische Wirkungen nachweisen lassen. Terpene stellen formal Polymerisationsprodukte des Isoprens dar, wobei nach der Anzahl der Isoprenreste zwischen Monoterpenen (C₁₀-Einheiten), Sesquiterpenen (C₁₅-Einheiten), Diterpenoiden (C₂₀-Einheiten), Sesterterpenen (C₂₅-Einheiten), Triterpenen (C₃₀-Einheiten), Tetraterpenen (C₄₀-Einheiten) und Polyterpenen unterschieden wird (vgl. RÖMPP-Chemielexikon, 10. Auflage, Georg Thieme Verlag, Stuttgart/New York, 1999, Seiten 4449 und 4450, Stichwort "Terpen(oid)e"). Darüber hinaus können Terpene auch als pharmakologische Wirksubstanzen Ausgangsstoffe für die Herstellung von Arzneimitteln oder Vitaminpräparaten sowie aufgrund ihrer oftmals bakterioziden bzw. pestiziden Wirkungen in der Landwirtschaft eingesetzt werden. Speziell für eine Anzahl von Monoterpenen sind pharmakologische Wirkungen in der Bekämpfung von Krankheiten bei erfolgter systemischen Behandlungen nachgewiesen, wobei insbesondere Menthol und Cineol, insbesondere 1,8-Cineol, zu nennen sind.

Speziell der erfindungsgemäß eingesetzte Wirkstoff Cineol, insbesondere 1,8-Cineol, gehört zu den bicyclischen Epoxy-Monoterpenen, genauer gesagt den Limonenoxiden. Synonyme Bezeichnungen für 1,8-Cineol mit der chemischen Summenformel C₁₀H₁₈O sind Eucalyptol, Limonen-1,8-oxid, 1,8-Epoxy-p-menthan oder 1,3,3-Trimethyl-2-oxabicyclo[2.2.2]octan. Es handelt sich um eine farblose Flüssigkeit mit würzigem, campherähnlichem Geruch mit einem Schmelzpunkt von +1,5 °C und mit einem Siedepunkt von 176 bis 177 °C, welche in Wasser unlöslich, aber mit den meisten organischen Lösemitteln mischbar ist.

Natürlich kommt 1,8-Cineol als Hauptbestandteil des Eukalyptusöls (Eukalyptusöl enthält bis zu 85 Gew.-% an 1,8-Cineol), aber auch in anderen Pflanzen vor, so z. B. in Minze, Heilsalbei, Thymian, Basilikum und im Teebaum. Darüber hinaus ist 1,8-Cineol beispielsweise auch in Niaouli-, Juniperus-, Piper-, Cannabis-, Kajeput-, Salbeiöl, Myrtenöl und anderen ätherischen Ölen enthalten.

Technisch bzw. pharmazeutisch aufgereinigtes 1,8-Cineol, welches im Allgemeinen mit 99,6 %-iger bis 99,8 %-iger Reinheit vorliegen kann, wird im Allgemeinen durch fraktionierte Destillation von Eukalyptusöl gewonnen.

1,8-Cineol wird nach dem Stand der Technik sowohl topisch (z. B. inhalativ) als auch systemisch (z. B. in Form von Kapseln), angewendet, meist als Mischöl zusammen mit einer Vielzahl weiterer Terpene.

Für weitergehende Einzelheiten zu dem Wirkstoff 1,8-Cineol kann beispielsweise verwiesen werden auf RÖMPP Chemielexikon, Georg Thieme Verlag, Stuttgart/New York, 10. Auflage, Band 1, 1996, Seite 752, Stichwort: "Cineol", sowie die dort referierte Literatur.

Infolge der Lipophilie des erfindungsgemäß bevorzugt peroral applizierten Cineols, insbesondere 1,8-Cineols, und zwar insbesondere in einer magensaftresistenten bzw. dünndarmlöslichen Form, wie auch nachfolgend noch angeführt, kann zudem eine Langzeitspeicherung in den betreffenden Epithelzellen des Darms angenommen werden, so dass auch eine langfristige und gleichmäßige Versorgung mit dem Cineol, insbesondere 1,8-Cineol, in Bezug auf das Darmsystem gewährleistet ist.

Die definierte Wirksamkeit des erfindungsgemäß eingesetzten Cineols, insbesondere 1,8-Cineols, lässt sich - wiederum ohne sich auf eine spezielle Theorie festlegen zu wollen - möglicherweise auch auf das ergänzend vorliegende und von der Anmelderin überraschend gefundene steroidartige Wirkungspotential von Cineol, insbesondere 1,8-Cineol, zurückführen, insbesondere im Hinblick auf eine weiterführende bzw. ergänzende Hemmung von Entzündungsmediatoren, d. h. Cineol, insbesondere 1 ,8-Cineol, insbesondere als Reinsubstanz, kann zudem ein steroidartiges Wirkungspotential aufweisen. Ätherische Mischöle dagegen (welche Cineol in Gemisch mit weiteren Terpenen und anderen Wirkstoffen enthalten) stimulieren die Prostaglandinproduktion und zeigen gegenüber reinem Cineol, insbesondere reinem 1,8-Cineol, wie es erfindungsgemäß bevorzugt eingesetzt wird, eine nur verminderte Hemmung beispielsweise der Leukotrien- und Zytokinproduktion; denn derartige Mischöle enthalten auch solche Substanzen, welche die Zellaktivität und die Mediatorproduktion stimulieren und daher nicht entzündungshemmend wirken, sondern Unverträglichkeitsreaktionen verursachen können, so dass ätherische Mischöle bzw. Ölgemische folglich im Allgemeinen sogar die Zellaktivität erhöhen und eine Entzündungsmediatorproduktion induzieren können. Im Unterschied hierzu jedoch kann Cineol, insbesondere 1,8-Cineol, insbesondere in Reinform, eine signifikante Hemmung der Mediatorproduktion hervorrufen; auch hierdurch kann somit ein entzündungshemmender Effekt unterstützt werden.

Im Rahmen der vorliegenden Erfindung sind die pathogenen Keime insbesondere ausgewählt aus der Gruppe von entzündungsverursachenden (entzündungsinduzierenden), entzündungsfördernden und mit Entzündungen im menschlichen Körper im Zusammenhang stehenden oder diese hervorrufenden Keimen, insbesondere aus der Gruppe von entzündungsverursachende und/oder entzündungsfördernde Substanzen produzierenden und/oder freisetzenden Keimen, sowie deren Kombinationen.

Somit kann es sich erfindungsgemäß insbesondere um pathogene Keime handeln, welche aufgrund ihrer speziellen Eigenschaften, beispielsweise und in nicht beschränkender Weise im Hinblick auf die Produktion entzündungsfördernder Substanzen oder dergleichen, einen entsprechenden Einfluss auf entzündliche Prozesse im menschlichen Körper aufweisen, insbesondere im Hinblick auf eine eine Entzündung fördernde, hervorrufende oder initiierende Wirkung. Hierbei kann es sich entsprechend auch um von den pathogenen Keimen stammende Entzündungsmediatoren handeln, welche insbesondere auch systemische bzw. darmübergreifende entzündliche Wirkungen entfalten können (beispielsweise durch systemische Resorption dieser Substanzen oder der zugrundeliegenden Keime).

Erfindungsgemäß ist es insbesondere vorgesehen, dass die pathogenen Keime ausgewählt sind aus der Gruppe von Bakterien, insbesondere die Gesundheit negativ beeinträchtigenden und/oder gesundheitsschädlichen Bakterien, vorzugsweise aus der Gruppe von entzündungsverursachenden, entzündungsinduzierenden und mit Entzündungen im menschlichen Körper im Zusammenhang stehenden oder diese hervorrufenden Bakterien, sowie deren Kombinationen.

Gemäß einer erfindungsgemäß bevorzugten Ausführungsform sind die pathogenen Keime ausgewählt aus der Gruppe von Bakterien aus der Familie der *Prevotellaceae*, vorzugsweise aus der Gruppe von Bakterien der Gattung *Prevotella.* Bakterien aus der Familie der *Prevotellaceae* bzw. der Gattung *Prevotella* sind oftmals an Infektionen bzw. an Entzündungsprozessen beteiligt.

Bei Bakterien der Gattung *Prevotella* handelt es sich insbesondere um gramnegative obligat anaerobe Arten, welche pleomorph stäbchenförmig, unbeweglich und sporenlos sind und welche einen chemoorganotrophen Gärungsstoffwechsel aufweisen, dessen Hauptendprodukte Acetat und Succinat sind. Zur Gattung *Prevotella* gehören im Allgemeinen etwa vierzig verschiedene Spezies, welche oftmals verschiedene Entzündungsprozesse, beispielsweise der Mundhöhle oder beispielsweise des Respirationstraktes, verursachen bzw. verstärken können.

Die Anmelderin hat überraschend herausgefunden, dass Bakterien der Familie der *Prevotellaceae*, insbesondere der Bakteriengattung *Prevotella,* bei an *Polyposis nasi* leidenden Patienten in erhöhtem Maße im Darm vorliegt, wobei der diesbezügliche Anteil bzw. die diesbezügliche Anzahl unter Verabreichung von Cineol, vorzugsweise 1,8-Cineol, in gleichermaßen völlig überraschender Weise nachhaltig verringert werden kann, wie nachfolgend noch aufgezeigt.

Im Rahmen der vorliegenden Erfindung kann es sich zudem insbesondere derart verhalten, dass das Cineol, vorzugsweise 1,8-Cineol, des Weiteren bzw. zusätzlich bzw. gleichermaßen zur Erhöhung, Vermehrung oder Unterstützung gesundheitsfördernder oder nichtpathogener, bevorzugt gesundheitsfördernder, Keime im menschlichen Darm, insbesondere in der menschlichen Darmflora, verwendet bzw. eingesetzt wird. Denn die Anmelderin hat ebenso unerwartet wie überraschend gefunden, dass der Anteil bzw. die Anzahl derart positiver Keime im menschlichen Darm unter Cineol- bzw. 1,8-Cineolanwendung deutlich erhöht werden kann.

Diesbezüglich kann somit neben einer Verringerung pathogener Keime auch eine Erhöhung bzw. Vermehrung bzw. Unterstützung gesundheitsfördernder bzw. nichtpathogener Keime durch die gezielte Verabreichung von Cineol, vorzugsweise 1,8-Cineol, erreicht werden, so dass auch auf dieser Basis insgesamt die Zusammensetzung des Mikrobioms des menschlichen Darms bzw. der Darmflora verbessert bzw. normalisiert werden kann. Somit kommt im Rahmen der vorliegenden Erfindung dem eingesetzten Cineol, vorzugsweise 1,8-Cineol, auch von daher insbesondere eine mehrfache Wirkung zu, nämlich einerseits im Hinblick auf eine Verringerung pathogener Keime und einer gezielten Unterstützung gesundheitsfördernder bzw. nichtpathogener Keime im menschlichen Darm.

In diesem Zusammenhang kann es erfindungsgemäß somit vorgesehen sein, dass das Cineol, vorzugsweise 1,8-Cineol, des Weiteren bzw. zusätzlich bzw. gleichermaßen zur Erhöhung der Keimbeladung oder der Keimanzahl oder des Keimanteils, bezogen auf die Gesamtkeimanzahl im menschlichen Darm, insbesondere in der menschlichen Darmflora, gesundheitsfördernder oder nichtpathogener, bevorzugt gesundheitsfördernder, Keime im menschlichen Darm, insbesondere in der menschlichen Darmflora, verwendet oder eingesetzt wird.

Insbesondere kann das Cineol, vorzugsweise 1,8-Cineol, des Weiteren und/oder zusätzlich und/oder gleichermaßen zur Verwendung bei der Erhöhung der Besiedlung des menschlichen Darms, insbesondere der menschlichen Darmflora, mit gesundheitsfördernden oder nichtpathogenen, bevorzugt gesundheitsfördernden, Keimen, verwendet oder eingesetzt werden.

Im Allgemeinen können im Rahmen der vorliegenden Erfindung die gesundheitsfördernden oder nichtpathogenen, bevorzugt gesundheitsfördernden, Keime ausgewählt sein aus der Gruppe von entzündungsverringernden, entzündungshemmenden und entzündungsinhibierenden Keimen, insbesondere aus der Gruppe von entzündungshemmende und entzündungsinhibierende Substanzen produzierenden und/oder freisetzenden Keimen, sowie deren Kombinationen. Insbesondere kann es sich bei den in Rede stehenden gesundheitsfördernden oder nichtpathogenen Keimen um solche Keime handelt, welche antientzündliche Substanzen produzieren oder freisetzen. Auch hierdurch kann es gezielt zu einer Verringerung oder Hemmung entzündlicher Prozesse im menschlichen Körper kommen.

Durch die Verringerung pathogener Keime insbesondere mit proinflammatorischer Wirkung einerseits und die Unterstützung gesundheitsfördernder bzw. nichtpathogener Keime, insbesondere mit antiinflammatorischer Wirkung, andererseits, wird somit im Rahmen der vorliegenden Erfindung ein doppelter Ansatz im Hinblick auf eine entzündungsverringernde bzw. -hemmende bzw. inhibierende Wirkung im Zusammenhang mit der Verabreichung von Cineol, vorzugsweise 1,8-Cineol, erreicht, so dass auch von daher eine sehr hohe Wirkeffizienz vorliegt, und zwar nicht nur im Hinblick auf entzündliche Prozesse bzw. Erkrankungen im menschlichen Darm selbst, sondern darüber hinaus auch in Bezug auf systemische bzw. lokale Entzündungen, welche andere bzw. vom menschlichen Darm verschiedene Körperbereiche des menschlichen Körpers betreffen, wie nachfolgend noch weiter ausgeführt wird.

Gemäß einer erfindungsgemäß bevorzugten Ausführungsform verhält es sich insbesondere derart, dass die gesundheitsfördernden oder nichtpathogenen, bevorzugt gesundheitsfördernden, Keime ausgewählt sind aus der Gruppe von Bakterien, insbesondere die Gesundheit positiv beeinflussenden und/oder gesundheitsförderlichen Bakterien, vorzugsweise aus der Gruppe von entzündungsverringernden, entzündungshemmenden und entzündungsinhibierenden Bakterien, sowie deren Kombinationen.

Insbesondere kann es im Rahmen der vorliegenden Erfindung vorgesehen sein, dass die gesundheitsfördernden oder nichtpathogenen, bevorzugt gesundheitsfördernden, Keime ausgewählt sind aus der Gruppe von Bakterien der Familie der *Ruminococcacea*, vorzugsweise aus der Gruppe von Bakterien der Gattung *Ruminococcus.* Was Bakterien der Familie *Ruminococcacea* bzw. der Gattung *Ruminococcus* anbelangt, so handelt es sich hierbei insbesondere um solche Bakterien, welche antiinflammatorisch wirkende kurzkettige Fettsäuren, wie Butyrat, produzieren, so dass den diesbezüglichen Bakterien eine positive Wirkung im Hinblick auf die Verringerung entzündlicher Prozesse im menschlichen Körper zugesprochen werden kann.

Erfindungsgemäß kann es auch vorgesehen sein, dass die gesundheitsfördernden oder nichtpathogenen, bevorzugt gesundheitsfördernden, Keime ausgewählt sind aus der Gruppe von Bakterien der Familien *Bacteroidaceae, Lachnospiraceae* und *Bifidobacteriaceae*, insbesondere *Bacteroidaceae* und *Lachnospiraceae,* vorzugsweise *Bacteroidaceae*, sowie deren Kombinationen. Insbesondere kann es erfindungsgemäß im Speziellen vorgesehen sein, dass die gesundheitsfördernden oder nichtpathogenen, bevorzugt gesundheitsfördernden, Keime ausgewählt sind aus der Gruppe von Bakterien der Gattung *Bacteroides, Lachnospira* und *Bifidobacterium*, insbesondere *Bacteroides* und *Lachnospira*, vorzugsweise *Bacteroides*, sowie deren Kombinationen. Auch zu den vorgenannten Familien bzw. Gattungen gehörende Bakterienspezies können mit einem positiven Effekt in Bezug auf die menschliche Darmflora bzw. mit antientzündlichen Effekten in Verbindung gebracht werden.

Was das Cineol, vorzugweise 1,8-Cineol, zudem anbelangt, so kann es erfindungsgemäß vorgesehen sein, dass dieses des Weiteren und/oder zusätzlich und/oder gleichermaßen zur Regeneration und/oder Sanierung der Keimbesiedlung des menschlichen Darms, insbesondere der menschlichen Darmflora, verwendet oder eingesetzt wird. Dies geht insbesondere mit einer Verringerung unerwünschter bzw. pathogener Keime bzw. mit einer Erhöhung erwünschter bzw. nichtpathogener, insbesondere gesundheitsfördernder, Keime im menschlichen Darm bzw. in der menschlichen Darmflora einher.

Erfindungsgemäß kann es sich auch derart verhalten, dass das Cineol, vorzugsweise 1,8-Cineol, des Weiteren bzw. zusätzlich bzw. gleichermaßen zur Erhöhung der Keimvielfalt bzw. zur Diversifikation der Keimbesiedlung des menschlichen Darms, insbesondere der menschlichen Darmflora, verwendet oder eingesetzt wird. Dabei geht die Erhöhung der Keimvielfalt bzw. Diversifikation insbesondere mit einer Erhöhung der Anzahl an Spezies der zugrundeliegenden Keime bzw. Bakterien einher, wobei es sich hierbei gleichermaßen insbesondere um gesundheitsfördernde oder nichtpathogene, bevorzugt gesundheitsfördernde, Keime, bzw. Bakterien handelt.

Erfindungsgemäß kann das Cineol, vorzugsweise 1,8-Cineol, insbesondere zur Reduktion der Keimlast und/oder der Keimanzahl der pathogenen Keime im menschlichen Darm und/oder zur Reduktion des Keimanteils der pathogenen Keime, bezogen auf die Gesamtkeimanzahl im menschlichen Darm, insbesondere in der menschlichen Darmflora, verwendet bzw. eingesetzt werden.

In diesem Zusammenhang kann das Cineol, vorzugsweise 1,8-Cineol, insbesondere auch zur Erhöhung der Keimbeladung oder der Keimanzahl oder des Keimanteils, bezogen auf die Gesamtkeimanzahl im menschlichen Darm, gesundheitsfördernder oder nichtpathogener, bevorzugt gesundheitsfördernder, Keime, insbesondere wie zuvor definiert, im menschlichen Darm, insbesondere in der menschlichen Darmflora, verwendet oder eingesetzt werden.

Was das Cineol, vorzugsweise 1,8-Cineol, im Hinblick auf seine erfindungsgemäße Verwendung weiterhin anbelangt, so kann das Cineol, vorzugsweise 1,8-Cineol, des Weiteren und/oder zusätzlich und/oder gleichermaßen als antiinflammatorischer Wirkstoff, insbesondere als systemisch wirkender antiinflammatorischer Wirkstoff, verwendet bzw. eingesetzt werden.

Insbesondere kann das Cineol, vorzugsweise 1,8-Cineol, des Weiteren bzw. zusätzlich bzw. gleichermaßen als Wirkstoff mit antiinflammatorischer Wirkung auf das insbesondere periphere Blut, vorzugsweise mit antiinflammatorischer Wirkung auf Immunzellen des insbesondere peripheren Bluts, verwendet bzw. eingesetzt werden.

Im Hinblick auf die diesbezügliche antiinflammatorische Wirkung kann es sich erfindungsgemäß - ohne sich auf diese Theorie beschränken oder berufen zu wollen - insbesondere um eine gewissermaßen indirekte Wirkung von Cineol, vorzugsweise 1,8-Cineol, handeln, nämlich insofern, als eine Verringerung der Keimlast von pathogenen Keimen und/oder eine Erhöhung der Besiedelung mit gesundheitsfördernden oder nichtpathogenen, bevorzugt gesundheitsfördernden, Keimen des menschlichen Darms, insbesondere der Darmflora, hervorgerufen wird, welche von einer Verbesserung des Zustands des Darmepithels begleitet sein kann, was mit entsprechenden entzündungsmindernden Effekten auch auf systemischer Ebene einhergeht. Wie zuvor angeführt, kann es zudem grundsätzlich der Fall sein, dass überschüssiges Cineol, welches nicht für die antidysbiotische Wirkung verbraucht wird, resorbiert wird und ergänzend einen systemischen Effekt hervorruft.

Erfindungsgemäß kann das Cineol, vorzugsweise 1,8-Cineol, des Weiteren bzw. zusätzlich bzw. gleichermaßen zur Suppression oder Abschwächung einer im Rahmen der entzündlichen Erkrankungen des menschlichen Körpers auftretenden Entzündungsmediation (d.h. Entzündungsvermittlung bzw. Entzündungskaskade) verwendet bzw. eingesetzt werden.

Insbesondere kann das Cineol, vorzugsweise 1,8-Cineol, eine supprimierende oder abschwächende Wirkung gegenüber einer im Rahmen der entzündlichen Erkrankungen des menschlichen Körpers auftretenden Entzündungsmediation ausüben.

Aufgrund der sozusagen universellen bzw. organübergreifenden Wirkung von Cineol, vorzugsweise 1,8-Cineol, insbesondere auf Basis der Verringerung pathogener Keime bzw. der Erhöhung gesundheitsfördernder bzw. nichtpathogener Keime im menschlichen Darm, insbesondere in der menschlichen Darmflora, mit den diesbezüglich systemischen Auswirkungen, können im Rahmen der diesbezüglichen Wirkung erfindungsgemäß eine Vielzahl verschiedener entzündlicher Erkrankungen verringert, vermieden bzw. geheilt werden, da aufgrund der zugrundeliegenden Mechanismen sozusagen ein universelles und im Hinblick auf die konkret zugrundeliegende entzündliche Erkrankung unabhängiges und somit allgemeingültiges Prinzip der Entzündungshemmung vorliegt, nämlich insbesondere im Zusammenhang mit bzw. infolge der gezielten Beeinflussung des menschlichen Darmsystems auf Basis der im menschlichen Darm bzw. in der menschlichen Darmflora vorliegenden Keime bzw. Bakterien.

Erfindungsgemäß kann es sich auch vor diesem Hintergrund insbesondere derart verhalten, dass die entzündlichen Erkrankungen des menschlichen Körpers im Zusammenhang mit den pathogenen Keimen im menschlichen Darm, insbesondere in der menschlichen Darmflora, stehen oder hierdurch verursacht bzw. induziert bzw. verstärkt werden.

Insbesondere kann es sich erfindungsgemäß derart verhalten, dass die entzündlichen Erkrankungen des menschlichen Körpers im Zusammenhang mit einer übermäßigen bzw. abnormen bzw. pathologischen Fehlbesiedlung, insbesondere Überbesiedlung, des menschlichen Darms, insbesondere der menschlichen Darmflora, mit den pathogenen Keimen stehen oder hierdurch verursacht bzw. induziert bzw. verstärkt werden.

Insbesondere kann es sich bei den Erkrankungen auch um solche entzündliche Erkrankungen handeln, welche im Zusammenhang mit einer Dysbiose bzw. einer Fehlbesiedlungen des menschlichen Darms, insbesondere der menschlichen Darmflora, mit pathogenen Keimen stehen bzw. hierdurch verursacht bzw. induziert und/oder verstärkt werden.

Erfindungsgemäß kann es sich im Allgemeinen derart verhalten, dass die entzündlichen Erkrankungen des menschlichen Körpers lokale entzündliche Erkrankungen oder systemische entzündliche Erkrankungen sind.

Erfindungsgemäß kann es sich insbesondere auch derart verhalten, dass die entzündlichen Erkrankungen des menschlichen Körpers akute entzündliche Erkrankungen oder chronische entzündliche Erkrankungen sind. In bevorzugter Weise handelt es sich um chronische entzündliche Erkrankungen.

Wie zuvor angeführt, können die zugrundeliegende Erkrankungen aus einer Vielzahl an Erkrankungen ausgewählt sein. Insbesondere können die entzündlichen Erkrankungen des menschlichen Körpers ausgewählt sein aus der Gruppe von (i) entzündlichen Erkrankungen der oberen Atemwege, insbesondere entzündlichen Erkrankungen der Nase und/oder der Nasennebenhöhlen und/oder des Mund/Rachen-Raums; (ii) entzündlichen Erkrankungen des Hals/Nasen/Ohren-Bereichs; (iii) entzündlichen Erkrankungen der unteren Atemwege, insbesondere entzündlichen Pulmonalerkrankungen (Lungenerkrankungen), entzündlichen Bronchialerkrankungen und entzündlichen bronchopulmonalen Erkrankungen; (iv) entzündlichen Erkrankungen des Gastrointestinaltrakts, insbesondere des Darms; (v) entzündlichen Erkrankungen der Haut; (vi) entzündlichen Erkrankungen des Urogenitaltraktes, insbesondere entzündlichen Erkrankungen der ableitenden Harnwege; (vii) entzündlich-rheumatoiden Erkrankungen, insbesondere Rheuma und Rheumatismus, und Erkrankungen des rheumatischen Formenkreises; und (viii) entzündlichen Erkrankungen (weiterer bzw. anderer) innerer Organe sowie deren Kombinationen.

Insbesondere können die entzündlichen Erkrankungen des menschlichen Körpers ausgewählt sein aus der Gruppe von Rhinitis, Sinusitis, Rhinosinusitis und Nasenpolypen (*Polyposis nasi et sinuum*) sowie deren Kombinationen.

Insbesondere kann es sich bei der zugrundeliegenden Erkrankung beispielsweise um polypöse Sinusitis, insbesondere chronisch polypöse Sinusitis; rezidivierende katarrhalische Sinusitis, insbesondere chronisch rezidivierende katarrhalische Sinusitis, handeln.

Insbesondere kann es sich im Hinblick auf die Rhinosinusitis beispielsweise um Rhinosinusitis mit Nasenpolypen (*Polyposis nasi et sinuum*) (RSwNP), insbesondere um eine chronische Rhinosinusitis mit Nasenpolypen (*Polyposis nasi et sinuum*) (CRSwNP), handeln. Grundsätzlich kann es sich erfindungsgemäß aber auch um Rhinosinusitis ohne Nasenpolypen (RSsNP) bzw. um eine chronische Rhinosinusitis ohne Nasenpolypen (CRSsNP) handeln.

Erfindungsgemäß können die entzündlichen Erkrankungen des menschlichen Körpers auch ausgewählt sein aus der Gruppe von entzündlichen Erkrankungen des Ohres, vorzugsweise Otitis, bevorzugt Otitis media, sowie deren Kombinationen. Hierbei kann es sich insbesondere um eine polypöse Otitis media, insbesondere um eine chronisch polypöse Otitis media handeln. Insbesondere kann es sich hierbei auch um eine chronisch polypöse Otitis media mit Cholesteatom oder ohne Cholesteatom handeln.

Weiterhin können die entzündlichen Erkrankungen des menschlichen Körpers ausgewählt sein aus der Gruppe von Bronchitis, Asthma bronchiale und chronischen obstruktiven Lungenerkrankungen (COPD) sowie deren Kombinationen.

Insbesondere können die entzündlichen Erkrankungen des menschlichen Körpers ausgewählt sein aus der Gruppe von *Morbus Crohn*, *Colitis ulcerosa* und chronisch entzündlichen Darmerkrankungen, wie *Inflammatory Bowel Disease* (IBD), sowie deren Kombinationen.

Erfindungsgemäß kann es zudem vorgesehen sein, dass die entzündlichen Erkrankungen des menschlichen Körpers ausgewählt sind aus der Gruppe von Ekzemen und Dermatitis sowie deren Kombinationen.

Die entzündlichen Erkrankungen des menschlichen Körpers können auch ausgewählt sein aus der Gruppe von Glomerulonephritis, Pyelonephritis, Cystitis und Uritritis, sowie deren Kombinationen.

Gleichermaßen kann es erfindungsgemäß der Fall sein, dass die entzündlichen Erkrankungen ausgewählt sind aus der Gruppe von entzündlichen Erkrankungen der Gallenwege, insbesondere Cholecystitis und Cholangitis, sowie deren Kombinationen.

Gemäß einer besonderen Ausführungsform der vorliegenden Erfindung gemäß diesem Erfindungsaspekt ist es insbesondere vorgesehen, dass das Cineol, vorzugsweise 1,8-Cineol, systemisch, insbesondere peroral oder parenteral, vorzugsweise peroral, appliziert wird. In diesem Zusammenhang kann es insbesondere vorgesehen sein, dass das Cineol, vorzugsweise 1,8-Cineol, zur systemischen, insbesondere peroralen oder parenteralen, vorzugsweise peroralen, Applikation hergerichtet ist. Auf diese Weise werden hohe Wirkdosen bzw. Wirkstoffpegel (d. h. Wirkstoffspiegel bzw. -konzentrationen) insbesondere im Bereich des Darms erreicht, so dass eine besonders gute Wirksamkeit bzw. Effizienz realisiert werden kann.

Insbesondere kann das Cineol, vorzugsweise 1,8-Cineol, in Form einer peroral zu verabreichenden Darreichungsform appliziert wird und/oder wobei das Cineol, vorzugsweise 1,8-Cineol, in einer peroral zu verabreichenden Darreichungsform hergerichtet sein.

Gemäß einer wiederum besonderen Ausführungsform der vorliegenden Erfindung gemäß diesem Aspekt kann es in diesem Zusammenhang insbesondere auch vorgesehen sein, dass das Cineol, vorzugsweise 1,8-Cineol, als magensaftresistente, aber dünndarmlösliche systemische Darreichungsform, bevorzugt als Kapsel, Dragee, Pille, Tablette oder dergleichen, appliziert wird. Insbesondere kann das Cineol, vorzugsweise 1,8-Cineol, zur Verabreichung als magensaftresistente, aber dünndarmlösliche systemische Darreichungsform, bevorzugt Kapsel, Dragee, Pille, Tablette oder dergleichen, hergerichtet sein. Durch die magensaftresistente bzw. dünndarmlösliche systemische Darreichungsform kann die Wirkdosis bzw. der Wirkstoffpegel im Bereich des Darms aufgrund der gezielten Freisetzung erst "vor Ort" nochmals erhöht werden. Im Allgemeinen ist es im Rahmen der vorliegenden Erfindung somit vorgesehen, dass die perorale Darreichungsform, insbesondere in Form einer Kapsel, magensaftresistent, aber dünndarmlöslich ausgebildet ist. Hierdurch wird ein besonders optimales Freisetzungsprofil erreicht, da der Wirkstoff ziel- und zweckgerichtet erst im Darm freigesetzt wird.

Die im Rahmen der vorliegenden Erfindung verwendeten Definitionen für die Begriffe "magensaftresistent" bzw. "dünndarmlöslich" sowie die diesbezüglichen Testmethoden sind in European Pharmacopoeia 7.0, 04/2010: 1502, Seiten 707 bis 709, Stichwort "Capsules", Unterkapitel "Gastro-Resistant Capsules" sowie European Pharmacopoeia 7.1, 04/2011: 20901, Seiten 3331 und 3332, Stichwort "2.9.1 Disintegration of Tablets and Capsules*"* wiedergegeben.

Unter dem Begriff "magensaftresistent" ist dabei im Rahmen der vorliegenden Erfindung insbesondere zu verstehen, dass die Kapseln mindestens zwei Stunden lang in 0,1 N Salzsäure, welche auf Temperaturen von 35 bis 39 °C erwärmt wird, unter ständiger Durchmischung aufbewahrt, insbesondere gerührt werden kann, ohne dass die Kapseln Anzeichen einer Zersetzung bzw. Risse oder andere Beschädigungen aufweisen.

Unter dem Begriff "dünndarmlöslich" ist im Rahmen der vorliegenden Erfindung hingegen insbesondere zu verstehen, dass die Kapseln in einer wässrigen Phosphatpuffer-Lösung, welche auf einen pH-Wert von etwa 6,8 eingestellt, unter Rühren bei Temperaturen im Bereich von 35 bis 39 °C innerhalb einer Stunde so weit zersetzt werden, dass die Wirksubstanz freigesetzt wird.

Für die diesbezügliche Anwendung von Cineol, insbesondere 1,8-Cineol, sind im Handel verschiedene Präparate, insbesondere auf Basis von im Allgemeinen magensaftresistenten, aber dünndarmlöslichen Darreichungsformen bzw. Kapseln, verfügbar (z. B. Soledum^{®}-Kapseln bzw. Soledum^{®} forte-Kapseln, vertrieben von der Cassella-med GmbH & Co. KG bzw. der Maria Clementine Martin Klosterfrau Vertriebsgesellschaft mbH, jeweils Köln, Deutschland).

Erfindungsgemäß kann es vorgesehen sein, dass das Cineol, vorzugsweise 1,8-Cineol, in pharmazeutisch wirksamen bzw. therapeutisch wirksamen Mengen verabreicht wird und/oder dass das Cineol, vorzugsweise 1,8-Cineol, zur Verabreichung in pharmazeutisch wirksamen bzw. therapeutisch wirksamen Mengen hergerichtet ist.

Im Rahmen der vorliegenden Erfindung ist es insbesondere vorgesehen, dass das Cineol, vorzugsweise 1,8-Cineol, mit einer Tagesdosis im Bereich von 1 bis 5.000 mg/diem, insbesondere im Bereich von 2 bis 3.000 mg/diem, vorzugsweise im Bereich von 5 bis 2.500 mg/diem, bevorzugt im Bereich von 10 bis 2.000 mg/diem, besonders bevorzugt im Bereich von 50 bis 1.500 mg/diem, verabreicht wird und/oder wobei das Cineol, vorzugsweise 1,8-Cineol, zur Verabreichung mit einer Tagesdosis im Bereich von 1 bis 5.000 mg/diem, insbesondere im Bereich von 2 bis 3.000 mg/diem, vorzugsweise im Bereich von 5 bis 2.500 mg/diem, bevorzugt im Bereich von 10 bis 2.000 mg/diem, besonders bevorzugt im Bereich von 50 bis 1.500 mg/diem, hergerichtet ist.

Insbesondere kann es im Rahmen der vorliegenden Erfindung gemäß diesem Erfindungsaspekt nach einer besonderen Ausführungsform auch vorgesehen sein, dass das Cineol, vorzugsweise 1,8-Cineol, als Reinstoff vorliegt bzw. verabreicht wird. In diesem Zusammenhang kann das Cineol, vorzugsweise 1,8-Cineol, mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 Gew.-%, bezogen auf das Cineol, vorzugsweise 1,8-Cineol, vorliegt bzw. verabreicht werden. Insbesondere kann es in diesem Zusammenhang auch vorgesehen sein, dass das Cineol, vorzugsweise 1,8-Cineol, frei von anderen Terpenen ist bzw. dass das Cineol, vorzugsweise 1,8-Cineol, keine anderen Terpene enthält.

Mit anderen Worten kann es im Rahmen der vorliegenden Erfindung gemäß diesem Erfindungsaspekt nach einer besonderen Ausführungsform insbesondere vorgesehen sein, dass das Cineol, vorzugsweise 1,8-Cineol, in Abwesenheit von anderen (weiteren) Terpenen vorliegt und/oder verabreicht wird.

Gemäß einer erfindungsgemäß besonderen Ausführungsform kann es also insbesondere vorgesehen sein, dass das Cineol, vorzugsweise 1,8-Cineol, als Reinstoff und/oder in Abwesenheit von anderen (weiteren) Terpenen vorliegt und/oder verabreicht wird.

Wie vorstehend geschildert, kann es also erfindungsgemäß bevorzugt sein, dass die monoterpenbasierte Wirksubstanz (d. h. Cineol, vorzugsweise 1,8-Cineol) in Form einer Reinsubstanz, d. h. als alleiniger bzw. technisch isolierter/aufgereinigter Wirkstoff bzw. frei von anderen Terpenen, eingesetzt wird. Auf diese Weise kann beispielsweise eine immer gleichbleibende Dosierung bzw. ein immer gleicher Gehalt an Wirksubstanz in der erfindungsgemäß vorgesehen Darreichungsform gewährleistet werden. Insbesondere sind auf diese Weise hohe Wirkstoffdosen bzw. -pegel auch am Wirkort realisierbar. Infolge der erfindungsgemäß bevorzugten Verwendung von Cineol, insbesondere 1,8-Cineol, in Reinform bzw. frei von anderen Terpenen, wird zudem auch ein besonders definiertes Wirkprofil im Hinblick auf die zugrundeliegende Indikation gewährleistet. Wie zuvor angeführt, wird durch die Verabreichung in Reinform auch ein definiertes steroidartiges Wirkprofil bereitgestellt.

Gemäß einer wiederum weiteren Ausführungsform der vorliegenden Erfindung kann es vorgesehen sein, dass das Cineol, vorzugsweise 1,8-Cineol, zusammen mit mindestens einem physiologisch unbedenklichen Träger (Exzipienten), vorliegt bzw. verabreicht wird.

Gemäß einer wiederum weiteren Ausführungsform der vorliegenden Erfindung kann es vorgesehen sein, dass das Cineol, vorzugsweise 1,8-Cineol, bevorzugt als Reinstoff und/oder in Abwesenheit von anderen (weiteren) Terpenen, zusammen mit mindestens einem physiologisch unbedenklichen Träger (Exzipienten), vorliegt bzw. verabreicht wird.

Insbesondere kann es dabei vorgesehen sein, dass der physiologisch unbedenkliche Träger (Exzipient) mit Cineol bzw. 1,8-Cineol mischbar und/oder hierin löslich ist.

Insbesondere kann der physiologisch unbedenkliche Träger (Exzipient) bei 20 °C und bei Atmosphärendruck im flüssigen oder festen, vorzugsweise flüssigen, Aggregatzustand vorliegen.

Wiederum weiterhin kann es in diesem Zusammenhang vorgesehen sein, dass der physiologisch unbedenkliche Träger (Exzipient) ausgewählt ist aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren.

Speziell mittelkettige Triglyceride, wie sie erfindungsgemäß bevorzugt als Träger bzw. Exzipient für die monoterpenhaltige Wirksubstanz zum Einsatz kommen, sind insbesondere halbsynthetische neutrale Glycerinester von gesättigten, im Allgemeinen unverzweigten Monocarbonsäuren mittlerer Kettenlänge (d. h. C₆-C₁₂-Ketten). Insbesondere bezeichnet der Begriff der mittelkettigen Triglyceride Gemische von Triglyceriden gesättigter Fettsäuren, hauptsächlich Caprylsäure (Octansäure) und Caprinsäure (Decansäure). Mittelkettige Triglyceride können im Allgemeinen aus Öl hergestellt werden, welches aus dem festen und getrockneten Teil des Endosperms von *Cocos nucifera* L. und/oder aus dem getrockneten Endosperm von *Elaeis guineenses* Jacq. extrahiert wird. Für weitergehende Einzelheiten zu dem Begriff der mittelkettigen Triglyceride kann beispielsweise verwiesen werden auf die Monographie Ph. Eur., 6. Ausgabe, Grundwerk 2008, Seiten 4224 bis 4226, sowie auf die Zeitschrift für Ernährungswissenschaft, Band 13, Heft 1/2, 1973, Seiten 6 ff., D. Sailer et al. "Mittelkettige Triglyceride - Klinische Physiologie und Anwendung").

Erfindungsgemäß bevorzugt ist es, wenn der Träger bzw. Exzipient in einem Wirksubstanz/Träger-Mengenverhältnis im Bereich von 1.000 : 1 bis 1 : 1.000, insbesondere 100:1 bis 1 : 100, vorzugsweise 50 : 1 bis 1 :50, besonders bevorzugt 10 : 1 bis 1 : 10, ganz besonders bevorzugt 5 : 1 bis 1 : 2, noch mehr bevorzugt 3 : 1 bis 1 : 1, eingesetzt wird. Durch die Mischung der Wirksubstanz (d. h. Cineol, insbesondere 1,8-Cineol) mit dem Exzipienten werden eine deutlich verbesserte Kompatibilität der Wirksubstanz mit den weiteren Inhaltsstoffen der Darreichungsform und eine verbesserte Stabilität, insbesondere Lagerstabilität, erreicht.

Gemäß einer gleichermaßen weiteren besonderen Ausführungsform der vorliegenden Erfindung kann es vorgesehen sein, dass das Cineol, vorzugsweise 1,8-Cineol, in bzw. in Form einer Zusammensetzung, insbesondere pharmazeutischen Zusammensetzung, insbesondere zusammen mit mindestens einem pharmazeutisch verträglichen und/oder physiologisch unbedenklichen Exzipienten (Träger), vorliegt und/oder verabreicht wird.

In diesem Zusammenhang kann es insbesondere vorgesehen sein, dass die Zusammensetzung das Cineol, vorzugsweise 1,8-Cineol, als alleinigen Wirkstoff, insbesondere als alleinigen pharmazeutischen Wirkstoff, enthält.

Weiterhin kann es dabei insbesondere vorgesehen sein, dass die Zusammensetzung das Cineol, vorzugsweise 1,8-Cineol, als Reinstoff enthält, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 Gew.-%, bezogen auf das Cineol, vorzugsweise 1,8-Cineol, und/oder vorzugsweise frei von anderen Terpenen.

Gleichermaßen kann es dabei auch vorgesehen sein, dass die Zusammensetzung das Cineol, vorzugsweise 1,8-Cineol, zumindest im Wesentlichen frei von anderen Terpenen enthält und/oder insbesondere wobei die Zusammensetzung zumindest im Wesentlichen kein weiteres Terpen enthält und/oder insbesondere wobei die Zusammensetzung zumindest im Wesentlichen frei von anderen Terpenen als Cineol, vorzugsweise 1,8-Cineol, ist.

Weiterhin kann es dabei vorgesehen sein, dass die Zusammensetzung das Cineol, vorzugsweise 1,8-Cineol, in wirksamen, insbesondere pharmazeutisch und/oder therapeutisch wirksamen, Mengen enthält.

Ebenfalls kann es in diesem Kontext erfindungsgemäß vorgesehen sein, dass die Zusammensetzung das Cineol, vorzugsweise 1,8-Cineol, bezogen auf die Zusammensetzung, in relativen Mengen im Bereich von 0,0001 bis 80 Gew.-%, insbesondere 0,001 bis 75 Gew.-%, vorzugsweise 0,005 bis 70 Gew.-%, bevorzugt 0,01 bis 60 Gew.-%, besonders bevorzugt 0,05 bis 55 Gew.-%, ganz besonders bevorzugt 0,1 bis 50 Gew.-%, enthält.

Schließlich kann es erfindungsgemäß in diesem Zusammenhang auch vorgesehen sein, dass der mindestens eine pharmazeutisch verträgliche und/oder physiologisch unbedenkliche Exzipient (Träger) mit Cineol bzw. 1,8-Cineol mischbar und/oder hierin löslich ist, vorzugsweise wobei der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegt und/oder vorzugsweise wobei der mindestens eine pharmazeutisch verträgliche und/oder physiologisch unbedenkliche Exzipient (Träger) ausgewählt ist aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren.

Gemäß einer besonderen Ausführungsform der vorliegenden Erfindung kann das Cineol, vorzugsweise 1,8-Cineol, in liposomenumgebener und/oder liposomal verpackter Form vorliegt und/oder verabreicht werden. Hierdurch ist ein besonders gutes Freisetzungsprofil gewährleistet.

Gemäß einer weiteren besonderen Ausführungsform der vorliegenden Erfindung kann das Cineol, vorzugsweise 1,8-Cineol, in micellierter Form bzw. in einer micellaren Darreichungsform vorliegen bzw. verabreicht werden.

Erfindungsgemäß kann es weiterhin vorgesehen sein, dass wobei das Cineol, vorzugsweise 1,8-Cineol, als Einzelwirkstoff bzw. als Monopräparat vorliegt bzw. verabreicht wird.

Erfindungsgemäß kann es in diesem Zusammenhang vorgesehen sein, dass das Cineol, vorzugsweise 1,8-Cineol, ohne bzw. in Abwesenheit von weiteren, von Cineol, vorzugsweise 1,8-Cineol, verschiedenen Wirkstoffen vorliegt bzw. verabreicht wird.

Demgegenüber kann es erfindungsgemäß aber auch vorgesehen sein, dass das Cineol, vorzugsweise 1,8-Cineol, zusammen mit mindestens einem weiteren Wirkstoff und/oder als Co-Therapeutikum vorliegt bzw. verabreicht wird.

In diesem Zusammenhang kann es insbesondere vorgesehen sein, dass der weitere Wirkstoff ausgewählt ist aus (i) entzündungshemmenden Wirkstoffen (Antiphlogistika), insbesondere nichtsteroidalen Entzündungshemmern (NSAIDs) oder steroidalen Entzündungshemmern, vorzugsweise steroidalen Entzündungshemmern, bevorzugt Kortikosteroiden; (ii) Antibiotika, (iii) Präbiotika; und (iv) die Darmflora positiv beeinflussenden Mikroorganismen; sowie deren Kombinationen.

Gemäß einer besonders bevorzugten Ausführungsform kann es dabei insbesondere vorgesehen sein, dass der weitere Wirkstoff getrennt, insbesondere räumlich getrennt, von dem Cineol, vorzugsweise 1,8-Cineol, aber funktional zusammenhängend hiermit eingesetzt bzw. verabreicht wird, insbesondere in Form eines Kit (Kit-of-part).

Auf die vorgenannte Art und Weise (d. h. Kombination oder gemeinsame Verabreichung mit mindestens einem weiteren Wirkstoff) kann insbesondere die Wirkung bzw. Effizienz des weiteren Wirkstoffs gesteigert werden. Gleichzeitig kann durch das Zusammenwirken die Sensitivität auch gegenüber dem weiteren Wirkstoff signifikant gesteigert werden, wie es beispielsweise für entzündungshemmende Wirkstoffe der Fall ist. Gleichermaßen kann die Wirkeffizienz beispielsweise durch kombinierte Verabreichung mit Präbiotika bzw. die Darmflora positiv beeinflussende Mikroorganismen verbessert werden, da hierdurch die Darmflora weiterführend beeinflusst bzw. aufgebaut werden kann, und zwar im Hinblick auf die Bevorzugung von nichtpathogenen bzw. gesundheitsfördernden Keimen bzw. im Hinblick auf eine weiterführende gezielte Verringerung pathogener Keime (wie es beispielsweise auch für den Einsatz vorzugsweise spezieller Antibiotika grundsätzlich so der Fall sein kann).

Insbesondere kann das Cineol, vorzugsweise 1,8-Cineol, zusammen mit mindestens einem Präbiotikum vorliegen bzw. verabreicht werden. In diesem Zusammenhang kann das Präbiotikum eine Substanz sein, welche insbesondere spezifisch das Wachstum von die Darmflora positiv beeinflussenden Mikroorganismen fördert. In diesem Kontext kann es sich zudem derart verhalten, dass das Präbiotikum ausgewählt ist aus der Gruppe von insbesondere natürlichen Polysacchariden, insbesondere natürlichen Oligosacchariden, und Zuckeralkoholen.

Insbesondere kann das Präbiotikum Gummi arabicum sein.

Gleichermaßen kann das Präbiotikum in diesem Zusammenhang ausgewählt sein aus der Gruppe von Fructooligosacchariden und Galaktooligosacchariden, insbesondere jeweils in Form von Tri- bis Pentasacchariden, und deren Kombinationen. Insbesondere kann das Präbiotikum ausgewählt sein aus der Gruppe von Inulin, Sucrose, Stachyose, Raffinose, Lactulose und deren Kombinationen.

Weiterhin kann es im Rahmen der vorliegenden Erfindung vorgesehen sein, dass das Cineol, vorzugsweise 1,8-Cineol, zusammen mit die Darmflora positiv beeinflussenden Mikroorganismen vorliegt bzw. verabreicht wird. Hierbei kann es sich insbesondere um gesundheitsfördernde bzw. nichtpathogene Mikroorganismen als solche handeln. Beispielsweise können die Mikroorganismen ausgewählt sein aus der Gruppe von Hefen und Bakterien. In diesem Kontext kann es erfindungsgemäß insbesondere vorgesehen sein, dass die Mikroorganismen ausgewählt sind aus der Gruppe von Spezies der Gattung *Saccharomyces*, insbesondere *Saccharomyces boulardii* und *Saccharomyces cervesiae*, vorzugsweise *Saccharomyces boulardii*, sowie deren Kombinationen. Gleichermaßen kann es erfindungsgemäß in diesem Zusammenhang vorgesehen sein, dass die Mikroorganismen ausgewählt sind aus der Gruppe von Spezies der Gattung *Bifidobacterium, Lactobacillus, Enterococcus, Escherichia, Streptococcus,* insbesondere jeweils in Form von deren die Darmflora positiv beeinflussenden Spezies, und deren Kombinationen.

Im Allgemeinen können die Mikroorganismen als Trockensubstanz, insbesondere biologisch aktive Trockensubstanz, insbesondere in lyophilisierter Form und/oder als Lyophilisat, vorliegen bzw. verabreicht werden. Dies ist insbesondere der Handhabung bei gleichzeitig hoher Wirkeffizienz zuträglich.

Die vorliegende Erfindung betrifft zudem gemäß dem vorliegenden Aspekt auch Cineol, vorzugsweise 1,8-Cineol, insbesondere Cineol zur Verwendung wie zuvor definiert, zur Verwendung bei der prophylaktischen bzw. therapeutischen Behandlung von Dysbiose bzw. von Fehlbesiedlungen des menschlichen Darms, insbesondere der menschlichen Darmflora, mit pathogenen Keimen.

Insbesondere kann im Rahmen der Behandlung von Dysbiose bzw. von Fehlbesiedlungen des menschlichen Darms, insbesondere der menschlichen Darmflora, mit pathogenen Keimen eine Regeneration und/oder Sanierung des Mikrobioms des menschlichen Darms, insbesondere der menschlichen Darmflora, durchgeführt werden.

Auch diesbezüglich kann im Rahmen der vorliegenden Erfindung eine Optimierung bzw. Regeneration der Darmflora erreicht werden, und zwar insbesondere auch vor dem Hintergrund bzw. zu Zwecken der Vermeidung, Verringerung oder Heilung entzündlicher Erkrankungen des menschlichen Körpers bzw. insbesondere zu Zwecken der prophylaktischen und/oder therapeutischen Behandlung entzündlicher Erkrankungen des menschlichen Körpers. Auch in diesem Zusammenhang kommt dem Cineol, insbesondere 1,8-Cineol, eine antidysbiotische Wirkung zu. Folglich kann Cineol, insbesondere 1,8-Cineol, diesbezüglich auch als Antidysbiotikum fungieren.

Die vorliegende Erfindung, und zwar sowohl gemäß dem ersten Aspekt der vorliegenden Erfindung als auch gemäß sämtlichen übrigen Aspekten der vorliegenden Erfindung, ist somit mit einer Vielzahl von Vorteilen und Besonderheiten verbunden, welche das erfindungsgemäße Therapiekonzept einzigartig und besonders, insbesondere hocheffizient, machen, und dies bei gleichzeitig guter Verträglichkeit und einfacher Anwendbarkeit der erfindungsgemäß vorliegenden bzw. eingesetzten Wirksubstanz.

Für weitergehende Einzelheiten zu diesem Erfindungsaspekt kann auch Bezug genommen werden auf die nachstehenden Ausführungen zu den weiteren Erfindungsaspekten, wobei diese Ausführungen gleichermaßen für den vorliegenden Erfindungsaspekt gelten.

Weiterer Gegenstand der vorliegenden Erfindung - gemäß einem **zweiten** Aspekt der vorliegenden Erfindung - ist zudem die erfindungsgemäße Verwendung von Cineol, vorzugsweise 1,8-Cineol, zur Verringerung pathogener Keime im menschlichen Darm, insbesondere in der menschlichen Darmflora, und/oder zur Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers.

Im Rahmen der erfindungsgemäßen Verwendung gemäß diesem Erfindungsaspekt ist es insbesondere vorgesehen, dass der Wirkstoff bzw. das Cineol, vorzugsweise 1,8-Cineol, peroral appliziert wird, und zwar insbesondere in Form einer magensaftresistenten, jedoch dünndarmlöslichen Darreichungsform bzw. Zusammensetzung.

Gemäß dem vorliegenden Aspekt betrifft die vorliegende Erfindung zudem auch die Verwendung von Cineol, vorzugsweise 1,8-Cineol, zur Herstellung eines Arzneimittels oder Medikaments zur Verringerung pathogener Keime im menschlichen Darm, insbesondere in der menschlichen Darmflora, und/oder zur Herstellung eines Arzneimittels oder Medikaments zur Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers.

Gleichermaßen betrifft die vorliegende Erfindung gemäß dem vorliegenden Aspekt auch die Verwendung von Cineol, vorzugsweise 1,8-Cineol, insbesondere eine wie vorliegend definierte Verwendung, (zur Herstellung eines Arzneimittels) zur prophylaktischen und/oder therapeutischen Behandlung von Dysbiose und/oder von Fehlbesiedlungen des menschlichen Darms, insbesondere der menschlichen Darmflora, mit pathogenen Keimen.

Diesbezüglich kann das Cineol, vorzugsweise 1,8-Cineol, insbesondere das Arzneimittel oder Medikament in pharmazeutisch wirksamen bzw. therapeutisch wirksamen Mengen verabreicht werden.

Insbesondere kann das Cineol, vorzugsweise 1,8-Cineol, insbesondere das Arzneimittel oder Medikament zur Verabreichung in pharmazeutisch wirksamen bzw. therapeutisch wirksamen Mengen hergerichtet sein.

Zudem kann das Cineol, vorzugsweise 1,8-Cineol, insbesondere das Arzneimittel oder Medikament, systemisch appliziert werden.

Erfindungsgemäß kann in diesem Zusammenhang das Cineol, vorzugsweise 1,8-Cineol, insbesondere das Arzneimittel oder Medikament oder die Zusammensetzung, zusammen mit mindestens einem weiteren Wirkstoff angewendet oder appliziert wird, insbesondere wobei der weitere Wirkstoff ausgewählt ist aus der Gruppe von (i) entzündungshemmenden Wirkstoffen (Antiphlogistika), insbesondere nichtsteroidalen Entzündungshemmern (NSAIDs) oder steroidalen Entzündungshemmern, vorzugsweise steroidalen Entzündungshemmern, bevorzugt Kortikosteroiden; (ii) Antibiotika, (iii) Präbiotika; und (iv) die Darmflora positiv beeinflussenden Mikroorganismen; sowie deren Kombinationen.

Für weitergehende Einzelheiten zu diesem Erfindungsaspekt kann Bezug genommen werden auf die Ausführungen zu den weiteren erfindungsgemäßen Aspekten, wobei diese Ausführungen gleichermaßen für den vorliegenden Erfindungsaspekt gelten.

Gleichermaßen ist Gegenstand der vorliegenden Erfindung - gemäß einem **dritten** Aspekt der vorliegenden Erfindung - die erfindungsgemäße Verwendung von Cineol, vorzugsweise 1,8-Cineol, als Darmbehandlungsmittel zur Verringerung pathogener Keime im menschlichen Darm, insbesondere in der menschlichen Darmflora; und/oder zur Verwendung bei der Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers; und/oder zur prophylaktischen und/oder therapeutischen Behandlung von Dysbiose und/oder von insbesondere bakteriellen Fehlbesiedlungen des menschlichen Darms, insbesondere der menschlichen Darmflora.

Diesbezüglich kann das Cineol, vorzugsweise 1,8-Cineol, in pharmazeutisch wirksamen bzw. therapeutisch wirksamen Mengen verabreicht werden.

Insbesondere kann das Cineol, vorzugsweise 1,8-Cineol, zur Verabreichung in pharmazeutisch wirksamen bzw. therapeutisch wirksamen Mengen hergerichtet sein.

Zudem kann das Cineol, vorzugsweise 1 ,8-Cineol, systemisch appliziert werden.

Erfindungsgemäß kann in diesem Zusammenhang das Cineol, vorzugsweise 1,8-Cineol, oder die Zusammensetzung, zusammen mit mindestens einem weiteren Wirkstoff angewendet oder appliziert wird, insbesondere wobei der weitere Wirkstoff ausgewählt ist aus der Gruppe von (i) entzündungshemmenden Wirkstoffen (Antiphlogistika), insbesondere nichtsteroidalen Entzündungshemmern (NSAIDs) oder steroidalen Entzündungshemmern, vorzugsweise steroidalen Entzündungshemmern, bevorzugt Kortikosteroiden; (ii) Antibiotika, (iii) Präbiotika; und (iv) die Darmflora positiv beeinflussenden Mikroorganismen; sowie deren Kombinationen.

Für weitergehende Einzelheiten zu diesem Erfindungsaspekt kann Bezug genommen werden auf die Ausführungen zu den weiteren erfindungsgemäßen Aspekten, wobei diese Ausführungen gleichermaßen für den vorliegenden Erfindungsaspekt gelten.

Ebenfalls Gegenstand der vorliegenden Erfindung - gemäß einem **vierten** Aspekt der vorliegenden Erfindung ist das erfindungsgemäße Verfahren zur Verringerung pathogener Keime im menschlichen Darm, insbesondere in der menschlichen Darmflora, bzw. zur der Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers, wobei bei dem Verfahren einem insbesondere eine Fehlbesiedlung, insbesondere Überbesiedlung, des Darms, insbesondere der Darmflora, mit den pathogenen Keimen aufweisenden menschlichen Patienten eine die Keimlast und/oder die Keimanzahl der pathogenen Keime im Darm verringernde und/oder absenkende Menge an Cineol, vorzugsweise 1,8-Cineol, verabreicht wird; und/oder wobei bei dem Verfahren einem insbesondere eine Fehlbesiedlung, insbesondere Überbesiedlung, des Darms, insbesondere der Darmflora, mit den pathogenen Keimen aufweisenden menschlichen Patienten eine eine Reduktion des Keimanteils der pathogenen Keime, bezogen auf die Gesamtkeimanzahl im Darm, insbesondere in der Darmflora, hervorrufende Menge an Cineol, vorzugsweise 1,8-Cineol, verabreicht wird; und/oder wobei bei dem Verfahren einem insbesondere eine Fehlbesiedlung, insbesondere Überbesiedlung, des Darms, insbesondere der Darmflora, mit den pathogenen Keimen aufweisenden menschlichen Patienten eine pharmazeutisch wirksame bzw. therapeutisch wirksame Menge an Cineol, vorzugsweise 1,8-Cineol, verabreicht wird.

Diesbezüglich kann das Cineol, vorzugsweise 1,8-Cineol, in pharmazeutisch wirksamen bzw. therapeutisch wirksamen Mengen verabreicht werden.

Insbesondere kann das Cineol, vorzugsweise 1,8-Cineol, zur Verabreichung in pharmazeutisch wirksamen bzw. therapeutisch wirksamen Mengen hergerichtet sein.

Zudem kann das Cineol, vorzugsweise 1 ,8-Cineol, systemisch appliziert werden.

Erfindungsgemäß kann in diesem Zusammenhang das Cineol, vorzugsweise 1,8-Cineol, oder die Zusammensetzung, zusammen mit mindestens einem weiteren Wirkstoff angewendet oder appliziert wird, insbesondere wobei der weitere Wirkstoff ausgewählt ist aus der Gruppe von (i) entzündungshemmenden Wirkstoffen (Antiphlogistika), insbesondere nichtsteroidalen Entzündungshemmern (NSAIDs) oder steroidalen Entzündungshemmern, vorzugsweise steroidalen Entzündungshemmern, bevorzugt Kortikosteroiden; (ii) Antibiotika, (iii) Präbiotika; und (iv) die Darmflora positiv beeinflussenden Mikroorganismen; sowie deren Kombinationen.

Gemäß diesem Aspekt betrifft die vorliegende Erfindung auch das erfindungsgemäße Verfahren, insbesondere das zuvor definierte Verfahren, zur prophylaktischen bzw. therapeutischen Behandlung von Dysbiose und/oder von Fehlbesiedlungen des menschlichen Darms, insbesondere der menschlichen Darmflora, mit pathogenen Keimen, wobei bei dem Verfahren einem die Dysbiose und/oder die Fehlbesiedlung aufweisenden und/oder hierunter leidenden Patienten, insbesondere einem die Fehlbesiedlung, insbesondere Überbesiedlung, des Darms, insbesondere der Darmflora, mit den pathogenen Keimen aufweisenden menschlichen Patienten und/oder einem unter Dysbiose leidenden Patienten eine pharmazeutisch wirksame bzw. therapeutisch wirksame Menge an Cineol, vorzugsweise 1,8-Cineol, verabreicht wird.

Für weitergehende Einzelheiten zu diesem Erfindungsaspekt kann Bezug genommen werden auf die Ausführungen zu den weiteren Erfindungsaspekten, wobei diese Ausführungen gleichermaßen für den vorliegenden Erfindungsaspekt gelten.

Ebenfalls Gegenstand der vorliegenden Erfindung - gemäß einem **fünften** Aspekt der vorliegenden Erfindung - ist zudem das Arzneimittel oder Medikament, insbesondere Darmbehandlungsmittel, zur (Verwendung bei der) Verringerung pathogener Keime im menschlichen Darm, insbesondere in der menschlichen Darmflora, und/oder zur (Verwendung bei der) Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers, wobei das Arzneimittel oder Medikament, insbesondere Darmbehandlungsmittel, Cineol, vorzugsweise 1,8-Cineol, zusammen mit mindestens einem pharmazeutisch verträglichen und/oder physiologisch unbedenklichen Exzipienten (Träger) enthält.

Gemäß diesem Aspekt betrifft die vorliegende Erfindung das erfindungsgemäße Arzneimittel bzw. Medikament, insbesondere Darmbehandlungsmittel, insbesondere wie zuvor definiert, zur prophylaktischen und/oder therapeutischen Behandlung von Dysbiose und/oder von Fehlbesiedlungen des menschlichen Darms, insbesondere der menschlichen Darmflora, mit pathogenen Keimen, wobei das Arzneimittel oder Medikament, insbesondere Darmbehandlungsmittel, Cineol, vorzugsweise 1,8-Cineol, zusammen mit mindestens einem pharmazeutisch verträglichen und/oder physiologisch unbedenklichen Exzipienten (Träger) enthält.

Diesbezüglich kann es erfindungsgemäß vorgesehen sein, dass das Arzneimittel oder Medikament, das Cineol, vorzugsweise 1,8-Cineol, als alleinigen Wirkstoff, insbesondere als alleinigen pharmazeutischen Wirkstoff, enthält.

Erfindungsgemäß kann es zudem vorgesehen sein, dass das Arzneimittel oder Medikament das Cineol, vorzugsweise 1,8-Cineol, als Reinstoff enthält, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 Gew.-%, bezogen auf das Cineol, vorzugsweise 1,8-Cineol, und/oder vorzugsweise frei von anderen Terpenen.

Insbesondere kann das Arzneimittel oder Medikament das Cineol, vorzugsweise 1,8-Cineol, frei von anderen Terpenen enthalten. Insbesondere kann das Arzneimittel oder Medikament kein weiteres Terpen enthalten. Insbesondere kann das Arzneimittel oder Medikament frei von anderen Terpenen als Cineol, vorzugsweise 1,8-Cineol, sein.

Erfindungsgemäß kann das Arzneimittel bzw. Medikament das Cineol, vorzugsweise 1,8-Cineol, in wirksamen, insbesondere pharmazeutisch und/oder therapeutisch wirksamen, Mengen enthalten.

Insbesondere kann es dabei vorgesehen sein, dass das erfindungsgemäße Arzneimittel oder Medikament das Cineol, vorzugsweise 1,8-Cineol, bezogen auf das Arzneimittel oder Medikament, in relativen Mengen im Bereich von 0,0001 bis 80 Gew.-%, insbesondere 0,001 bis 75 Gew.-%, vorzugsweise 0,005 bis 70 Gew.- %, bevorzugt 0,01 bis 60 Gew.-%, besonders bevorzugt 0,05 bis 55 Gew.-%, ganz besonders bevorzugt 0,1 bis 50 Gew.-%, enthält.

Gemäß einer wiederum weiteren besonderen Ausführungsform gemäß diesem Erfindungsaspekt kann es auch vorgesehen sein, dass der mindestens eine pharmazeutisch verträgliche bzw. physiologisch unbedenkliche Exzipient (Träger) mit Cineol bzw. 1,8-Cineol mischbar und/oder hierin löslich ist, vorzugsweise wobei der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegt und/oder vorzugsweise wobei der mindestens eine pharmazeutisch verträgliche und/oder physiologisch unbedenkliche Exzipient (Träger) ausgewählt ist aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren.

Diesbezüglich kann das Cineol, vorzugsweise 1,8-Cineol, insbesondere das Arzneimittel oder Medikament, in pharmazeutisch wirksamen bzw. therapeutisch wirksamen Mengen verabreicht werden.

Insbesondere kann das Cineol, vorzugsweise 1,8-Cineol, insbesondere das Arzneimittel oder Medikament, zur Verabreichung in pharmazeutisch wirksamen bzw. therapeutisch wirksamen Mengen hergerichtet sein.

Zudem kann das Cineol, vorzugsweise 1,8-Cineol, insbesondere das Arzneimittel oder Medikament, systemisch appliziert werden.

Erfindungsgemäß kann in diesem Zusammenhang das Cineol, vorzugsweise 1,8-Cineol, insbesondere das Arzneimittel oder Medikament, oder die Zusammensetzung, zusammen mit mindestens einem weiteren Wirkstoff angewendet oder appliziert wird, insbesondere wobei der weitere Wirkstoff ausgewählt ist aus der Gruppe von (i) entzündungshemmenden Wirkstoffen (Antiphlogistika), insbesondere nichtsteroidalen Entzündungshemmern (NSAIDs) oder steroidalen Entzündungshemmern, vorzugsweise steroidalen Entzündungshemmern, bevorzugt Kortikosteroiden; (ii) Antibiotika, (iii) Präbiotika; und (iv) die Darmflora positiv beeinflussenden Mikroorganismen; sowie deren Kombinationen.

Für weitergehende Einzelheiten zu diesem Erfindungsaspekt kann Bezug genommen werden auf die vorstehenden Ausführungen zu den weiteren Erfindungsaspekten, wobei diese Ausführungen gleichermaßen für den vorliegenden Erfindungsaspekt gelten.

Wiederum weiterer Gegenstand der vorliegenden Erfindung - gemäß einem **sechsten** Aspekt der vorliegenden Erfindung ist zudem die Zusammensetzung nach der Erfindung, insbesondere pharmazeutische Zusammensetzung, zur (Verwendung bei der) Verringerung pathogener Keime im menschlichen Darm, insbesondere in der menschlichen Darmflora, und/oder zur (Verwendung bei der) Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers, wobei die Zusammensetzung Cineol, vorzugsweise 1,8-Cineol, insbesondere zusammen mit mindestens einem pharmazeutisch verträglichen und/oder physiologisch unbedenklichen Exzipienten (Träger), enthält.

In diesem Zusammenhang betrifft die vorliegende Erfindung gemäß diesem Aspekt auch die Zusammensetzung nach der Erfindung, vorzugsweise pharmazeutische Zusammensetzung, insbesondere wie zuvor definiert, zur (Verwendung bei der) prophylaktischen und/oder therapeutischen Behandlung von Dysbiose und/oder von Fehlbesiedlungen des menschlichen Darms, insbesondere der menschlichen Darmflora, mit pathogenen Keimen, wobei die Zusammensetzung Cineol, vorzugsweise 1,8-Cineol, insbesondere zusammen mit mindestens einem pharmazeutisch verträglichen und/oder physiologisch unbedenklichen Exzipienten (Träger), enthält.

Die Zusammensetzung kann das Cineol, vorzugsweise 1,8-Cineol, als alleinigen Wirkstoff, insbesondere als alleinigen pharmazeutischen Wirkstoff, enthalten.

Zudem kann die Zusammensetzung das Cineol, vorzugsweise 1,8-Cineol, als Reinstoff enthält, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 Gew.-%, bezogen auf das Cineol, vorzugsweise 1,8-Cineol, und/oder vorzugsweise frei von anderen Terpenen aufweisen.

Die Zusammensetzung nach der Erfindung kann das Cineol, vorzugsweise 1,8-Cineol, frei von anderen Terpenen enthalten.

Insbesondere kann die Zusammensetzung kein weiteres Terpen enthalten. Gleichermaßen kann die Zusammensetzung frei von anderen Terpenen als Cineol, vorzugsweise 1,8-Cineol, sein.

Erfindungsgemäß ist es insbesondere vorgesehen, dass die Zusammensetzung das Cineol, vorzugsweise 1,8-Cineol, in wirksamen, insbesondere pharmazeutisch und/oder therapeutisch wirksamen, Mengen enthält.

Diesbezüglich kann die Zusammensetzung das Cineol, vorzugsweise 1,8-Cineol, bezogen auf die Zusammensetzung, in relativen Mengen im Bereich von 0,0001 bis 80 Gew.-%, insbesondere 0,001 bis 75 Gew.-%, vorzugsweise 0,005 bis 70 Gew.- %, bevorzugt 0,01 bis 60 Gew.-%, besonders bevorzugt 0,05 bis 55 Gew.-%, ganz besonders bevorzugt 0,1 bis 50 Gew.-%, enthalten.

Insbesondere kann der mindestens eine pharmazeutisch verträgliche bzw. physiologisch unbedenkliche Exzipient (Träger) mit Cineol bzw. 1,8-Cineol mischbar und/oder hierin löslich ist, vorzugsweise wobei der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegt.

Diesbezüglich kann der mindestens eine pharmazeutisch verträgliche und/oder physiologisch unbedenkliche Exzipient (Träger) ausgewählt ist aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren.

Insbesondere kann das Cineol, vorzugsweise 1,8-Cineol, insbesondere die Zusammensetzung, in pharmazeutisch wirksamen bzw. therapeutisch wirksamen Mengen verabreicht werden. Insbesondere kann das Cineol, vorzugsweise 1,8-Cineol, insbesondere die Zusammensetzung, zur Verabreichung in pharmazeutisch wirksamen bzw. therapeutisch wirksamen Mengen hergerichtet sein. Insbesondere kann das Cineol, vorzugsweise 1,8-Cineol, insbesondere das Arzneimittel oder Medikament oder die Zusammensetzung, systemisch appliziert werden.

Auch diesbezüglich kann das Cineol, vorzugsweise 1,8-Cineol, insbesondere die Zusammensetzung, zusammen mit mindestens einem weiteren Wirkstoff angewendet oder appliziert werden, insbesondere wobei der weitere Wirkstoff ausgewählt ist aus der Gruppe von (i) entzündungshemmenden Wirkstoffen (Antiphlogistika), insbesondere nichtsteroidalen Entzündungshemmern (NSAIDs) oder steroidalen Entzündungshemmern, vorzugsweise steroidalen Entzündungshemmern, bevorzugt Kortikosteroiden; (ii) Antibiotika, (iii) Präbiotika; und (iv) die Darmflora positiv beeinflussenden Mikroorganismen; sowie deren Kombinationen.

Für weitergehende Einzelheiten zu diesem Erfindungsaspekt kann Bezug genommen werden auf die Ausführungen zu den weiteren Erfindungsaspekten, wobei diese Ausführungen gleichermaßen für den vorliegenden Erfindungsaspekt gelten.

Schließlich ist Gegenstand der vorliegenden Erfindung - gemäß einem **siebten** Aspekt - der vorliegenden Erfindung - gleichermaßen die pharmazeutische Kombination zur (Verwendung bei der) Verringerung pathogener Keime im menschlichen Darm, insbesondere in der menschlichen Darmflora, und/oder zur (Verwendung bei der) Reduzierung der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers, wobei die pharmazeutische Kombination mindestens die nachfolgenden Komponenten (A) und (B) umfasst:
(A) Cineol, vorzugsweise 1 ,8-Cineol; sowie
(B) mindestens einen weiteren Wirkstoff, ausgewählt aus der Gruppe von (i) entzündungshemmenden Wirkstoffen (Antiphlogistika), insbesondere nichtsteroidalen Entzündungshemmern (NSAIDs) oder steroidalen Entzündungshemmern, vorzugsweise steroidalen Entzündungshemmern, bevorzugt Kortikosteroiden; (ii) Antibiotika, (iii) Präbiotika; und (iv) die Darmflora positiv beeinflussenden Mikroorganismen; sowie deren Kombinationen.

Gemäß dem vorliegenden Aspekt betrifft die vorliegende Erfindung auch die pharmazeutische Kombination, insbesondere eine wie zuvor definierte pharmazeutische Kombination, zur (Verwendung bei der) prophylaktischen und/oder therapeutischen Behandlung von Dysbiose und/oder von Fehlbesiedlungen des menschlichen Darms, insbesondere der menschlichen Darmflora, mit pathogenen Keimen, wobei die pharmazeutische Kombination mindestens die nachfolgenden Komponenten (A) und (B) umfasst:
(A) Cineol, vorzugsweise 1,8-Cineol; sowie
(B) mindestens einen weiteren Wirkstoff, ausgewählt aus der Gruppe von (i) entzündungshemmenden Wirkstoffen (Antiphlogistika), insbesondere nichtsteroidalen Entzündungshemmern (NSAIDs) oder steroidalen Entzündungshemmern, vorzugsweise steroidalen Entzündungshemmern, bevorzugt Kortikosteroiden; (ii) Antibiotika, (iii) Präbiotika; und (iv) die Darmflora positiv beeinflussenden Mikroorganismen; sowie deren Kombinationen.

Auch im Rahmen dieses Erfindungsaspekts ist es insbesondere vorgesehen, dass der Wirkstoff der Komponente (A) bzw. das Cineol, vorzugsweise 1,8-Cineol, in pharmazeutisch wirksamen, bzw. therapeutisch wirksamen Mengen verabreicht wird bzw. dass das Cineol, vorzugsweise 1,8-Cineol, zur Verabreichung in pharmazeutisch wirksamen bzw. therapeutisch wirksamen Mengen hergerichtet ist. Dies gilt in entsprechender Weise auch für den Wirkstoff gemäß der Komponente (B).

Insbesondere kann der Wirkstoff der Komponente (A) bzw. das Cineol, vorzugsweise 1,8-Cineol, systemisch appliziert werden. Auch der weitere Wirkstoff gemäß der Komponente (B) kann insbesondere gleichermaßen systemisch angewendet bzw. appliziert werden.

Gemäß einer besonderen Ausführungsform dieses Erfindungsaspekts kann es insbesondere vorgesehen sein, dass die Komponenten (A) und (B) getrennt voneinander, insbesondere räumlich getrennt voneinander, aber funktional zusammenhängend und/oder funktional einander zugeordnet vorliegen und/oder verabreicht werden.

Weiterhin kann es gemäß einer wiederum besonderen Ausführungsform dieses Erfindungsaspekts insbesondere vorgesehen sein, dass die pharmazeutische Kombination in Form eines Kit (Kit-of-parts), insbesondere als Kit der Komponenten (A) und (B), vorliegt.

Insbesondere können die Komponenten (A) und (B) als Kit (Kit-of-parts) vorliegen bzw. hergerichtet sein bzw. verabreicht werden.

Als Kit bzw. Kit-of-parts wird im Rahmen der vorliegenden Erfindung insbesondere eine Einheit bzw. Anordnung bzw. Zusammenstellung bzw. Kombination der beiden Komponenten (A) und (B) verstanden, bei welcher die beiden Komponenten (A) und (B) zwar getrennt und/oder separat, insbesondere räumlich getrennt und/oder räumlich separiert, vorliegen, aber als funktional zusammengehörende bzw. als funktional einander zugeordnete Komponenten vorgesehen sind bzw. verabreicht werden.

Für weitergehende Einzelheiten zu diesem Erfindungsaspekt kann Bezug genommen werden auf die Ausführungen zu den weiteren Erfindungsaspekten, wobei diese Ausführungen gleichermaßen für den vorliegenden Erfindungsaspekt gelten.

Weitere vorteilhafte Eigenschaften, Aspekte und Merkmale der vorliegenden Erfindung ergeben sich auch aus der folgenden Beschreibung von in den Figuren dargestellten Ausführungsbeispielen, wie sie nachfolgend noch im Detail angeführt sind. Es zeigt:

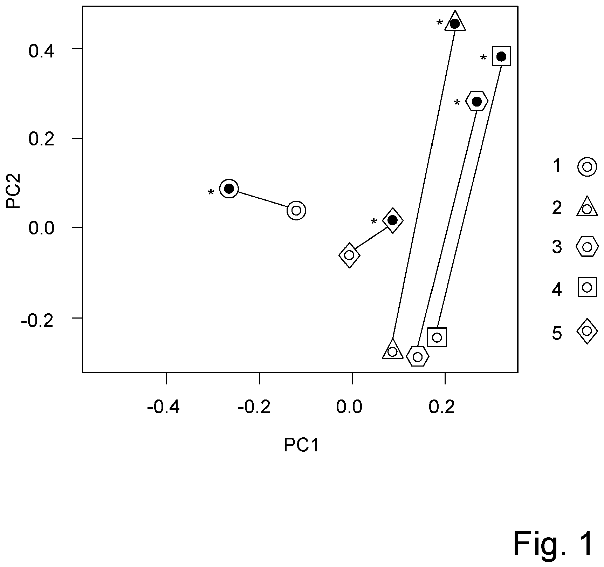

- Fig. 1: eine graphische Darstellung einer Hauptkoordinatenanalyse (PCoA) von untersuchten Stuhlproben von fünf Patienten (1-5) mit *Polyposis nasi*, und zwar vor (ohne Kennzeichnung, nicht ausgefüllte Kreissymbole) und nach 2-wöchiger Einnahme von 1,8-Cineol (mit "*" gekennzeichnet, ausgefüllte Kreissymbole);

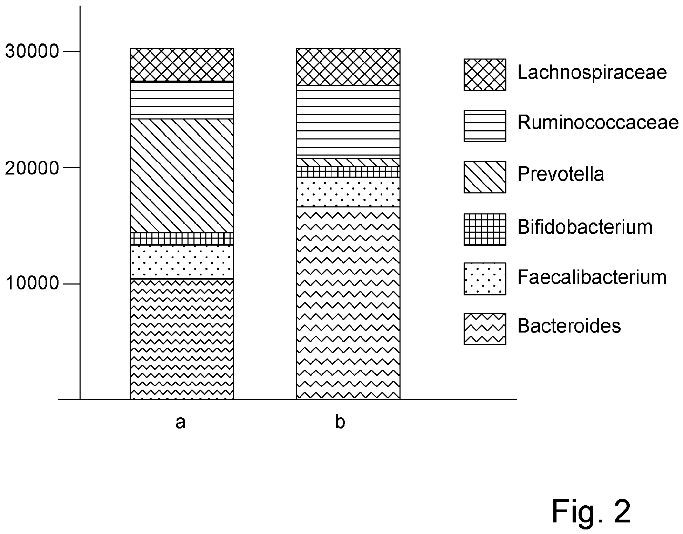

- Fig. 2: eine graphische Darstellung in Form eines Balkendiagramms zum Einfluss von 1,8-Cineol auf das Mikrobiom des Darms bzw. auf die Darmflora von Patienten mit *Polyposis nasi*; in Bezug auf die X-Achse zeigt die Bezeichnung "a" die Situation bzw. Zusammensetzung des Mikrobioms des Darms vor Verabreichung von Cineol und die Bezeichnung "b" die diesbezügliche Situation nach 2-wöchiger Einnahme von Cineol; weiterhin zeigt die Y-Achse die Anzahl der durchgeführten Sequenzierungs-Lesevorgänge;

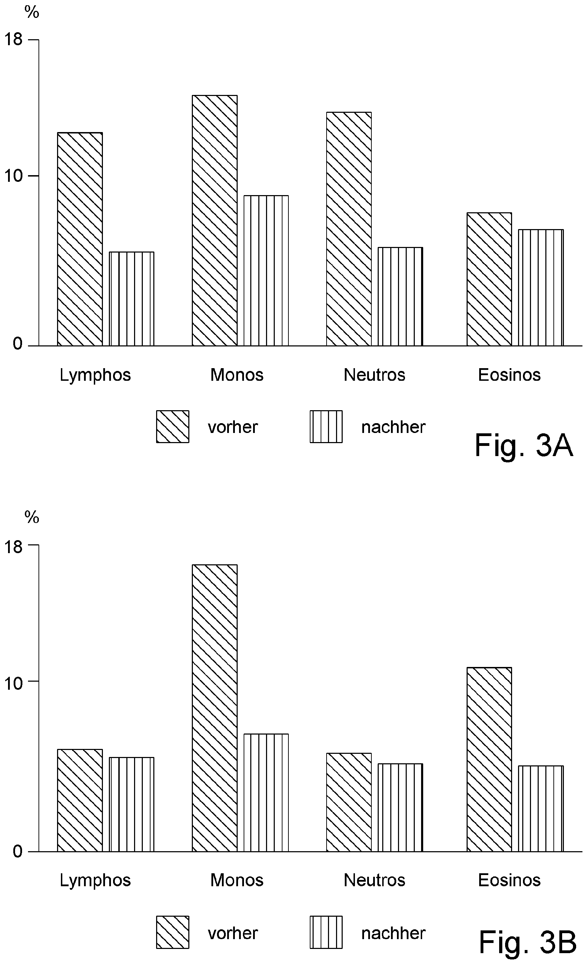

- Fig. 3A/B: eine jeweilige graphische Darstellung in Form von Balkendiagrammen, wobei aufgezeigt wird, dass die Einnahme von 1,8-Cineol eine deutliche Abnahme der zellulären Komplexe verschiedener Leukozyten hervorruft [mit "Lymphos" = Lymphozyten; "Monos" = Monozyten; "Neutros" = Neutrophile; "Eosinos" = Eosinophile im Blut von Patienten mit *Polyposis nasi*; aufgezeigt ist jeweils die Situation vor ("vorher") und nach 2-wöchiger Verabreichung von 1,8-Cineol ("nachher"); Fig. 3A bezieht sich auf die Daten n1, und Fig. 3B bezieht sich auf die Daten n2, vgl. auch nachfolgende Ausführungen)];

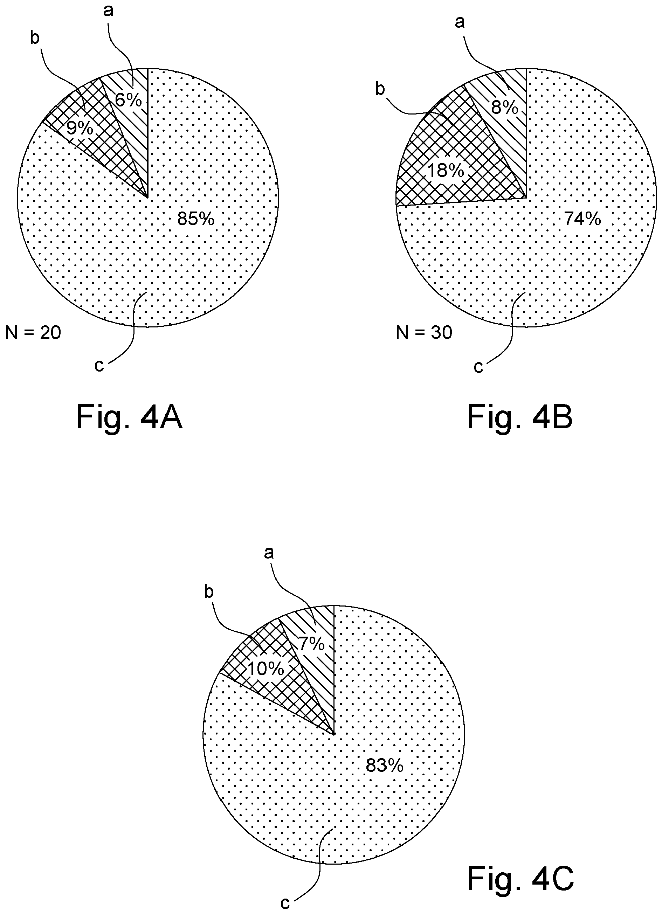

- Fig. 4A/B/C: eine jeweilige graphische Darstellung auf Basis von Kreisdiagrammen, welche insgesamt die Verminderung der Population sogenannter "classical"-Monozyten und eine deutliche Zunahme von sogenannten "intermediate"-Monozyten im Blut von untersuchten von Patienten mit *Polyposis nasi* (Fig. 4B) im Vergleich zu gesunden Spendern (Fig. 4A) zeigt, wonach zudem gezeigt wird, dass nach Einnahme von 1,8-Cineol über einen Zeitraum von zwei Wochen eine deutliche Rückverschiebung hinsichtlich der Verteilung der jeweiligen Monozyten vorliegt (Fig. 4C), welche mit dem Verteilungsmuster gesunder Spender vergleichbar ist; in diesem Zusammenhang zeigt das Kreisdiagramm gemäß Fig. 4A somit die Situation für gesunde Spender, das Kreisdiagramm gemäß Fig. 4B somit die Situation für Patienten mit *Polyposis nasi* und das Kreisdiagramm gemäß Fig. 4C die Situation für Patienten mit *Polyposis nasi* nach Einnahme von 1,8-Cineol für einen Zeitraum von zwei Wochen (mit a = "non-classical"-Monozyten, b = "intermediate"-Monozyten und c = "classical"-Monozyten);

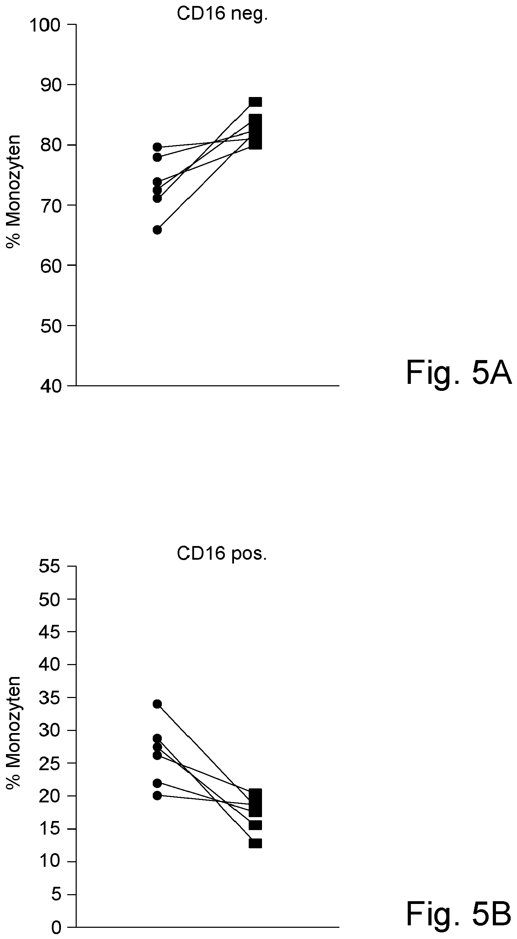

- Fig. 5A/B: eine jeweilige graphische Darstellung der Monozytenverteilung unter dem Einfluss einer 2-wöchigen Therapie mit 1,8-Cineol bzw. vor und nach 2-wöchiger Therapie mit 1,8-Cineol; die Untersuchungen zeigen ein starkes Ansprechen auf die Einnahme von 1,8-Cineol, wobei sich bei den untersuchten Patienten eine deutliche Zunahme an CD16-"classical"-Monozyten ("CD16 neg."; Fig. 5A") und zugleich eine deutliche Abnahme der "intermediate"-Monozyten ("CD16 pos."; Fig. 5B") nach Therapie mit 1,8-Cineol zeigt [in Fig. 5A/B zeigt die jeweilige linke Kreisdarstellung die Situation vor Durchführung der Cineol-Behandlung und die jeweilige rechte Kästchendarstellung die Situation nach durchgeführter Cineol-Therapie].

Für weitergehende Einzelheiten zu den in den Figurendarstellungen dargestellten Ausführungsformen und Ausführungsbeispiele kann zur Vermeidung unnötiger Wiederholungen auf die obigen Ausführungen sowie auf die nachfolgenden ergänzenden Ausführungen zu den Ausführungsbeispielen verwiesen werden, welche in Bezug auf die Figurendarstellungen entsprechend gelten.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### AUSFÜHRUNGSBEISPIELE:

### Beispiel 1: Antientzündliche Wirkung von 1,8-Cineol auf das Darmmikrobiom sowie antidysbiotische bzw. antipathogene Wirkung von 1,8-Cineol in Bezug auf pathogene Darmkeime

Das menschliche Mikrobiom, insbesondere auch das Mikrobiom des Darmes, hat grundlegenden regulatorischen Einfluss auf Entzündungsprozesse und die dazugehörige Immunregulation.

Vorliegend kann gezeigt werden, dass eine direkte lokale Einflussnahme von 1,8-Cineol, welches als dünndarmgängiges bzw. -lösliches Präparat verabreicht wird (Soledum^{®} forte, Gehalt an Cineol: 200 mg), auf das Mikrobiom des Darmes vorliegt, wobei auch weitreichende Auswirkungen auf systemische und lokale Entzündungsprozesse im menschlichen Körper beobachtet werden.

Im Rahmen der Untersuchungen werden die Stuhlproben von Patienten mit Polyposis nasi unter dem Einfluss von 1,8-Cineol analysiert. Dazu wird zunächst vor Beginn der Verabreichung von 1,8-Cineol eine Stuhlprobe genommen und dann erneut nach 2-wöchiger Einnahme von 3-mal täglich 1,8-Cineol (Soledum^{®} forte, Gehalt an 1 ,8-Cineol: 200 mg).

Die Stuhlproben werden mit *Stool Stabilizer* (Firma: Stratec Molekular) versehen und zunächst bis zur weiterführenden Verarbeitung bei einer Temperatur von - 20 °C gelagert. Die anschließende DNA-Extraktion wird mit dem *PSP Spin Stool DNA Plus-Kit* entsprechend der Angaben des Herstellers (Stratec Molecular) durchgeführt. Die isolierte DNA wird schließlich für die Mikrobiomsequenzierung *(TG MiSeq Reagent Kit v3* & *NEBNext*^{®} *Library Quant Kit for Illumina*) eingesetzt und ausgewertet.

Die Ergebnisse sind in Fig. 1 veranschaulicht, und zwar in Form einer Hauptkoordinatenanalyse (PCoA) der untersuchten Stuhlproben der Patienten (1 bis 5) mit Polyposis nasi vor (ohne Kennzeichnung, nicht ausgefüllte Kreissymbole) und nach nach 2-wöchiger Einnahme von 1,8-Cineol (mit "*" gekennzeichnet, ausgefüllte Kreissymbole).

Die Untersuchungen zeigen bei den analysierten Patientenproben eine deutliche Verschiebung der sogenannten Beta-Diversität anhand der Hauptkoordinatenanalyse (PCoA), also die Heterogenität bzw. sozusagen die Unähnlichkeit der gemessenen Proben vor und nach der Einnahme von 1,8-Cineol, was bedeutet, dass unter Einfluss von 1,8-Cineol eine entsprechende Veränderung vorliegt.

Im Anschluss an diese erste Auswertung hinsichtlich vorliegender Alterationen bzw. Veränderungen des Darmmikrobioms der Patienten mit Polyposis nasi nach 2-wöchiger Einnahme von 1,8-Cineol wird eine detaillierte Auswertung der durchgeführten Mikrobiom-Sequenzierung hinsichtlich der identifizierten Bakteriengattungen vorgenommen.

Die diesbezüglichen Untersuchungen zeigen eine deutliche Verschiebung der Zusammensetzung bzw. Komposition des Darmmikrobioms der Patienten mit Polyposis nasi nach 2-wöchiger Einnahme von 1,8-Cineol. Fig. 2 veranschaulicht dabei den Einfluss von 1,8-Cineol auf das Darmmikrobiom von Patienten mit Polyposis nasi (wobei die Bezeichnung "a" die Situation bzw. Zusammensetzung des Mikrobioms des Darms vor Verabreichung von Cineol und die Bezeichnung "b" die Situation nach 2-wöchiger Einnahme von Cineol betrifft; weiterhin zeigt die Y-Achse die Anzahl der durchgeführten Sequenzierungs-Lesevorgänge).

In diesem Zusammenhang verdeutlicht Fig. 2 somit die erfindungsgemäß aufgefundenen deutlichen Verschiebungen verschiedener Bakteriengattungen: Insbesondere zeigen die Analysen des Mikrobioms, dass die Bakteriengattung *Prevotella* als pathogener bzw. entzündungsfördernder Keim im Darm der untersuchten Patienten mit Polyposis nasi sehr prominent vorliegt und nach 2-wöchiger Einnahme von 1,8-Cineol deutlich verringert ist, wobei diesbezüglich sogar ein nahezu vollständiges Verschwinden beobachtet werden kann.

Zur Gattung Prevotella gehören im Allgemeinen etwa vierzig verschiedene Spezies, welche oftmals verschiedene Entzündungsprozesse der Mundhöhle (*Prevotella bivia*, *Prevotella disiens*) oder des Respirationstraktes (*Prevotella melaninogenica*, *Prevotella intermedia*) verursachen.

Zudem ist eine deutliche Veränderung des Auftretens von Keimen etwa bei der sehr häufigen Gattung *Bacteroides*, welche nichtpathogen und insbesondere gesundheitsfördend und für den Menschen insgesamt positive Auswirkungen hat, zu beobachten, nämlich dahingehend, dass diese nach der Therapie mit 1,8-Cineol deutlich häufiger vorliegen Bei weiteren zur normalen menschlichen Darmflora gehörenden Familien bzw. Gattungen, wie etwa *Lachnospiraceae*, *Bifidobacterium* oder *Faecalibacterium* sind ebenfalls gewisse Veränderungen bzw. Verschiebungen mit einer Tendenz zur Zunahme nach Durchführung der Therapie zu beobachten.

Weiterführende Auswertungen der durchgeführten Mikrobiomsequenzierungen führen darüber hinaus zur Identifizierung einer deutlichen Zunahme von Bakterien der Familie *Ruminococcacea* bzw. der Gattung *Ruminococcus* infolge der Einnahme von 1,8-Cineol, wobei derartige Bakterien dafür bekannt sind, antientzündliche kurzkettige Fettsäuren, wie etwa Butyrat, zu produzieren.

Die Untersuchungen zeigen somit die positiven Auswirkungen von 1,8-Cineol auf die Zusammensetzung des menschlichen Mikrobioms des Darms bzw. der Darmflora mit der Verringerung entzündlich wirkender bzw. negativer Keime und der Zunahme antientzündlicher bzw. positiver Keime, was mit einer Einflussnahme auch auf gesamtkörperliche bzw. systemische entzündliche Vorgänge einhergeht.

### Beispiel 2: Antientzündliche Wirkung von 1,8-Cineol in Bezug auf Immunzellen im peripheren Blut

Wie vorstehend erläutert, bewirkt das 1,8-Cineol zum einen am primären Wirkort, nämlich dem menschlichen Darm, eine positive Wirkung auf das Darmepithel mit den dort vorliegenden Immunzellen, und zwar insbesondere auf Basis der Verringerung pathogener Keime im menschlichen Mikrobiom des Darms, so dass deren negativer Einfluss auf das Darmepithel verringert bzw. unterbunden wird (vgl. vorangehendes Ausführungsbeispiel). Dies wiederum führt zu einer Verbesserung des diesbezüglichen Immunstatus (d.h. Induzierung einer aktiven bzw. primären Entzündungshemmungsvermittlung). Dies wird durch die nachfolgenden Versuch dokumentiert.

Vorliegend werden Untersuchungen im Hinblick auf die Regulation zirkulierender Blutzellen/Immunzellen bei Patienten mit Polyposis nasi unter dem Einfluss von 1 ,8-Cineol beschrieben.

Die Analysen mittels Fluoreszenzmikroskopie zeigen eine deutliche Zunahme der Komplexformation zirkulierender Thrombozyten (CD41) mit unterschiedlichen Immunzellen (DAPI) bei Patienten mit Polyposis nasi und deuten zudem auf eine hierdurch bedingte Regulation der Immunfunktionen, wie etwa die Expressionslevel des Zytokins TNFa.

Weiterführend werden diese zellulären Komplexe mittels Durchflusszytometer hinsichtlich unterschiedlicher Leukozytenpopulationen und hinsichtlich der Einflussnahme von 1 ,8-Cineol genauer spezifiziert.

Hierbei zeigt sich, dass diese zellulären Komplexe im Blut von Patienten mit Polyposis nasi insbesondere im Zusammenhang mit Monozyten auftreten und dass nach der Einnahme von 1,8-Cineol (vgl. obige Ausführungen) über einen Zeitraum von zwei Wochen eine massive Reduzierung dieser Monozytenkomplexe zu beobachten ist, wie anhand der nachfolgenden Tabellen und in Fig. 3A/3B gezeigt [mit "Lymphos" = Lymphozyten; "Monos" = Monozyten; "Neutros" = Neutrophile; "Eosinos" = Eosinophile im Blut von Patienten mit *Polyposis nasi*; aufgezeigt ist jeweils die Situation vor ("vorher") und nach 2-wöchiger Verabreichung von 1,8-Cineol ("nachher"); Fig. 3A bezieht sich auf die Daten n1 gemäß Tabelle 1, und Fig. 3B bezieht sich auf die Daten n2 gemäß Tabelle 2)].

**Tabelle 1:**

| **n1** | **vorher** | **nachher** |
|---|---|---|
| **Lymphos** | 12,4 % | 5,54 % |
| **Monos** | 14,7 % | 8,79 % |
| **Neutros** | 13,9 % | 5,02 % |
| **Eosinos** | 7,3 % | 5,92 % |

**Tabelle 2:**

| **n1** | **vorher** | **nachher** |
|---|---|---|
| **Lymphos** | 5,4 % | 5,12 % |
| **Monos** | 16,8 % | 6,28 % |
| **Neutros** | 5,37 % | 5,09 % |
| **Eosinos** | 10,7 % | 4,8 % |

Die im Blut zirkulierenden Monozyten lassen sich anhand ihrer Oberflächenproteine CD14 und CD16 mittels Durchflusszytometrie in die drei verschiedenen Subtypen unterteilen, nämlich "classical", "intermediate" und "non-classical". Bei der Durchflusszytometrie werden die Zellen hinsichtlich der Zellgröße, Granularität und der Fluoreszenzmarkierung spezifischer Epitope durch das Passieren spezifischer Laser analysiert. Dadurch können verschiedene Zellpopulationen differenziert und charakterisiert werden. Während sich die "classical"-Monozyten nach der Einwanderung in entzündliches Gewebe zu antientzündlichen Makrophagen differenzieren, werden aus den "non-classical"-Monozyten immunsuppressive Makrophagen, welche die entzündlichen Prozesse nicht unterbinden und somit das damit verbundene Krankheitsbild aufrechterhalten.

Die Untersuchungen zeigen eine Verminderung der Population der "classical"-Monozyten und eine deutliche Zunahme der Population der "intermediate"-Monozyten im Blut der untersuchten Patienten mit Polyposis nasi im Vergleich zu gesunden Spendern, was somit auf eine möglicherweise erhöhte Infiltration des entzündlichen Polypengewebes durch immunsuppressive Makrophagen und eine daraus resultierende persistierende inflammatorische Progression der Nasenpolypen hindeutet. Hierzu kann auch auf Fig. 4 A/B/C verwiesen werden.

In diesem Zusammenhang zeigt Fig. 4 A/B/C die gefundene Verminderung der Population der "classical"-Monozyten und die deutliche Zunahme der "intermediate"- Monozyten im Blut der untersuchten Patienten mit Polyposis nasi im Vergleich zu gesunden Spendern.

Nach 2-wöchiger Einnahme von 1,8-Cineol werden wiederum die Monozytenpopulationen im Blut der Patienten mit Polyposis nasi mittels Durchflusszytometer untersucht und es wird eine deutliche Normalisierung bzw. Rückverschiebung der Verteilung der Monozytensubpopulationen festgestellt, vergleichbar mit dem Verteilungsmuster bei gesunden Personen bzw. Spendern.

Fig. 4 A/B/C zeigt insgesamt die Verminderung der Population der "classical"-Monozyten und eine deutliche Zunahme der "intermediate"-Monozyten im Blut von untersuchten von Patienten mit *Polyposis nasi* (Fig. 4B) im Vergleich zu gesunden Spendern (Fig. 4A). Zudem wird gezeigt, dass nach Einnahme von 1,8-Cineol über einen Zeitraum von zwei Wochen eine deutliche Normalisierung bzw. Rückverschiebung hinsichtlich der Verteilung der jeweiligen Monozyten resultiert (Fig. 4C), welche mit dem Verteilungsmuster gesunder Spender vergleichbar ist. Das Kreisdiagramm gemäß Fig. 4A zeigt die Situation für gesunde Spender, das Kreisdiagramm gemäß Fig. 4B die Situation für Patienten mit *Polyposis nasi* und das Kreisdiagramm gemäß Fig. 4C die Situation für Patienten mit *Polyposis nasi* nach Therapie bzw. Einnahme von 1,8-Cineol für einen Zeitraum von zwei Wochen (mit a = "non-classical"-Monozyten, b = "intermediate"-Monozyten und c = "classical"-Monozyten).

Diese Beobachtungen werden nun bei weiteren Patienten individuell mittels Durchflusszytometer umfassend analysiert, insbesondere unter der Einflussnahme von 1,8-Cineol. Die Untersuchungen bestätigen ein starkes Ansprechen der zirkulierenden Immunzellen auf die Einnahme von 1,8-Cineol bei den untersuchten Patienten, einhergehend mit einer Restauration der Monozytenverteilung, und zwar vergleichbar mit der gesunden Situation. Hierzu kann auch auf Fig. 5A/B verwiesen werden.

Fig. 5A/B die Monozytenverteilung unter dem Einfluss einer 2-wöchigen Therapie mit 1,8-Cineol bzw. vor und nach 2-wöchiger Therapie mit 1,8-Cineol; die Untersuchungen zeigen ein starkes Ansprechen auf die Einnahme von 1,8-Cineol, wobei bei den untersuchten Patienten eine deutliche Zunahme an CD16-"classical"-Monozyten ("CD16 neg."; Fig. 5A") und zugleich eine deutliche Abnahme der "intermediate"-Monozyten ("CD16 pos."; Fig. 5B") nach Therapie mit 1,8-Cineol zu beobachten ist (in Fig. 5A/B zeigt die jeweilige linke Kreisdarstellung die Situation vor Cineol-Behandlung und die jeweilige rechte Kästchendarstellung die Situation nach Cineol-Behandlung).

Fig. 5 A/B zeigt somit die Monozytenverteilung unter dem Einfluss einer 2-wöchigen Therapie mit 1,8-Cineol, wobei ein starkes Ansprechen auf die Einnahme von 1,8-Cineol gezeigt wird. Bei den untersuchten Patienten zeigt sich eine deutliche Zunahme der CD16-"classical"-Monozyten (Fig. 5A) und zugleich eine deutliche Abnahme der "intermediate"-Monozyten (Fig. 5B).

### Beispiel 3: Patientenwahrnehmungen und klinische Anwendungen

Wie vorstehend gleichermaßen erläutert, bewirkt das 1,8-Cineol nicht nur am primären Wirkort, nämlich dem menschlichen Darm, eine positive Wirkung auf das Darmepithel mit den dort vorliegenden Immunzellen, sondern auch im Hinblick auf die Verbesserung des diesbezüglichen Immunstatus (d.h. Induzierung einer aktiven bzw. primären Entzündungshemmungsvermittlung). Dies wiederum führt auch zu einer systemisch relevanten Verbesserung entzündlicher Prozesse bzw. Parameter im menschlichen Körper bei klinischer Anwendung. Dies kann anhand der Therapie verschiedenster Entzündungserkrankungen des menschlichen Körpers nachgewiesen werden (was ein Indiz für die organunspezifische bzw. regiounspezifische und damit systemisch universelle Entzündungshemmung ist).

Ergänzend zu den molekularbiologischen Untersuchungen wird auch die individuelle Wahrnehmung und Lebensqualität der Patienten hinsichtlich der 2-wöchigen Einnahme von 1,8-Cineol erhoben. Von den herangezogenen Polyposis-Patienten liegt eine überwiegend positive Resonanz hinsichtlich der 2-wöchigen Einnahme von 1,8-Cineol vor.

Bei 38 Patienten (23 Männer / 15 Frauen zwischen 28 und 72 Jahren, Durchschnittsalter 54 Jahre) jeweils mit Polyposis nasi wurde eine Therapie mit 1,8-Cineol durchgeführt (3-mal 200 mg 1,8-Cineol in Form von Kapseln "Soledum^{®} forte").

Die behandelten Patienten berichten von einem deutlich verbesserten Sekretabfluss und einer entsprechend verbesserten Nasenatmung. In einem Fall wurden bei dem Patienten relativ kleine Nasenpolypen diagnostiziert, welche direkt auf der Riechrinne lokalisiert sind. Bei diesem Patienten wird nach 2-wöchiger Einnahme von 1,8-Cineol ein deutlicher Rückgang der Polypen festgestellt, wobei auch eine deutliche Verbesserung des Riechempfindens vorliegt.

### Beispiel 4: 1,8-Cineol im klinischen Einsatz

Darüber hinaus wird die Wirkung von 1,8-Cineol auf Basis der nachfolgenden Einsatzbereiche weiterführend untersucht, wobei über den jeweils angegebenen Zeitraum 3-mal täglich 200 mg 1,8-Cineol verbreicht werden (Soledum^{®} forte):
1. Chronisch polypöse Sinusitis
2. Chronisch rezidivierende katarralische Sinusitis
3. Chronisch polypöse Otitis media ohne Cholesteatom
4. Chronisch polypöse Otitis media mit Cholesteatom

| | |
|---|---|
| Ad 1. | nach 2-wöchiger Verabreichung (präoperativ) Rückgang der Begleitsymptome der Polypen, wie anteriore und posteriore wässrige Rhinorrhoe. In Einzelfällen zudem Verbesserung der Riechleistung; postoperativ positiver Einfluss auf die Wundheilung und in Kombination mit einem nasalen Steroid Reduktion von Rezidiven und systemischer Kortisongabe; |
| Ad 2. | nach 2-wöchiger Verabreichung (präoperativ) Rückgang der Begleitsymptome der Polypen, wie anteriore und posteriore wässrige Rhinorrhoe; deutliche Verbesserung der Riechleistung; postoperativ positiver Einfluss auf die Wundheilung, Reduktion von nasalen Steroiden; |
| Ad 3. | nach 2-wöchiger Verabreichung (präoperativ) bei zuvor therapierefraktärer Otorrhoe (lokale und systemische Antibiotika) Rückgang der Otorrhoe, so dass eine Operation ermöglicht wird; bei Zustand nach Tympanoplastik erneute Otorrhoe mit Rezidivperforation; nach 4-wöchiger Verabreichung Rückgang der Otorrhoe und spontaner Trommelfellverschluss; |
| Ad 4. | nach 2-wöchiger Verabreichung (präoperativ) bei zuvor therapierefraktärer Otorrhoe (lokale und systemische Antibiotika) Rückgang der Begleit-Otorrhoe, so dass eine Operation ermöglicht wird; keine Reduktion der Cholesteatommassen, aber positiver Effekt auf die entzündete Schleimhaut des Mittelohres und Mastoid; bei Zustand nach Tympanoplastik erneute Otorrhoe mit Rezidivperforation. Nach 4-wöchiger Verabreichung Rückgang der Otorrhoe und spontaner Trommelfellverschluss. |

### Beispiel 5: 1,8-Cineol im weiteren klinischen Einsatz

Patienten mit persitierendem Asthma bronchiale, welche mit einer Kombinationstherapie aus inhalativem Glukokortikoid und inhalativem langwirksamem Beta-2-Sympathomimetika sowie Theophyllin peroral behandelt werden, erhalten eine Woche lang 1,8-Cineol (Soledum^{®}forte-Kapseln) 4-mal 200 mg/die peroral. Bereits nach einwöchiger Therapie mit der vorgenannten Dosis kann eine leichte bis mittlere Verbesserung der Lungenfunktion erreicht werden. Nach zwölfwöchiger Dauertherapie hat sich das persistierende Bronchialasthma so stabilisiert, dass der inhalative Glukokortikoidbedarf um mehr als 50 % reduziert werden kann und bei einigen der behandelten Patienten die Glukokortikoide zeitweise sogar ganz abgesetzt werden können. Bei einigen Patienten kann auch der Betamimetikabedarf signifikant reduziert werden. Die Therapie mit 1,8-Cineol wird ohne Nebenwirkungen gut vertragen. Die Versuchsergebnisse belegen, dass 1,8-Cineol aufgrund seiner entzündungshemmenden Wirkung die Wirksamkeit inhalativer Atemwegstherapeutika in signifikanter Weise steigern und auf diese Weise deren Dosisbedarf signifikant reduziert werden kann.

Insgesamt eignet sich somit Cineol, vorzugsweise 1,8-Cineol, auf Basis der überraschend aufgefundenen Erkenntnisse der Anmelderin in effizienter Weise zur Verwendung bei der Verringerung pathogener Keime im menschlichen Darm bzw. zur Verwendung bei der Reduktion der Besiedlung des menschlichen Darms mit pathogenen Keimen, vorzugsweise zu Zwecken der Vermeidung, Verringerung oder Heilung entzündlicher Erkrankungen des menschlichen Körpers bzw. vorzugsweise zu Zwecken der prophylaktischen und/oder therapeutischen Behandlung entzündlicher Erkrankungen des menschlichen Körpers.

Die vorliegende Erfindung wird durch die folgenden Aspekte beschrieben, welche gleichermaßen Gegenstand der vorliegenden Erfindung sind:
Aspekt 1:
   1. Cineol, vorzugsweise 1,8-Cineol, zur Verwendung bei der Verringerung pathogener Keime im menschlichen Darm, insbesondere in der menschlichen Darmflora, und/oder zur Verwendung bei der Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers.
Aspekt 2:
   2. Cineol, vorzugsweise 1,8-Cineol, zur Verwendung nach Aspekt 1,
   wobei das Cineol, vorzugsweise 1,8-Cineol, zur Reduktion der Keimlast und/oder der Keimanzahl der pathogenen Keime im menschlichen Darm und/oder zur Reduktion des Keimanteils der pathogenen Keime, bezogen auf die Gesamtkeimanzahl im menschlichen Darm, insbesondere in der menschlichen Darmflora, verwendet oder eingesetzt wird.
Aspekt 3:
   3. Cineol, vorzugsweise 1,8-Cineol, zur Verwendung nach Aspekt 1 oder 2,
   wobei die pathogenen Keime ausgewählt sind aus der Gruppe von entzündungsverursachenden (entzündungsinduzierenden), entzündungsfördernden und mit Entzündungen im menschlichen Körper im Zusammenhang stehenden oder diese hervorrufenden Keimen, insbesondere aus der Gruppe von entzündungsverursachende und entzündungsfördernde Substanzen produzierenden und/oder freisetzenden Keimen, sowie deren Kombinationen.
Aspekt 4:
   4. Cineol, vorzugsweise 1,8-Cineol, zur Verwendung nach einem der vorangehenden Aspekten,
   wobei die pathogenen Keime ausgewählt sind aus der Gruppe von Bakterien, insbesondere die Gesundheit negativ beeinträchtigenden und/oder gesundheitsschädlichen Bakterien, vorzugsweise aus der Gruppe von entzündungsverursachenden, entzündungsinduzierenden und mit Entzündungen im menschlichen Körper im Zusammenhang stehenden oder diese hervorrufenden Bakterien, sowie deren Kombinationen.
Aspekt 5:
   5. Cineol, vorzugsweise 1,8-Cineol, zur Verwendung nach einem der vorangehenden Aspekten,
   wobei die pathogenen Keime ausgewählt sind aus der Gruppe von Bakterien aus der Familie der *Prevotellaceae,* vorzugsweise aus der Gruppe von Bakterien der Gattung *Prevotella.*
Aspekt 6:
   6. Cineol, vorzugsweise 1,8-Cineol, zur Verwendung nach einem der vorangehenden Aspekten,
   wobei das Cineol, vorzugsweise 1,8-Cineol, des Weiteren und/oder zusätzlich und/oder gleichermaßen zur Erhöhung, Vermehrung oder Unterstützung gesundheitsfördernder oder nichtpathogener, bevorzugt gesundheitsfördernder, Keime im menschlichen Darm, insbesondere in der menschlichen Darmflora, verwendet oder eingesetzt wird.
Aspekt 7:
   7. Cineol, vorzugsweise 1,8-Cineol, zur Verwendung nach einem der vorangehenden Aspekten,
   wobei das Cineol, vorzugsweise 1,8-Cineol, des Weiteren und/oder zusätzlich und/oder gleichermaßen zur Erhöhung der Keimbeladung oder der Keimanzahl oder des Keimanteils, bezogen auf die Gesamtkeimanzahl im menschlichen Darm, insbesondere in der menschlichen Darmflora, gesundheitsfördernder oder nichtpathogener, bevorzugt gesundheitsfördernder, Keime im menschlichen Darm, insbesondere in der menschlichen Darmflora, verwendet oder eingesetzt wird.
Aspekt 8:
   8. Cineol, vorzugsweise 1,8-Cineol, zur Verwendung nach einem der vorangehenden Aspekten,
   wobei das Cineol, vorzugsweise 1,8-Cineol, des Weiteren und/oder zusätzlich und/oder gleichermaßen zur Verwendung bei der Erhöhung der Besiedlung des menschlichen Darms, insbesondere der menschlichen Darmflora, mit gesundheitsfördernden oder nichtpathogenen, bevorzugt gesundheitsfördernden, Keimen, verwendet oder eingesetzt wird.
Aspekt 9:
   9. Cineol, vorzugsweise 1,8-Cineol, zur Verwendung nach einem der Aspekte 6 bis 8,
   wobei die gesundheitsfördernden oder nichtpathogenen, bevorzugt gesundheitsfördernden, Keime ausgewählt sind aus der Gruppe von entzündungsverringernden, entzündungshemmenden und entzündungsinhibierenden Keimen, insbesondere aus der Gruppe von entzündungshemmende und entzündungsinhibierende Substanzen produzierenden und/oder freisetzenden Keimen, sowie deren Kombinationen.
Aspekt 10:
   10. Cineol, vorzugsweise 1,8-Cineol, zur Verwendung nach einem der Aspekte 6 bis 9,
   wobei die gesundheitsfördernden oder nichtpathogenen, bevorzugt gesundheitsfördernden, Keime ausgewählt sind aus der Gruppe von Bakterien, insbesondere die Gesundheit positiv beeinflussenden und/oder gesundheitsförderlichen Bakterien, vorzugsweise aus der Gruppe von entzündungsverringernden, entzündungshemmenden und entzündungsinhibierenden Bakterien, sowie deren Kombinationen.
Aspekt 11:
   11. Cineol, vorzugsweise 1,8-Cineol, zur Verwendung nach Aspekt 9 oder 10,
   wobei die gesundheitsfördernden oder nichtpathogenen, bevorzugt gesundheitsfördernden, Keime ausgewählt sind aus der Gruppe von Bakterien der Familie der *Ruminococcacea*, vorzugsweise aus der Gruppe von Bakterien der Gattung *Ruminococcus.*
Aspekt 12:
   12. Cineol, vorzugsweise 1,8-Cineol, zur Verwendung nach Aspekt 9 oder 10,
   wobei die gesundheitsfördernden oder nichtpathogenen, bevorzugt gesundheitsfördernden, Keime ausgewählt sind aus der Gruppe von Bakterien der Familien *Bacteroidaceae, Lachnospiraceae* und *Bifidobacteriaceae*, insbesondere *Bacteroidaceae* und *Lachnospiraceae,* vorzugsweise *Bacteroidaceae,* sowie deren Kombinationen; und/oder
   wobei die gesundheitsfördernden oder nichtpathogenen, bevorzugt gesundheitsfördernden, Keime ausgewählt sind aus der Gruppe von Bakterien der Gattung *Bacteroides, Lachnospira* und *Bifidobacterium*, insbesondere *Bacteroides* und *Lachnospira*, vorzugsweise *Bacteroides,* sowie deren Kombinationen.
Aspekt 13:
   13. Cineol, vorzugsweise 1,8-Cineol, zur Verwendung nach einem der vorangehenden Aspekten,
   wobei das Cineol, vorzugsweise 1,8-Cineol, des Weiteren und/oder zusätzlich und/oder gleichermaßen zur Regeneration und/oder Sanierung der Keimbesiedlung des menschlichen Darms, insbesondere der menschlichen Darmflora, verwendet oder eingesetzt wird.
Aspekt 14:
   14. Cineol, vorzugsweise 1,8-Cineol, zur Verwendung nach einem der vorangehenden Aspekten,
   wobei das Cineol, vorzugsweise 1,8-Cineol, des Weiteren und/oder zusätzlich und/oder gleichermaßen zur Erhöhung der Keimvielfalt und/oder zur Diversifikation der Keimbesiedlung des menschlichen Darms, insbesondere der menschlichen Darmflora, verwendet oder eingesetzt wird,
   insbesondere zur Reduktion der Keimlast und/oder der Keimanzahl der pathogenen Keime im menschlichen Darm und/oder zur Reduktion des Keimanteils der pathogenen Keime, bezogen auf die Gesamtkeimanzahl im menschlichen Darm, insbesondere in der menschlichen Darmflora; und/oder
   insbesondere zur Erhöhung der Keimbeladung oder der Keimanzahl oder des Keimanteils, bezogen auf die Gesamtkeimanzahl im menschlichen Darm, gesundheitsfördernder oder nichtpathogener, bevorzugt gesundheitsfördernder, Keime, insbesondere wie zuvor definiert, im menschlichen Darm, insbesondere in der menschlichen Darmflora.
Aspekt 15:
   15. Cineol, vorzugsweise 1,8-Cineol, zur Verwendung nach einem der vorangehenden Aspekten,
   wobei das Cineol, vorzugsweise 1,8-Cineol, des Weiteren und/oder zusätzlich und/oder gleichermaßen als antiinflammatorischer Wirkstoff, insbesondere als systemisch wirkender antiinflammatorischer Wirkstoff, verwendet oder eingesetzt wird; und/oder
   wobei das Cineol, vorzugsweise 1,8-Cineol, des Weiteren und/oder zusätzlich und/oder gleichermaßen als Wirkstoff mit antiinflammatorischer Wirkung auf das insbesondere periphere Blut, vorzugsweise mit antiinflammatorischer Wirkung auf Immunzellen des insbesondere peripheren Bluts, verwendet oder eingesetzt wird.
Aspekt 16:
   16. Cineol, vorzugsweise 1,8-Cineol, zur Verwendung nach einem der vorangehenden Aspekten,
   wobei das Cineol, vorzugsweise 1,8-Cineol, des Weiteren und/oder zusätzlich und/oder gleichermaßen zur Suppression oder Abschwächung einer im Rahmen der entzündlichen Erkrankungen des menschlichen Körpers auftretenden Entzündungsmediation verwendet oder eingesetzt wird; und/oder
   wobei das Cineol, vorzugsweise 1,8-Cineol, eine supprimierende oder abschwächende Wirkung gegenüber einer im Rahmen der entzündlichen Erkrankungen des menschlichen Körpers auftretenden Entzündungsmediation ausübt.
Aspekt 17:
   17. Cineol, vorzugsweise 1,8-Cineol, zur Verwendung nach einem der vorangehenden Aspekten,
   wobei die entzündlichen Erkrankungen des menschlichen Körpers im Zusammenhang mit den pathogenen Keimen im menschlichen Darm, insbesondere in der menschlichen Darmflora, stehen oder hierdurch verursacht und/oder induziert und/oder verstärkt werden; und/oder
   wobei die entzündlichen Erkrankungen des menschlichen Körpers im Zusammenhang mit einer übermäßigen und/oder abnormen und/oder pathologischen Fehlbesiedlung, insbesondere Überbesiedlung, des menschlichen Darms, insbesondere der menschlichen Darmflora, mit den pathogenen Keimen stehen oder hierdurch verursacht und/oder induziert und/oder verstärkt werden; und/oder
   wobei die entzündlichen Erkrankungen des menschlichen Körpers im Zusammenhang mit einer Dysbiose und/oder einer Fehlbesiedlungen des menschlichen Darms, insbesondere der menschlichen Darmflora, mit pathogenen Keimen stehen oder hierdurch verursacht und/oder induziert und/oder verstärkt werden; und/oder
   wobei das Cineol, vorzugsweise 1,8-Cineol, eine antidysbiotische, insbesondere eine Fehlbesiedlung des menschlichen Darms mit pathogenen Keimen entgegenwirkende, Wirkung aufweist, insbesondere wobei das das Cineol, vorzugsweise 1,8-Cineol, eine den systemischen Immunstatus des menschlichen Körpers verbessernde und/oder regulierende, insbesondere eine eine Entzündungshemmung induzierende, Wirkung aufweist.
Aspekt 18:
   18. Cineol, vorzugsweise 1,8-Cineol, zur Verwendung nach einem der vorangehenden Aspekten,
   wobei die entzündlichen Erkrankungen des menschlichen Körpers lokale entzündliche Erkrankungen oder systemische entzündliche Erkrankungen sind; und/oder
   wobei die entzündlichen Erkrankungen des menschlichen Körpers akute entzündliche Erkrankungen oder chronische entzündliche Erkrankungen sind.
Aspekt 19:
   19. Cineol, vorzugsweise 1,8-Cineol, zur Verwendung nach einem der vorangehenden Aspekten,
   wobei die entzündlichen Erkrankungen des menschlichen Körpers ausgewählt sind aus der Gruppe von (i) entzündlichen Erkrankungen der oberen Atemwege, insbesondere entzündlichen Erkrankungen der Nase und/oder der Nasennebenhöhlen und/oder des Mund/Rachen-Raums; (ii) entzündlichen Erkrankungen des Hals/Nasen/Ohren-Bereichs; (iii) entzündlichen Erkrankungen der unteren Atemwege, insbesondere entzündlichen Pulmonalerkrankungen (Lungenerkrankungen), entzündlichen Bronchialerkrankungen und entzündlichen bronchopulmonalen Erkrankungen; (iv) entzündlichen Erkrankungen des Gastrointestinaltrakts, insbesondere des Darms; (v) entzündlichen Erkrankungen der Haut; (vi) entzündlichen Erkrankungen des Urogenitaltraktes, insbesondere entzündlichen Erkrankungen der ableitenden Harnwege; (vii) entzündlichrheumatoiden Erkrankungen, insbesondere Rheuma und Rheumatismus, und Erkrankungen des rheumatischen Formenkreises; und (viii) entzündlichen Erkrankungen (weiterer bzw. anderer) innerer Organe sowie deren Kombinationen.
Aspekt 20:
   20. Cineol, vorzugsweise 1,8-Cineol, zur Verwendung nach einem der vorangehenden Aspekten,
   wobei die entzündlichen Erkrankungen des menschlichen Körpers ausgewählt sind aus der Gruppe von Rhinitis, Sinusitis, Rhinosinusitis und Nasenpolypen (*Polyposis nasi et sinuum*) sowie deren Kombinationen; und/oder
   wobei die entzündlichen Erkrankungen des menschlichen Körpers ausgewählt sind aus der Gruppe von entzündlichen Erkrankungen des Ohres, vorzugsweise Otitis, bevorzugt Otitis media, sowie deren Kombinationen; und/oder
   wobei die entzündlichen Erkrankungen des menschlichen Körpers ausgewählt sind aus der Gruppe von Bronchitis, Asthma bronchiale und chronischen obstruktiven Lungenerkrankungen (COPD) sowie deren Kombinationen; und/oder
   wobei die entzündlichen Erkrankungen des menschlichen Körpers ausgewählt sind aus der Gruppe von *Morbus Crohn*, *Colitis ulcerosa* und chronisch entzündlichen Darmerkrankungen, wie *Inflammatory Bowel Disease* (IBD), sowie deren Kombinationen; und/oder
   wobei die entzündlichen Erkrankungen des menschlichen Körpers ausgewählt sind aus der Gruppe von Ekzemen und Dermatitis sowie deren Kombinationen; und/oder
   wobei die entzündlichen Erkrankungen des menschlichen Körpers ausgewählt sind aus der Gruppe von Glomerulonephritis, Pyelonephritis, Cystitis und Uritritis, sowie deren Kombinationen; und/oder
   wobei die entzündlichen Erkrankungen des menschlichen Körpers ausgewählt sind aus der Gruppe von entzündlichen Erkrankungen der Gallenwege, insbesondere Cholecystitis und Cholangitis, sowie deren Kombinationen.
Aspekt 21:
   21. Cineol, vorzugsweise 1,8-Cineol, zur Verwendung nach einem der vorangehenden Aspekte,
   wobei das Cineol, vorzugsweise 1,8-Cineol, systemisch, insbesondere peroral oder parenteral, vorzugsweise peroral, appliziert wird und/oder wobei das Cineol, vorzugsweise 1,8-Cineol, zur systemischen, insbesondere peroralen oder parenteralen, vorzugsweise peroralen, Applikation hergerichtet ist.
Aspekt 22:
   22. Cineol, vorzugsweise 1,8-Cineol, zur Verwendung nach einem der vorangehenden Aspekte,
   wobei das Cineol, vorzugsweise 1,8-Cineol, in Form einer peroral zu verabreichenden Darreichungsform appliziert und/oder verabreicht wird und/oder wobei das Cineol, vorzugsweise 1,8-Cineol, in einer peroral zu verabreichenden Darreichungsform hergerichtet ist.
Aspekt 23:
   23. Cineol, vorzugsweise 1,8-Cineol, zur Verwendung nach einem der vorangehenden Aspekte,
   wobei das Cineol, vorzugsweise 1,8-Cineol, als magensaftresistente, aber dünndarmlösliche systemische Darreichungsform, bevorzugt als Kapsel, Dragee, Pille, Tablette oder dergleichen, appliziert wird und/oder wobei das Cineol, vorzugsweise 1,8-Cineol, zur Verabreichung als magensaftresistente, aber dünndarmlösliche systemische Darreichungsform, bevorzugt als Kapsel, Dragee, Pille, Tablette oder dergleichen, hergerichtet ist.
Aspekt 24:
   24. Cineol, vorzugsweise 1,8-Cineol, zur Verwendung nach einem der vorangehenden Aspekte,
   wobei das Cineol, vorzugsweise 1,8-Cineol, in pharmazeutisch wirksamen und/oder therapeutisch wirksamen Mengen verabreicht wird und/oder wobei das Cineol, vorzugsweise 1,8-Cineol, zur Verabreichung in pharmazeutisch wirksamen und/oder therapeutisch wirksamen Mengen hergerichtet ist; und/oder
   wobei das Cineol, vorzugsweise 1,8-Cineol, mit einer Tagesdosis im Bereich von 1 bis 5.000 mg/diem, insbesondere im Bereich von 2 bis 3.000 mg/diem, vorzugsweise im Bereich von 5 bis 2.500 mg/diem, bevorzugt im Bereich von 10 bis 2.000 mg/diem, besonders bevorzugt im Bereich von 50 bis 1.500 mg/diem, verabreicht wird und/oder wobei das Cineol, vorzugsweise 1,8-Cineol, zur Verabreichung mit einer Tagesdosis im Bereich von 1 bis 5.000 mg/diem, insbesondere im Bereich von 2 bis 3.000 mg/diem, vorzugsweise im Bereich von 5 bis 2.500 mg/diem, bevorzugt im Bereich von 10 bis 2.000 mg/diem, besonders bevorzugt im Bereich von 50 bis 1.500 mg/diem, hergerichtet ist.
Aspekt 25:
   25. Cineol, vorzugsweise 1,8-Cineol, zur Verwendung nach einem der vorangehenden Aspekte,
   wobei das Cineol, vorzugsweise 1,8-Cineol, als Reinstoff vorliegt und/oder verabreicht wird;
   wobei das Cineol, vorzugsweise 1,8-Cineol, mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 Gew.-%, bezogen auf das Cineol, vorzugsweise 1,8-Cineol, vorliegt und/oder verabreicht wird; und/oder
   wobei das Cineol, vorzugsweise 1,8-Cineol, frei von anderen Terpenen ist und/oder insbesondere wobei das Cineol, vorzugsweise 1,8-Cineol, keine anderen Terpene enthält; und/oder
   wobei das Cineol, vorzugsweise 1,8-Cineol, in Abwesenheit von anderen (weiteren) Terpenen vorliegt und/oder verabreicht wird; und/oder
   wobei das Cineol, vorzugsweise 1,8-Cineol, als Reinstoff und/oder in Abwesenheit von anderen (weiteren) Terpenen vorliegt und/oder verabreicht wird.
Aspekt 26:
   26. Cineol, vorzugsweise 1,8-Cineol, zur Verwendung nach einem der vorangehenden Aspekte,
   wobei das Cineol, vorzugsweise 1,8-Cineol, zusammen mit mindestens einem physiologisch unbedenklichen Träger (Exzipienten), vorliegt und/oder verabreicht wird;
   insbesondere wobei der physiologisch unbedenkliche Träger (Exzipient) mit Cineol, vorzugsweise 1,8-Cineol, mischbar und/oder hierin löslich ist; und/oder
   insbesondere wobei der physiologisch unbedenkliche Träger (Exzipient) bei 20 °C und bei Atmosphärendruck im flüssigen oder festen, vorzugsweise flüssigen, Aggregatzustand vorliegt; und/oder
   insbesondere wobei der physiologisch unbedenkliche Träger (Exzipient) ausgewählt ist aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren; und/oder
   insbesondere wobei das Cineol, vorzugsweise 1,8-Cineol, als Reinstoff und/oder in Abwesenheit von anderen (weiteren) Terpenen vorliegt und/oder verabreicht wird; und/oder
   wobei das Cineol, vorzugsweise 1,8-Cineol, bevorzugt als Reinstoff und/oder in Abwesenheit von anderen (weiteren) Terpenen, zusammen mit mindestens einem physiologisch unbedenklichen Träger (Exzipienten) vorliegt und/oder verabreicht wird.
Aspekt 27:
   27. Cineol, vorzugsweise 1,8-Cineol, zur Verwendung nach einem der vorangehenden Aspekte,
   wobei das Cineol, vorzugsweise 1,8-Cineol, in und/oder in Form einer Zusammensetzung, insbesondere pharmazeutischen Zusammensetzung, insbesondere zusammen mit mindestens einem pharmazeutisch verträglichen und/oder physiologisch unbedenklichen Exzipienten (Träger), vorliegt und/oder verabreicht wird;
   insbesondere wobei die Zusammensetzung das Cineol, vorzugsweise 1,8-Cineol, als alleinigen Wirkstoff, insbesondere als alleinigen pharmazeutischen Wirkstoff, enthält; und/oder
   insbesondere wobei die Zusammensetzung das Cineol, vorzugsweise 1,8-Cineol, als Reinstoff enthält, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 Gew.-%, bezogen auf das Cineol, vorzugsweise 1,8-Cineol, und/oder vorzugsweise frei von anderen Terpenen; und/oder
   insbesondere wobei die Zusammensetzung das Cineol, vorzugsweise 1,8-Cineol, zumindest im Wesentlichen frei von anderen Terpenen enthält und/oder insbesondere wobei die Zusammensetzung zumindest im Wesentlichen kein weiteres Terpen enthält und/oder insbesondere wobei die Zusammensetzung zumindest im Wesentlichen frei von anderen Terpenen als Cineol, vorzugsweise 1,8-Cineol, ist; und/oder
   insbesondere wobei das Cineol, vorzugsweise 1,8-Cineol, als Reinstoff und/oder in Abwesenheit von anderen (weiteren) Terpenen vorliegt und/oder verabreicht wird; und/oder
   insbesondere wobei die Zusammensetzung das Cineol, vorzugsweise 1,8-Cineol, in wirksamen, insbesondere pharmazeutisch und/oder therapeutisch wirksamen, Mengen enthält; und/oder
   insbesondere wobei die Zusammensetzung das Cineol, vorzugsweise 1,8-Cineol, bezogen auf die Zusammensetzung, in relativen Mengen im Bereich von 0,0001 bis 80 Gew.-%, insbesondere im Bereich von 0,001 bis 75 Gew.-%, vorzugsweise im Bereich von 0,005 bis 70 Gew.-%, bevorzugt im Bereich von 0,01 bis 60 Gew.-%, besonders bevorzugt im Bereich von 0,05 bis 55 Gew.-%, ganz besonders bevorzugt im Bereich von 0,1 bis 50 Gew.-%, enthält; und/oder
   insbesondere wobei der mindestens eine pharmazeutisch verträgliche und/oder physiologisch unbedenkliche Exzipient (Träger) mit Cineol, vorzugsweise 1,8-Cineol, mischbar und/oder hierin löslich ist, vorzugsweise wobei der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegt und/oder vorzugsweise wobei der mindestens eine pharmazeutisch verträgliche und/oder physiologisch unbedenkliche Exzipient (Träger) ausgewählt ist aus der Gruppe von Fettsäureestern, bevorzugt Triglyceriden von Fettsäuren, besonders bevorzugt mittelkettigen Triglyceriden (*Medium Chain Triglycerides* bzw. MCT), ganz besonders bevorzugt Triglyceriden von C₆-C₁₂-Fettsäuren.
Aspekt 28:
   28. Cineol, vorzugsweise 1,8-Cineol, zur Verwendung nach einem der vorangehenden Aspekten,
   wobei das Cineol, vorzugsweise 1,8-Cineol, in liposomenumgebener und/oder liposomal verpackter Form vorliegt und/oder verabreicht wird; und/oder
   wobei das Cineol, vorzugsweise 1,8-Cineol, in micellierter Form und/oder in einer micellaren Darreichungsform vorliegt und/oder verabreicht wird.
Aspekt 29:
   29. Cineol, vorzugsweise 1,8-Cineol, zur Verwendung nach einem der Aspekte 1 bis 28,
   wobei das Cineol, vorzugsweise 1,8-Cineol, als Einzelwirkstoff und/oder als Monopräparat vorliegt und/oder verabreicht wird; und/oder
   wobei das Cineol, vorzugsweise 1,8-Cineol, ohne und/oder in Abwesenheit von weiteren, von Cineol, vorzugsweise 1,8-Cineol, verschiedenen Wirkstoffen vorliegt und/oder verabreicht wird.
Aspekt 30:
   30. Cineol, vorzugsweise 1,8-Cineol, zur Verwendung nach einem der Aspekte 1 bis 28,
   wobei das Cineol, vorzugsweise 1,8-Cineol, zusammen mit mindestens einem weiteren Wirkstoff und/oder als Co-Therapeutikum vorliegt und/oder verabreicht wird;
   insbesondere wobei der weitere Wirkstoff ausgewählt ist aus (i) entzündungshemmenden Wirkstoffen (Antiphlogistika), insbesondere nichtsteroidalen Entzündungshemmern (NSAIDs) oder steroidalen Entzündungshemmern, vorzugsweise steroidalen Entzündungshemmern, bevorzugt Kortikosteroiden; (ii) Antibiotika; (iii) Präbiotika; und (iv) die Darmflora positiv beeinflussenden Mikroorganismen; sowie deren Kombinationen; und/oder
   insbesondere wobei der weitere Wirkstoff getrennt, insbesondere räumlich getrennt, von dem Cineol, vorzugsweise 1,8-Cineol, aber funktional zusammenhängend hiermit eingesetzt und/oder verabreicht wird, insbesondere in Form eines Kit (Kit-of-part).
Aspekt 31:
   31. Cineol, vorzugsweise 1,8-Cineol, zur Verwendung nach einem der Aspekten 1 bis 28 oder nach Aspekt 30,
   wobei das Cineol, vorzugsweise 1,8-Cineol, zusammen mit mindestens einem Präbiotikum vorliegt und/oder verabreicht wird;
   insbesondere wobei das Präbiotikum eine Substanz ist, welche insbesondere spezifisch das Wachstum von die Darmflora positiv beeinflussenden Mikroorganismen fördert; und/oder
   insbesondere wobei das Präbiotikum ausgewählt ist aus der Gruppe von insbesondere natürlichen Polysacchariden, insbesondere natürlichen Oligosacchariden, und Zuckeralkoholen; und/oder
   insbesondere wobei das Präbiotikum Gummi arabicum ist; und/oder
   insbesondere wobei das Präbiotikum ausgewählt ist aus der Gruppe von Fructooligosacchariden und Galaktooligosacchariden, insbesondere jeweils in Form von Tri- bis Pentasacchariden, und deren Kombinationen; und/oder
   insbesondere wobei das Präbiotikum ausgewählt ist aus der Gruppe von Inulin, Sucrose, Stachyose, Raffinose, Lactulose und deren Kombinationen.
Aspekt 32:
   32. Cineol, vorzugsweise 1,8-Cineol, zur Verwendung nach einem der Aspekte 1 bis 28 und 30 oder 31.
   wobei das Cineol, vorzugsweise 1,8-Cineol, zusammen mit die Darmflora positiv beeinflussenden Mikroorganismen vorliegt und/oder verabreicht wird;
   insbesondere wobei die Mikroorganismen ausgewählt sind aus der Gruppe von Hefen und Bakterien; und/oder
   insbesondere wobei die Mikroorganismen ausgewählt sind aus der Gruppe von Spezies der Gattung *Saccharomyces,* insbesondere *Saccharomyces boulardii* und *Saccharomyces cervesiae*, vorzugsweise *Saccharomyces boulardii,* sowie deren Kombinationen; und/oder
   insbesondere wobei die Mikroorganismen ausgewählt sind aus der Gruppe von Spezies der Gattung *Bifidobacterium*, *Lactobacillus*, *Enterococcus*, *Escherichia*, *Streptococcus*, insbesondere jeweils in Form von deren die Darmflora positiv beeinflussenden Spezies, und deren Kombinationen; und/oder
   insbesondere wobei die Mikroorganismen als Trockensubstanz, insbesondere biologisch aktive Trockensubstanz, insbesondere in lyophilisierter Form und/oder als Lyophilisat, vorliegen und/oder verabreicht werden.
Aspekt 33:
   33. Cineol, vorzugsweise 1,8-Cineol, insbesondere Cineol zur Verwendung nach einem der vorangehenden Aspekte, zur Verwendung bei der prophylaktischen und/oder therapeutischen Behandlung von Dysbiose und/oder von Fehlbesiedlungen des menschlichen Darms, insbesondere der menschlichen Darmflora, mit pathogenen Keimen.
Aspekt 34:
   34. Verwendung von Cineol, vorzugsweise 1,8-Cineol, zur Verringerung pathogener Keime im menschlichen Darm, insbesondere in der menschlichen Darmflora, und/oder zur Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers.
Aspekt 35:
   35. Verwendung von Cineol, vorzugsweise 1,8-Cineol, zur Herstellung eines Arzneimittels oder Medikaments zur Verringerung pathogener Keime im menschlichen Darm, insbesondere in der menschlichen Darmflora, und/oder zur Herstellung eines Arzneimittels oder Medikaments zur Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers.
Aspekt 36:
   36. Verwendung von Cineol, vorzugsweise 1,8-Cineol, insbesondere Verwendung nach Aspekt 34 oder 35, (zur Herstellung eines Arzneimittels) zur prophylaktischen und/oder therapeutischen Behandlung von Dysbiose und/oder von Fehlbesiedlungen des menschlichen Darms, insbesondere der menschlichen Darmflora, mit pathogenen Keimen.
Aspekt 37:
   37. Verwendung von Cineol, vorzugsweise 1,8-Cineol, als Darmbehandlungsmittel zur Verringerung pathogener Keime im menschlichen Darm, insbesondere in der menschlichen Darmflora; und/oder zur Verwendung bei der Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers; und/oder zur prophylaktischen und/oder therapeutischen Behandlung von Dysbiose und/oder von insbesondere bakteriellen Fehlbesiedlungen des menschlichen Darms, insbesondere der menschlichen Darmflora.
Aspekt 38:
   38. Verfahren zur Verringerung pathogener Keime im menschlichen Darm, insbesondere in der menschlichen Darmflora, und/oder zur der Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers,
   wobei bei dem Verfahren einem insbesondere eine Fehlbesiedlung, insbesondere Überbesiedlung, des Darms, insbesondere der Darmflora, mit den pathogenen Keimen aufweisenden menschlichen Patienten eine die Keimlast und/oder die Keimanzahl der pathogenen Keime im Darm verringernde und/oder absenkende Menge an Cineol, vorzugsweise 1,8-Cineol, verabreicht wird; und/oder
   wobei bei dem Verfahren einem insbesondere eine Fehlbesiedlung, insbesondere Überbesiedlung, des Darms, insbesondere der Darmflora, mit den pathogenen Keimen aufweisenden menschlichen Patienten eine eine Reduktion des Keimanteils der pathogenen Keime, bezogen auf die Gesamtkeimanzahl im Darm, insbesondere in der Darmflora, hervorrufende Menge an Cineol, vorzugsweise 1,8-Cineol, verabreicht wird; und/oder
   wobei bei dem Verfahren einem insbesondere eine Fehlbesiedlung, insbesondere Überbesiedlung, des Darms, insbesondere der Darmflora, mit den pathogenen Keimen aufweisenden menschlichen Patienten eine pharmazeutisch wirksame und/oder therapeutisch wirksame Menge an Cineol, vorzugsweise 1,8-Cineol, verabreicht wird.
Aspekt 39:
   39. Verfahren, insbesondere Verfahren nach Aspekt 38, zur prophylaktischen und/oder therapeutischen Behandlung von Dysbiose und/oder von Fehlbesiedlungen des menschlichen Darms, insbesondere der menschlichen Darmflora, mit pathogenen Keimen,
   wobei bei dem Verfahren einem die Dysbiose und/oder die Fehlbesiedlung aufweisenden und/oder hierunter leidenden Patienten, insbesondere einem die Fehlbesiedlung, insbesondere Überbesiedlung, des Darms, insbesondere der Darmflora, mit den pathogenen Keimen aufweisenden Patienten und/oder einem unter Dysbiose leidenden Patienten eine pharmazeutisch wirksame und/oder therapeutisch wirksame Menge an Cineol, vorzugsweise 1,8-Cineol, verabreicht wird.
Aspekt 40:
   40. Arzneimittel oder Medikament, insbesondere Darmbehandlungsmittel,
   zur (Verwendung bei der) Verringerung pathogener Keime im menschlichen Darm, insbesondere in der menschlichen Darmflora, und/oder zur (Verwendung bei der) Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers,
   wobei das Arzneimittel oder Medikament, insbesondere Darmbehandlungsmittel, Cineol, vorzugsweise 1,8-Cineol, zusammen mit mindestens einem pharmazeutisch verträglichen und/oder physiologisch unbedenklichen Exzipienten (Träger) enthält.
Aspekt 41:
   41. Arzneimittel oder Medikament, insbesondere Darmbehandlungsmittel, insbesondere Arzneimittel oder Medikament nach Aspekt 40,
   zur prophylaktischen und/oder therapeutischen Behandlung von Dysbiose und/oder von Fehlbesiedlungen des menschlichen Darms, insbesondere der menschlichen Darmflora, mit pathogenen Keimen,
   wobei das Arzneimittel oder Medikament, insbesondere Darmbehandlungsmittel, Cineol, vorzugsweise 1,8-Cineol, zusammen mit mindestens einem pharmazeutisch verträglichen und/oder physiologisch unbedenklichen Exzipienten (Träger) enthält.
Aspekt 42:
   42. Arzneimittel oder Medikament, insbesondere Darmbehandlungsmittel, nach Aspekt 40 oder 41 bzw. Arzneimittel oder Medikament, insbesondere Darmbehandlungsmittel, zur Verwendung nach Aspekt 40 oder 41,
   wobei das Arzneimittel oder Medikament das Cineol, vorzugsweise 1,8-Cineol, als alleinigen Wirkstoff, insbesondere als alleinigen pharmazeutischen Wirkstoff, enthält; und/oder
   wobei das Arzneimittel oder Medikament das Cineol, vorzugsweise 1,8-Cineol, als Reinstoff enthält, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 Gew.-%, bezogen auf das Cineol, vorzugsweise 1,8-Cineol, und/oder vorzugsweise frei von anderen Terpenen; und/oder
   wobei das Arzneimittel oder Medikament das Cineol, vorzugsweise 1,8-Cineol, frei von anderen Terpenen enthält und/oder wobei das Arzneimittel oder Medikament kein weiteres Terpen enthält und/oder wobei das Arzneimittel oder Medikament frei von anderen Terpenen als Cineol, vorzugsweise 1,8-Cineol, ist; und/oder
   wobei das Arzneimittel oder Medikament das Cineol, vorzugsweise 1,8-Cineol, in wirksamen, insbesondere pharmazeutisch und/oder therapeutisch wirksamen, Mengen enthält; und/oder
   wobei das Arzneimittel oder Medikament das Cineol, vorzugsweise 1,8-Cineol, bezogen auf das Arzneimittel oder Medikament, in relativen Mengen im Bereich von 0,0001 bis 80 Gew.-%, insbesondere im Bereich von 0,001 bis 75 Gew.-%, vorzugsweise im Bereich von 0,005 bis 70 Gew.-%, bevorzugt im Bereich von 0,01 bis 60 Gew.-%, besonders bevorzugt im Bereich von 0,05 bis 55 Gew.-%, ganz besonders bevorzugt im Bereich von 0,1 bis 50 Gew.-%, enthält; und/oder
   wobei der mindestens eine pharmazeutisch verträgliche und/oder physiologisch unbedenkliche Exzipient (Träger) mit Cineol, vorzugsweise 1,8-Cineol, mischbar und/oder hierin löslich ist, vorzugsweise wobei der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegt und/oder vorzugsweise wobei der mindestens eine pharmazeutisch verträgliche und/oder physiologisch unbedenkliche Exzipient (Träger) ausgewählt ist aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren.
Aspekt 43:
   43. Zusammensetzung, insbesondere pharmazeutische Zusammensetzung, zur (Verwendung bei der) Verringerung pathogener Keime im menschlichen Darm, insbesondere in der menschlichen Darmflora, und/oder zur (Verwendung bei der) Reduktion der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers,
   wobei die Zusammensetzung Cineol, vorzugsweise 1,8-Cineol, insbesondere zusammen mit mindestens einem pharmazeutisch verträglichen und/oder physiologisch unbedenklichen Exzipienten (Träger), enthält.
Aspekt 44:
   44. Zusammensetzung, vorzugsweise pharmazeutische Zusammensetzung, insbesondere Zusammensetzung nach Aspekt 43, zur (Verwendung bei der) prophylaktischen und/oder therapeutischen Behandlung von Dysbiose und/oder von Fehlbesiedlungen des menschlichen Darms, insbesondere der menschlichen Darmflora, mit pathogenen Keimen,
   wobei die Zusammensetzung Cineol, vorzugsweise 1,8-Cineol, insbesondere zusammen mit mindestens einem pharmazeutisch verträglichen und/oder physiologisch unbedenklichen Exzipienten (Träger), enthält.
Aspekt 45:
   45. Zusammensetzung, insbesondere pharmazeutische Zusammensetzung, nach Aspekt 43 oder 44 bzw. Zusammensetzung, insbesondere pharmazeutische Zusammensetzung, zur Verwendung nach Aspekt 43 oder 44,
   wobei die Zusammensetzung das Cineol, vorzugsweise 1,8-Cineol, als alleinigen Wirkstoff, insbesondere als alleinigen pharmazeutischen Wirkstoff, enthält; und/oder
   wobei die Zusammensetzung das Cineol, vorzugsweise 1,8-Cineol, als Reinstoff enthält, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 Gew.-%, bezogen auf das Cineol, vorzugsweise 1,8-Cineol, und/oder vorzugsweise frei von anderen Terpenen; und/oder
   wobei die Zusammensetzung das Cineol, vorzugsweise 1,8-Cineol, frei von anderen Terpenen enthält und/oder wobei die Zusammensetzung kein weiteres Terpen enthält und/oder wobei die Zusammensetzung frei von anderen Terpenen als Cineol, vorzugsweise 1,8-Cineol, ist; und/oder
   wobei die Zusammensetzung das Cineol, vorzugsweise 1,8-Cineol, in wirksamen, insbesondere pharmazeutisch und/oder therapeutisch wirksamen, Mengen enthält; und/oder
   wobei die Zusammensetzung das Cineol, vorzugsweise 1,8-Cineol, bezogen auf die Zusammensetzung, in relativen Mengen im Bereich von 0,0001 bis 80 Gew.-%, insbesondere im Bereich von 0,001 bis 75 Gew.-%, vorzugsweise im Bereich von 0,005 bis 70 Gew.-%, bevorzugt im Bereich von 0,01 bis 60 Gew.-%, besonders bevorzugt im Bereich von 0,05 bis 55 Gew.-%, ganz besonders bevorzugt im Bereich von 0,1 bis 50 Gew.-%, enthält; und/oder
   wobei der mindestens eine pharmazeutisch verträgliche und/oder physiologisch unbedenkliche Exzipient (Träger) mit Cineol, vorzugsweise 1,8-Cineol, mischbar und/oder hierin löslich ist, vorzugsweise wobei der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegt und/oder vorzugsweise wobei der mindestens eine pharmazeutisch verträgliche und/oder physiologisch unbedenkliche Exzipient (Träger) ausgewählt ist aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren.
Aspekt 46:
   46. Cineol, vorzugsweise 1,8-Cineol, zur Verwendung nach einem der Aspekte 1 bis 33, Verwendung nach einem der Aspekte 34 bis 37, Verfahren nach Aspekt 38 oder 39, Arzneimittel oder Medikament nach einem der Aspekte 40 bis 42 sowie Zusammensetzung nach einem der Aspekte 43 bis 45,
   wobei das Cineol, vorzugsweise 1,8-Cineol, insbesondere das Arzneimittel oder Medikament oder die Zusammensetzung, in pharmazeutisch wirksamen und/oder therapeutisch wirksamen Mengen verabreicht wird und/oder wobei das Cineol, vorzugsweise 1,8-Cineol, insbesondere das Arzneimittel oder Medikament oder die Zusammensetzung, zur Verabreichung in pharmazeutisch wirksamen und/oder therapeutisch wirksamen Mengen hergerichtet ist; und/oder
   wobei das Cineol, vorzugsweise 1,8-Cineol, insbesondere das Arzneimittel oder Medikament oder die Zusammensetzung, systemisch appliziert wird; und/oder
   wobei das Cineol, vorzugsweise 1,8-Cineol, insbesondere das Arzneimittel oder Medikament oder die Zusammensetzung, zusammen mit mindestens einem weiteren Wirkstoff angewendet oder verabreicht wird, insbesondere wobei der weitere Wirkstoff ausgewählt ist aus der Gruppe von (i) entzündungshemmenden Wirkstoffen (Antiphlogistika), insbesondere nichtsteroidalen Entzündungshemmern (NSAIDs) oder steroidalen Entzündungshemmern, vorzugsweise steroidalen Entzündungshemmern, bevorzugt Kortikosteroiden; (ii) Antibiotika; (iii) Präbiotika; und (iv) die Darmflora positiv beeinflussenden Mikroorganismen; sowie deren Kombinationen.
Aspekt 47:
   47. Pharmazeutische Kombination zur (Verwendung bei der) Verringerung pathogener Keime im menschlichen Darm, insbesondere in der menschlichen Darmflora, und/oder zur (Verwendung bei der) Reduzierung der Besiedlung des menschlichen Darms, insbesondere der menschlichen Darmflora, mit pathogenen Keimen, vorzugsweise zu Zwecken der Vermeidung, Verringerung oder Heilung entzündlicher Erkrankungen des menschlichen Körpers und/oder vorzugsweise zu Zwecken der prophylaktischen und/oder therapeutischen Behandlung entzündlicher Erkrankungen des menschlichen Körpers,
   wobei die pharmazeutische Kombination mindestens die nachfolgenden Komponenten (A) und (B) umfasst:
   (A) Cineol, vorzugsweise 1,8-Cineol; sowie
   (B) mindestens einen weiteren Wirkstoff, ausgewählt aus der Gruppe von (i) entzündungshemmenden Wirkstoffen (Antiphlogistika), insbesondere nichtsteroidalen Entzündungshemmern (NSAIDs) oder steroidalen Entzündungshemmern, vorzugsweise steroidalen Entzündungshemmern, bevorzugt Kortikosteroiden; (ii) Antibiotika; (iii) Präbiotika; und (iv) die Darmflora positiv beeinflussenden Mikroorganismen; sowie deren Kombinationen.
Aspekt 48:
   48. Pharmazeutische Kombination, insbesondere pharmazeutische Kombination nach Aspekt 47, zur (Verwendung bei der) prophylaktischen und/oder therapeutischen Behandlung von Dysbiose und/oder von Fehlbesiedlungen des menschlichen Darms, insbesondere der menschlichen Darmflora, mit pathogenen Keimen,
   wobei die pharmazeutische Kombination mindestens die nachfolgenden Komponenten (A) und (B) umfasst:
   (A) Cineol, vorzugsweise 1,8-Cineol; sowie
   (B) mindestens einen weiteren Wirkstoff, ausgewählt aus der Gruppe von (i) entzündungshemmenden Wirkstoffen (Antiphlogistika), insbesondere nichtsteroidalen Entzündungshemmern (NSAIDs) oder steroidalen Entzündungshemmern, vorzugsweise steroidalen Entzündungshemmern, bevorzugt Kortikosteroiden; (ii) Antibiotika; (iii) Präbiotika; und (iv) die Darmflora positiv beeinflussenden Mikroorganismen; sowie deren Kombinationen.
Aspekt 49:
   49. Pharmazeutische Kombination nach Aspekt 47 oder 48 und/oder pharmazeutische Kombination zur Verwendung nach Aspekt 47 oder 48,
   wobei die Komponenten (A) und (B) getrennt voneinander, insbesondere räumlich getrennt voneinander, aber funktional zusammenhängend und/oder funktional einander zugeordnet vorliegen und/oder verabreicht werden; und/oder
   wobei die pharmazeutische Kombination in Form eines Kits (Kit-of-parts), insbesondere als Kit der Komponenten (A) und (B), vorliegt und/oder wobei die Komponenten (A) und (B) als Kit (Kit-of-parts) vorliegen und/oder hergerichtet sind und/oder verabreicht werden.
Aspekt 50:
   50. Verwendung nach einem der Aspekte 34 bis 37, Verfahren nach Aspekt 38 oder 39, Arzneimittel oder Medikament nach einem der Aspekte 40 bis 42, Zusammensetzung nach einem der Aspekte 43 bis 45 sowie pharmazeutische Kombination nach einem der Aspekte 47 bis 49,
   jeweils gekennzeichnet durch eines oder mehrere der Merkmale der Aspekte 1 bis 33.

## Patentansprüche

1. Zusammensetzung, insbesondere in Form eines Nahrungsergänzungsmittels oder Darmbehandlungsmittels, zur Verwendung bei der prophylaktischen und/oder therapeutischen Behandlung von Dysbiose und/oder von Fehlbesiedlungen des menschlichen Darms mit pathogenen Keimen.
wobei die Zusammensetzung Cineol, vorzugsweise 1,8-Cineol, insbesondere zusammen mit mindestens einem pharmazeutisch verträglichen und/oder physiologisch unbedenklichen Exzipienten (Träger), enthält.

2. Zusammensetzung zur Verwendung nach Anspruch 1,
wobei pathogene Keime im menschlichen Darm verringert werden und/oder die Besiedlung des menschlichen Darms mit pathogenen Keimen reduziert wird und wobei zusätzlich die Besiedlung des menschlichen Darms mit gesundheitsfördernden oder nichtpathogenen Keimen erhöht wird.

3. Zusammensetzung zur Verwendung nach Anspruch 1 oder Anspruch 2,
wobei die pathogenen Keime ausgewählt sind aus der Gruppe von entzündungsverursachenden, entzündungsinduzierenden und mit Entzündungen im menschlichen Körper im Zusammenhang stehenden oder diese hervorrufenden Bakterien sowie deren Kombinationen und wobei die gesundheitsfördernden oder nichtpathogenen Keime ausgewählt sind aus der Gruppe von entzündungsverringernden, entzündungshemmenden und entzündungsinhibierenden Keimen sowie deren Kombinationen,

4. Zusammensetzung zur Verwendung nach einem der vorangehenden Ansprüche,
wobei die pathogenen Keime ausgewählt sind aus der Gruppe von entzündungsverursachenden, entzündungsinduzierenden und mit Entzündungen im menschlichen Körper im Zusammenhang stehenden oder diese hervorrufenden Keimen, insbesondere aus der Gruppe von entzündungsverursachende und entzündungsfördernde Substanzen produzierenden und/oder freisetzenden Keimen, sowie deren Kombinationen; und/oder
wobei die pathogenen Keime ausgewählt sind aus der Gruppe von Bakterien, insbesondere die Gesundheit negativ beeinträchtigenden und/oder gesundheitsschädlichen Bakterien, vorzugsweise aus der Gruppe von entzündungsverursachenden, entzündungsinduzierenden und mit Entzündungen im menschlichen Körper im Zusammenhang stehenden oder diese hervorrufenden Bakterien, sowie deren Kombinationen; und/oder
wobei die pathogenen Keime ausgewählt sind aus der Gruppe von Bakterien aus der Familie der *Prevotellaceae*, vorzugsweise aus der Gruppe von Bakterien der Gattung *Prevotella.*

5. Zusammensetzung zur Verwendung nach einem der vorangehenden Ansprüche,
wobei die gesundheitsfördernden oder nichtpathogenen, bevorzugt gesundheitsfördernden, Keime ausgewählt sind aus der Gruppe von entzündungsverringernden, entzündungshemmenden und entzündungsinhibierenden Keimen, insbesondere aus der Gruppe von entzündungshemmende und entzündungsinhibierende Substanzen produzierenden und/oder freisetzenden Keimen, sowie deren Kombinationen; und/oder
wobei die gesundheitsfördernden oder nichtpathogenen, bevorzugt gesundheitsfördernden, Keime ausgewählt sind aus der Gruppe von Bakterien, insbesondere die Gesundheit positiv beeinflussenden und/oder gesundheitsförderlichen Bakterien, vorzugsweise aus der Gruppe von entzündungsverringernden, entzündungshemmenden und entzündungsinhibierenden Bakterien, sowie deren Kombinationen; und/oder
wobei die gesundheitsfördernden oder nichtpathogenen, bevorzugt gesundheitsfördernden, Keime ausgewählt sind aus der Gruppe von Bakterien der Familie der *Ruminococcacea*, vorzugsweise aus der Gruppe von Bakterien der Gattung *Ruminococcus*; und/oder
wobei die gesundheitsfördernden oder nichtpathogenen, bevorzugt gesundheitsfördernden, Keime ausgewählt sind aus der Gruppe von Bakterien der Familien *Bacteroidaceae, Lachnospiraceae* und *Bifidobacteriaceae*, insbesondere *Bacteroidaceae* und *Lachnospiraceae,* vorzugsweise *Bacteroidaceae,* sowie deren Kombinationen; und/oder
wobei die gesundheitsfördernden oder nichtpathogenen, bevorzugt gesundheitsfördernden, Keime ausgewählt sind aus der Gruppe von Bakterien der Gattung *Bacteroides, Lachnospira* und *Bifidobacterium*, insbesondere *Bacteroides* und *Lachnospira*, vorzugsweise *Bacteroides,* sowie deren Kombinationen.

6. Zusammensetzung zur Verwendung nach einem der vorangehenden Ansprüche,
wobei die Zusammensetzung und/oder das Cineol, vorzugsweise 1,8-Cineol, des Weiteren und/oder zusätzlich und/oder gleichermaßen zur Erhöhung der Keimvielfalt und/oder zur Diversifikation der Keimbesiedlung des menschlichen Darms, insbesondere der menschlichen Darmflora, verwendet oder eingesetzt wird,
insbesondere zur Reduktion der Keimlast und/oder der Keimanzahl der pathogenen Keime im menschlichen Darm und/oder zur Reduktion des Keimanteils der pathogenen Keime, bezogen auf die Gesamtkeimanzahl im menschlichen Darm, insbesondere in der menschlichen Darmflora; und/oder
insbesondere zur Erhöhung der Keimbeladung oder der Keimanzahl oder des Keimanteils, bezogen auf die Gesamtkeimanzahl im menschlichen Darm, gesundheitsfördernder oder nichtpathogener, bevorzugt gesundheitsfördernder, Keime, insbesondere wie zuvor definiert, im menschlichen Darm, insbesondere in der menschlichen Darmflora.

7. Zusammensetzung zur Verwendung nach einem der vorangehenden Ansprüche,
wobei die Zusammensetzung das Cineol, vorzugsweise 1,8-Cineol, als alleinigen Wirkstoff, insbesondere als alleinigen pharmazeutischen Wirkstoff, enthält; und/oder
wobei die Zusammensetzung das Cineol, vorzugsweise 1,8-Cineol, als Reinstoff enthält, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 Gew.-%, bezogen auf das Cineol, vorzugsweise 1,8-Cineol, und/oder vorzugsweise frei von anderen Terpenen; und/oder
wobei die Zusammensetzung das Cineol, vorzugsweise 1,8-Cineol, zumindest im Wesentlichen frei von anderen Terpenen enthält und/oder insbesondere wobei die Zusammensetzung zumindest im Wesentlichen kein weiteres Terpen enthält und/oder insbesondere wobei die Zusammensetzung zumindest im Wesentlichen frei von anderen Terpenen als Cineol, vorzugsweise 1,8-Cineol, ist; und/oder
wobei das Cineol, vorzugsweise 1,8-Cineol, als Reinstoff und/oder in Abwesenheit von anderen (weiteren) Terpenen vorliegt und/oder verabreicht wird; und/oder
wobei die Zusammensetzung das Cineol, vorzugsweise 1,8-Cineol, in wirksamen, insbesondere pharmazeutisch und/oder therapeutisch wirksamen, Mengen enthält; und/oder
wobei die Zusammensetzung das Cineol, vorzugsweise 1,8-Cineol, bezogen auf die Zusammensetzung, in relativen Mengen im Bereich von 0,0001 bis 80 Gew.-%, insbesondere im Bereich von 0,001 bis 75 Gew.-%, vorzugsweise im Bereich von 0,005 bis 70 Gew.-%, bevorzugt im Bereich von 0,01 bis 60 Gew.-%, besonders bevorzugt im Bereich von 0,05 bis 55 Gew.-%, ganz besonders bevorzugt im Bereich von 0,1 bis 50 Gew.-%, enthält; und/oder
wobei der mindestens eine pharmazeutisch verträgliche und/oder physiologisch unbedenkliche Exzipient (Träger) mit Cineol, vorzugsweise 1,8-Cineol, mischbar und/oder hierin löslich ist, vorzugsweise wobei der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegt und/oder vorzugsweise wobei der mindestens eine pharmazeutisch verträgliche und/oder physiologisch unbedenkliche Exzipient (Träger) ausgewählt ist aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren.

8. Zusammensetzung zur Verwendung nach einem der vorangehenden Ansprüche,
wobei das Cineol, vorzugsweise 1,8-Cineol, als Einzelwirkstoff und/oder als Monopräparat vorliegt und/oder verabreicht wird; und/oder
wobei das Cineol, vorzugsweise 1,8-Cineol, ohne und/oder in Abwesenheit von weiteren, von Cineol, vorzugsweise 1,8-Cineol, verschiedenen Wirkstoffen vorliegt und/oder verabreicht wird.

9. Zusammensetzung zur Verwendung nach einem der vorangehenden Ansprüche,
wobei das Cineol, vorzugsweise 1,8-Cineol, zusammen mit mindestens einem weiteren Wirkstoff und/oder als Co-Therapeutikum vorliegt und/oder verabreicht wird;
insbesondere wobei der weitere Wirkstoff ausgewählt ist aus (i) entzündungshemmenden Wirkstoffen (Antiphlogistika), insbesondere nichtsteroidalen Entzündungshemmern (NSAIDs) oder steroidalen Entzündungshemmern, vorzugsweise steroidalen Entzündungshemmern, bevorzugt Kortikosteroiden; (ii) Antibiotika; (iii) Präbiotika; und (iv) die Darmflora positiv beeinflussenden Mikroorganismen; sowie deren Kombinationen; und/oder
insbesondere wobei der weitere Wirkstoff getrennt, insbesondere räumlich getrennt, von dem Cineol, vorzugsweise 1,8-Cineol, aber funktional zusammenhängend hiermit eingesetzt und/oder verabreicht wird, insbesondere in Form eines Kit (Kit-of-part).

10. Zusammensetzung zur Verwendung nach einem der vorangehenden Ansprüche,
wobei das Cineol, vorzugsweise 1,8-Cineol, zusammen mit mindestens einem Präbiotikum vorliegt und/oder verabreicht wird;
insbesondere wobei das Präbiotikum eine Substanz ist, welche insbesondere spezifisch das Wachstum von die Darmflora positiv beeinflussenden Mikroorganismen fördert; und/oder
insbesondere wobei das Präbiotikum ausgewählt ist aus der Gruppe von insbesondere natürlichen Polysacchariden, insbesondere natürlichen Oligosacchariden, und Zuckeralkoholen; und/oder
insbesondere wobei das Präbiotikum Gummi arabicum ist; und/oder
insbesondere wobei das Präbiotikum ausgewählt ist aus der Gruppe von Fructooligosacchariden und Galaktooligosacchariden, insbesondere jeweils in Form von Tri- bis Pentasacchariden, und deren Kombinationen; und/oder
insbesondere wobei das Präbiotikum ausgewählt ist aus der Gruppe von Inulin, Sucrose, Stachyose, Raffinose, Lactulose und deren Kombinationen.

11. Zusammensetzung zur Verwendung nach einem der vorangehenden Ansprüche,
wobei das Cineol, vorzugsweise 1,8-Cineol, zusammen mit die Darmflora positiv beeinflussenden Mikroorganismen vorliegt und/oder verabreicht wird;
insbesondere wobei die Mikroorganismen ausgewählt sind aus der Gruppe von Hefen und Bakterien; und/oder
insbesondere wobei die Mikroorganismen ausgewählt sind aus der Gruppe von Spezies der Gattung *Saccharomyces,* insbesondere *Saccharomyces boulardii* und *Saccharomyces cervesiae*, vorzugsweise *Saccharomyces boulardii,* sowie deren Kombinationen; und/oder
insbesondere wobei die Mikroorganismen ausgewählt sind aus der Gruppe von Spezies der Gattung *Bifidobacterium*, *Lactobacillus*, *Enterococcus*, *Escherichia*, *Streptococcus*, insbesondere jeweils in Form von deren die Darmflora positiv beeinflussenden Spezies, und deren Kombinationen; und/oder
insbesondere wobei die Mikroorganismen als Trockensubstanz, insbesondere biologisch aktive Trockensubstanz, insbesondere in lyophilisierter Form und/oder als Lyophilisat, vorliegen und/oder verabreicht werden.

12. Zusammensetzung zur Verwendung nach einem der vorangehenden Ansprüche,
wobei die Zusammensetzung und/oder das Cineol, vorzugsweise 1,8-Cineol, systemisch, insbesondere peroral oder parenteral, vorzugsweise peroral, appliziert wird und/oder wobei die Zusammensetzung und/oder das Cineol, vorzugsweise 1,8-Cineol, zur systemischen, insbesondere peroralen oder parenteralen, vorzugsweise peroralen, Applikation hergerichtet ist.

13. Zusammensetzung zur Verwendung nach einem der vorangehenden Ansprüche,
wobei die Zusammensetzung und/oder das Cineol, vorzugsweise 1,8-Cineol, in Form einer peroral zu verabreichenden Darreichungsform appliziert und/oder verabreicht wird und/oder wobei die Zusammensetzung und/oder das Cineol, vorzugsweise 1,8-Cineol, in einer peroral zu verabreichenden Darreichungsform hergerichtet ist.

14. Zusammensetzung zur Verwendung nach einem der vorangehenden Ansprüche,
wobei die Zusammensetzung und/oder das Cineol, vorzugsweise 1,8-Cineol, als magensaftresistente, aber dünndarmlösliche systemische Darreichungsform, bevorzugt als Kapsel, Dragee, Pille, Tablette oder dergleichen, appliziert wird und/oder wobei die Zusammensetzung und/oder das Cineol, vorzugsweise 1,8-Cineol, zur Verabreichung als magensaftresistente, aber dünndarmlösliche systemische Darreichungsform, bevorzugt als Kapsel, Dragee, Pille, Tablette oder dergleichen, hergerichtet ist.

15. Zusammensetzung zur Verwendung nach einem der vorangehenden Ansprüche,
wobei die Zusammensetzung das Cineol, vorzugsweise 1,8-Cineol, als alleinigen Wirkstoff, insbesondere als alleinigen pharmazeutischen Wirkstoff, enthält; und/oder
wobei die Zusammensetzung das Cineol, vorzugsweise 1,8-Cineol, als Reinstoff enthält, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 Gew.-%, bezogen auf das Cineol, vorzugsweise 1,8-Cineol, und/oder vorzugsweise frei von anderen Terpenen; und/oder
wobei die Zusammensetzung das Cineol, vorzugsweise 1,8-Cineol, frei von anderen Terpenen enthält und/oder wobei die Zusammensetzung kein weiteres Terpen enthält und/oder wobei die Zusammensetzung frei von anderen Terpenen als Cineol, vorzugsweise 1,8-Cineol, ist; und/oder
wobei die Zusammensetzung das Cineol, vorzugsweise 1,8-Cineol, in wirksamen, insbesondere pharmazeutisch und/oder therapeutisch wirksamen, Mengen enthält; und/oder
wobei die Zusammensetzung das Cineol, vorzugsweise 1,8-Cineol, bezogen auf die Zusammensetzung, in relativen Mengen im Bereich von 0,0001 bis 80 Gew.-%, insbesondere im Bereich von 0,001 bis 75 Gew.-%, vorzugsweise im Bereich von 0,005 bis 70 Gew.-%, bevorzugt im Bereich von 0,01 bis 60 Gew.-%, besonders bevorzugt im Bereich von 0,05 bis 55 Gew.-%, ganz besonders bevorzugt im Bereich von 0,1 bis 50 Gew.-%, enthält; und/oder
wobei der mindestens eine pharmazeutisch verträgliche und/oder physiologisch unbedenkliche Exzipient (Träger) mit Cineol, vorzugsweise 1,8-Cineol, mischbar und/oder hierin löslich ist, vorzugsweise wobei der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegt und/oder vorzugsweise wobei der mindestens eine pharmazeutisch verträgliche und/oder physiologisch unbedenkliche Exzipient (Träger) ausgewählt ist aus der Gruppe von Fettsäureestern, bevorzugt Triglyceriden von Fettsäuren, besonders bevorzugt mittelkettigen Triglyceriden *(Medium Chain Triglycerides* bzw. MCT), ganz besonders bevorzugt Triglyceriden von C₆-C₁₂-Fettsäuren.
